# EUROPEAN PATENT SPECIFICATION

(11) **EP 2 683 834 B1**
(45) Date of publication and mention of the grant of the patent: **06.12.2017**
(21) Application number: 12711229.0
(22) Date of filing: 08.03.2012
(51) Int. Cl.: C12Q 1/68

(54) **METHODS AND BIOMARKERS FOR DETECTION OF GASTROINTESTINAL CANCERS**
VERFAHREN UND BIOMARKER FÜR DEN NACHWEIS VON GASTROINTESTINALKARZINOM
MÉTHODES ET BIOMARQUEURS DE DÉTECTION DE CANCERS GASTRO-INTESTINAUX

(30) Priority: 10.03.2011 US 201161451198 P
(43) Date of publication of application: 15.01.2014
(73) Proprietor: Oslo Universitetssykehus HF, 0424 Oslo (NO)
(72) Inventor: LOTHE, Ragnhild, A., N-0267 Oslo (NO); LIND, Guro, E., N-0770 Oslo (NO); AHMED, Deeqa, N-1253 Oslo (NO); ANDRESEN, Kim, N-0765 Oslo (NO); SKOTHEIM, Rolf, I., N-0372 Oslo (NO)
(74) Representative: Algemeen Octrooi- en Merkenbureau B.V.
(86) International application number: PCT/IB2012/000524
(87) International publication number: WO 2012/120374

(56) References cited:
- WO-A1-2011/002029
- WO-A2-2006/113671
- WO-A2-2007/149269
- ALI D ET AL: "694 Identification of novel epigenetic biomarkers in colorectal cancer, GLDC and PPP1R14A", EUROPEAN JOURNAL OF CANCER. SUPPLEMENT, PERGAMON, OXFORD, GB, vol. 8, no. 5, 1 June 2010 (2010-06-01), page 175, XP027105921, ISSN: 1359-6349 [retrieved on 2010-06-01]
- Deeqa Ahmed Mohamd Ali: "Identification of novel epigenetic biomarkers in colorectal cancer, GLDC and PPP1R14A", , 1 January 2010 (2010-01-01), XP055031459, Retrieved from the Internet: URL:http://www.ous-research.no/cancerpreve ntion/docs/PDFer/masteroppgave_deeqa.pdf [retrieved on 2012-07-02]
- RAFAEL A IRIZARRY ET AL: "The human colon cancer methylome shows similar hypo- and hypermethylation at conserved tissue-specific CpG island shores", NATURE GENETICS, vol. 41, no. 2, 1 February 2009 (2009-02-01), pages 178-186, XP055029485, ISSN: 1061-4036, DOI: 10.1038/ng.298
- CALDWELL G M ET AL: "The Wnt antagonist sFRP1 in colorectal tumorigenesis", CANCER RESEARCH, AMERICAN ASSOCIATION FOR CANCER RESEARCH, US, vol. 64, no. 3, 1 February 2004 (2004-02-01), pages 883-888, XP002348528, ISSN: 0008-5472, DOI: 10.1158/0008-5472.CAN-03-1346

## Description

### FIELD OF THE INVENTION

The present invention relates to methods and biomarkers (e.g., epigenetic biomarkers) for detection of gastrointestinal cancers (e.g., colorectal cancer, gastric cancer, pancreatic cancer, liver cancer, cancer of the gall bladder and/or bile ducts (e.g., cholangiocarcinoma)) in biological samples (e.g., tissue samples, stool samples, blood samples, plasma samples, cell samples, gall samples, bile samples, serum samples).

### BACKGROUND OF THE INVENTION

Cholangiocarcinoma (CCA) is the second most prevalent primary hepatobiliary malignancy and represents about 3% of all gastrointestinal cancers (1;2). According to the localization, CCAs are classified as being either extrahepatic or intrahepatic. Common for these subtypes is that they arise from the biliary epithelium and that they are difficult to diagnose. CCA is associated with inflammatory conditions in the biliary system, and patients with risk factors such as primary sclerosing cholangitis and liver fluke infestations have a higher risk of developing this malignancy (3-6). The generally late clinical presentation of CCA results in a high mortality, with a reported 5-year survival of only 5-15% (1;2).

The diagnostics of CCA remains challenging. The current clinical strategy for rising a suspicion of malignancy includes a combination of various imaging modalities, as well as biliary brush cytology and analysis of a few serum markers (4;7;8). However, CCA can often not be confirmed until a laparoscopy has been performed (4). The most commonly used molecular marker for detecting CCA is carbohydrate antigen 19-9 (9). Unfortunately, this marker harbors limitations such as dependence of the Lewis phenotype and elevated levels due to the presence of other gastrointestinal malignancies or even benign conditions such as acute cholangitis and cirrhosis (10-12).

Tumor-specific molecular alterations, including both genetic- and epigenetic aberrations, have been shown to play important roles in cancer development (13-16). Impaired epigenetic regulation, including aberrant DNA methylation, is frequently reported in cancers (13;16-18). In humans, DNA methylation occurs at the 5-position of cytosines in a CpG context (19). The bulk of the genome is methylated at the majority of these CpG sites, whereas dense CpG clusters, so-called CpG islands are usually devoid of methylation. Aberrant DNA hypermethylation of CpG islands located in the promoter region of genes is associated with transcriptional silencing (20;21). Loss of expression of essential tumor suppressor genes may lead to tumor development. Since aberrant DNA methylation also has been shown to be an early event in tumorigenesis (17;22-24), such targets may represent attractive biomarkers for early detection. Several genes, including *RASSF1A* and *CDKN2A* (*p16*) have so far been analyzed for promoter methylation in CCA (Table 4). However, only genes frequently hypermethylated in tumors and unmethylated in normal tissue represent promising biomarkers. CCAs can only be cured by radical resection or in selected cases by liver transplantation. Most frequently, patients have too advanced disease at presentation to be candidates for surgery. The identification of suitable epigenetic CCA biomarkers with high sensitivity and specificity may facilitate cancer diagnostics at an early stage and thus contribute to increase survival of this patient cohort which presently holds poor outcome.

Colorectal cancer is one of the most common malignancies in the Western world, with an incidence of 3600 new cases per year in Norway alone. Colon cancer is cancer of the large intestine (colon), the lower part of the digestive system. Rectal cancer is cancer of the last several inches of the colon. Together, they're often referred to as colorectal cancers. Most cases of colon cancer begin as small, noncancerous (benign) clumps of cells called adenomatous polyps. Over time some of these polyps become colon cancers.

With early detection and treatment, colorectal cancers have a low mortality rate. However, later stage cancers require invasive treatments with unpleasant side effects and exhibit a much higher mortality rate.

Better, more effective non-invasive tests for early detection of cholangiocarcinoma, colorectal and other gastrointestinal cancers are needed to lower the morbidity and mortality associated with colorectal cancer.

### SUMMARY OF THE INVENTION

The present invention relates to methods and biomarkers (e.g., epigenetic biomarkers) for detection of gastrointestinal cancers (e.g., colorectal cancer, gastric cancer, pancreatic cancer, liver cancer, cancer of the gall bladder and/or bile ducts (e.g., cholangiocarcinoma)) in biological samples (e.g., tissue samples, stool samples, blood samples, plasma samples, cell samples, gall samples, bile samples, serum samples).

In experiments conducted during the course of developing some embodiments of the present invention, using a combined approach of microarray analysis and *in vitro, in vivo* and *in silico* analysis *GLDC* and *PPP1R14A* were identified as novel epigenetically deregulated genes in colorectal cancer. Both genes were unmethylated in normal mucosa samples, and frequently methylated in colorectal tumors, resulting in a sensitivity of 60% and 57%, respectively, and a specificity of 100%.

Further experiments identified several hypermethylated genes, where *CDO1, DCLK1, ZNF331* isoform "c" and *SCAN18* isoform "b" were found to be 100% methylated in bile duct- and colorectal cancer cell lines. The strict selection criteria applied throughout this approach along with the frequent promoter hypermethylation observed among primary tumours make *CDO1*, *DCLK1, ZNF331* isoform "c" and *SCAN18* isoform "b" useful biomarkers for early cancer detection.

For example, in some embodiments, the present invention provides a set of methylation specific nucleic acid detection sequences, said detection sequences being specific for *GLDC* and for one or more genes selected from the group consisting *of CDO1, DCLK1, SCAN18* and *ZNF331,* optionally for at least one additional gene being selected from the group consisting *of SFRP1* and *PPP1R14A.* In some embodiments, the present invention provides a use of the set of nucleic acid sequences for detecting a cancerous condition in a subject (e.g., a gastrointestinal neoplasm; e.g., colorectal cancer). In some embodiments, the detection sequences are probes specific for *GLDC* and one or more of *CDO1, DCLK1, SCAN18 and ZNF331* (SEQ ID NOs.: 185, 187, 188, 189, and 190, respectively), and optionally *PPP1R14A* (SEQ ID NO 186). In some embodiments, the detection sequences are primers specific for *GLDC,* and one or more of *CDO1, DCLK1, ZSCAN18 and ZNF331* (SEQ ID NOs.: 185, 187, 188, 189, and 190, respectively), and optionally *PPP1R14A* (SEQ ID NO 186). In some preferred embodiments, the methylation specific nucleic acid detection sequences are MSP (methylation-specific PCR) primers. Exemplary primer sets include, but are not limited to: for *CDO1* (SEQ ID NO:17 and 18, SEQ ID NO:19 and 20, and SEQ ID NO:21 and 22); for *DCLK1* (SEQ ID NO:39 and 40, SEQ ID NO:41 and 42, and SEQ ID NO:43 and 44); for *ZSCAN18* (SEQ ID NO:161 and 162, SEQ ID NO:163 and 164, and SEQ ID NO:165 and 166). In some embodiments, the methylation specific nucleic acid detection sequence comprises a primer and probe set for quantitative MSP. Exemplary primer and probe set include, but are not limited to: for *CDO1* (SEQ ID NOs.:170, 171 and 172); for *DCLK1* (SEQ ID NOs:173, 174 and 175); for *SCAN18* (SEQ ID NOs:179, 180 and 181); and for *SFRP1* (176, 177 and 178). The detection sequences may be specific for regions upstream of the target gene, within the target gene, or downstream of the target gene. In some preferred embodiments, the detection sequences are specific for a region of the target sequence defined by a region that begins approximately -500, -400, -300, -200, -150, -100, -75 or -50 base pairs from the start codon of the target gene and ends at a position approximately -10, -10, 0, 10, 20, 30, 40, 50, 100, 120, 150 or 200 base pairs from the start codon of the target gene.

Further embodiments of the present invention provide a method for detecting a gastrointestinal neoplasm in a subject comprising: obtaining DNA from a biological sample of the subject; and determining the level, presence, or frequency of methylation of nucleic acid polymers corresponding to *GLDC* and one or more genes, selected from the group, consisting of *CDO1*, *DCLK1, SCAN18* and *ZNF331,* and optionally *PPP1R14A.* In some embodiments, the nucleic acid comprises a CpG island or a CpG island shore. In some embodiments, the CpG island or shore is present in a coding region or a regulatory region (e.g., a promoter). In some embodiments, determining of the level of altered methylation of a nucleic acid polymer comprises determining the methylation frequency of the CpG island or island shore. In some embodiments, determining of the level of a nucleic acid polymer with altered methylation is achieved by a technique selected from, for example, methylation-specific PCR, quantitative methylation-specific PCR, methylation-sensitive DNA restriction enzyme analysis, quantitative bisulfite pyrosequencing, next generation sequencing and bisulfite genomic sequencing PCR. In some embodiments, the methods employ the methylation specific nucleic acid detection sequences described in detail above. In some embodiments, the method further comprises generating a risk profile. In some embodiments, the gastrointestinal neoplasm is colorectal cancer. In some embodiments, the method permits detection of gastrointestinal cancer in the subject with a sensitivity of at least 85% at a specificity of at least 85%. In other embodiments, the method permits detection of gastrointestinal cancer in the subject with a sensitivity of at least 80% at a specificity of at least 90%. The biological sample can be a tissue sample, a stool sample, a blood sample, a plasma sample, a cell sample, a gall sample, a bile sample or a serum sample.

Additional embodiments of the present invention relates to the use of a kit for detecting the presence of a gastrointestinal neoplasm in a mammal, the kit comprising reagents useful, sufficient, or necessary for detecting and/or characterizing level, presence, or frequency of methylation of *GLDC* and one or more genes selected from the group consisting of *CDO1*, *DCLK1, SCAN18* and *ZNF331,* and optionally *PPP1R14A.* The kits employ the methylation specific nucleic acid detection sequences described in detail above.

Additional embodiments will be apparent to persons skilled in the relevant art based on the teachings contained herein.

### DESCRIPTION OF THE DRAWINGS

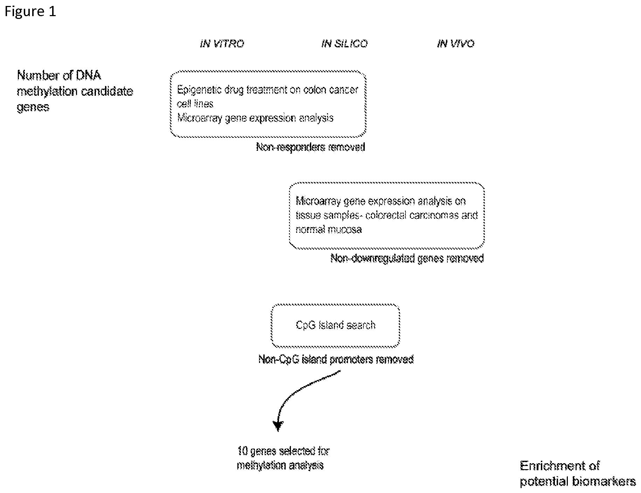

**Figure 1****.** Strategy to select novel DNA methylation candidate genes.

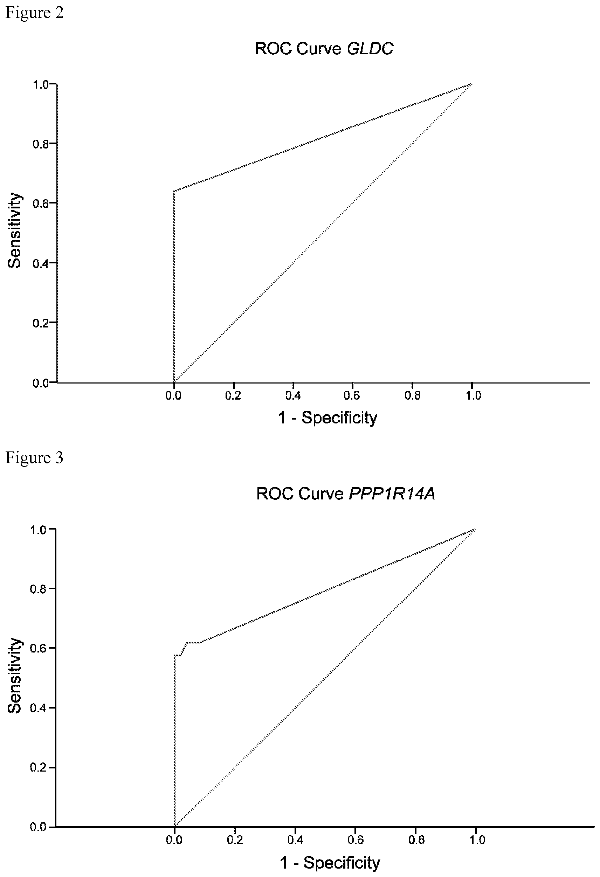

**Figure 2****.** ROC curve analysis from quantitative methylation-specific PCR results of *GLDC.*
**Figure 3****.** Roc curve analysis from quantitative methylation-specifc PCR results of *PPP1R14A.*

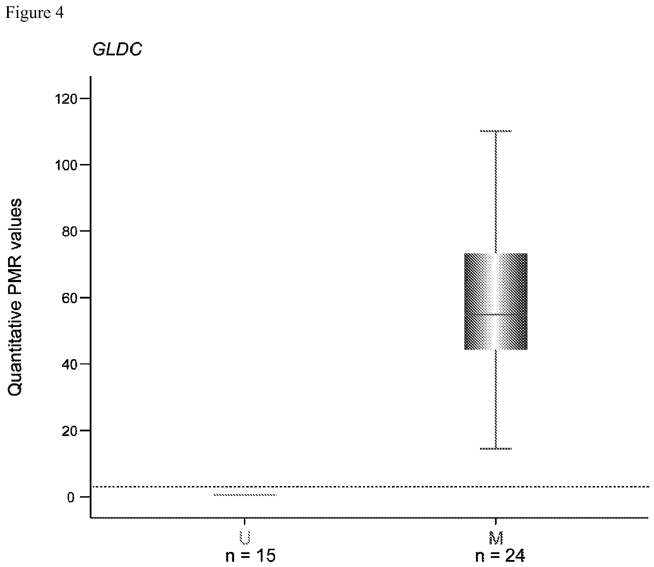

**Figure 4****.** Comparison of scores obtained from conventional MSP and quantitative MSP analysis of *GLDC.*

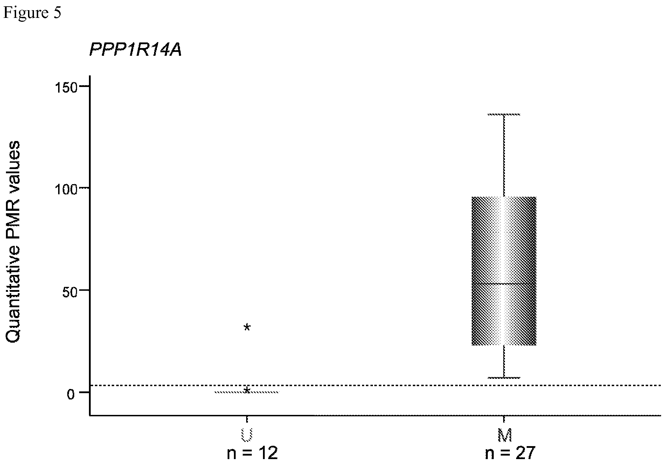

**Figure 5****.** Comparison of scores obtained from conventional MSP and quantitative MSP of PPP1R14A.

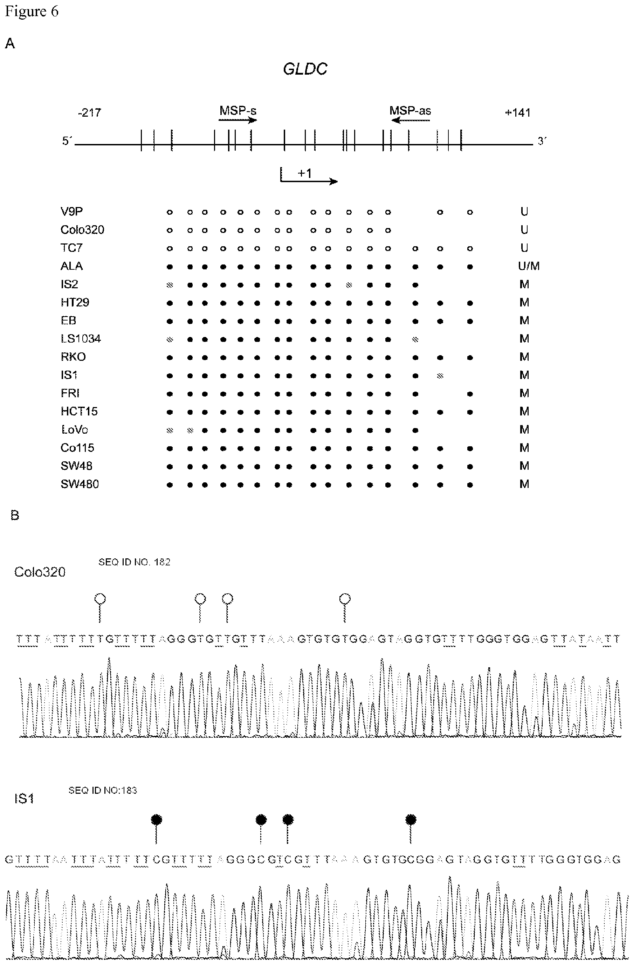

**Figure** 6. Bisulfite sequencing verifies site specific methylation within the *GLDC* promoter. **A**) The upper part of the figure is a schematic presentation of the CpG sites amplified by bisulfite sequencing primers. **B**) Representative bisulfite sequencing electropherograms of the *GLDC* promoter in colon cancer cell lines.

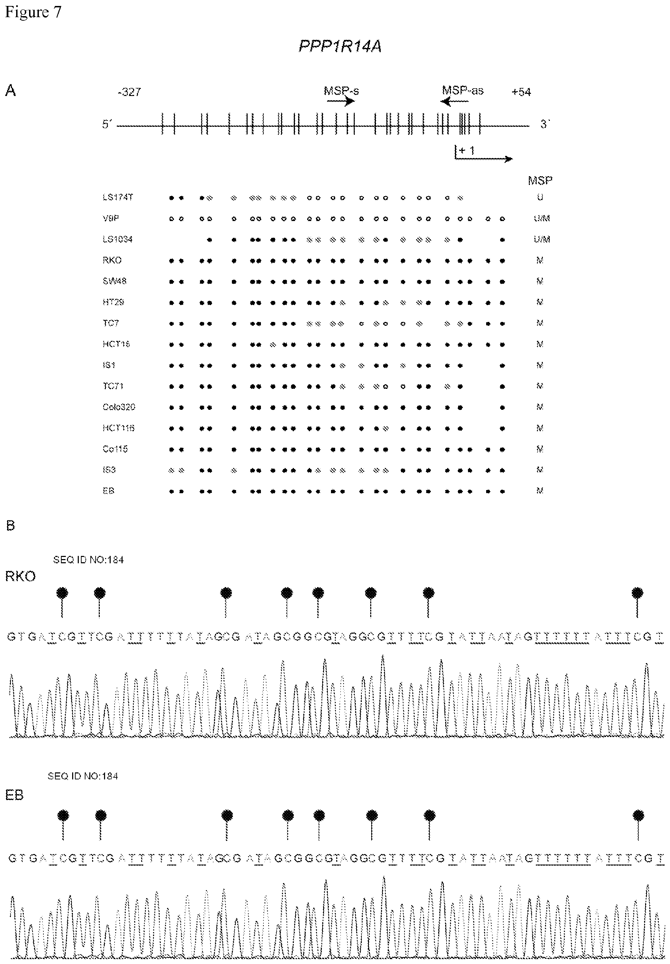

**Figure 7****.** Bisulfite sequencing verifies site specific methylation within the *PPP1R14A* promoter. **A**) The upper part of the figure is a schematic presentation of the CpG sites amplified by bisulfite sequencing primers. **B**) Representative bisulfite sequencing electropherograms of the *PPP1R14A* promoter in colon cancer cell lines.

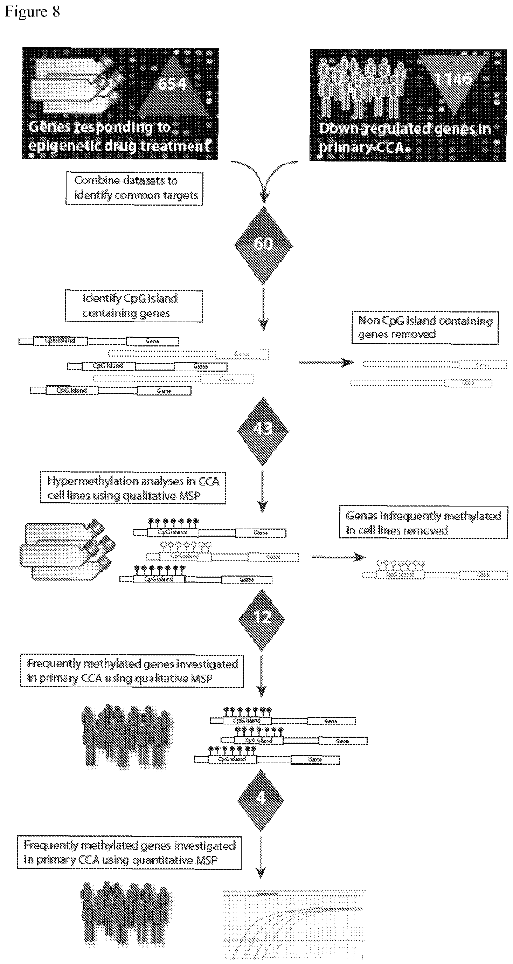

**Figure 8****.** Epigenome-wide experimental approach for identifying hypermethylated genes in cholangiocarcinomas. Six CCA cell lines were cultured with and without a combination of epigenetic drugs (5-aza-2'deoxycytidine and trichostatin A). Array-elements responding to epigenetic drug treatment were compared to previously published down-regulated genes in CCAs compared to cancer free tissue. Common genes, harboring a CpG island in the promoter region, were investigated for hypermethylation in cancer cell lines from colon, bile duct, liver, gall bladder, and pancreas. Genes frequently methylated in CCA cell lines were subsequently investigated in patient material using MSP. The most promising candidates from this analysis were further evaluated by qMSP. Numbers indicate the number of genes fulfilling the selection criteria in each experimental step and subsequently subjected to further analyses.

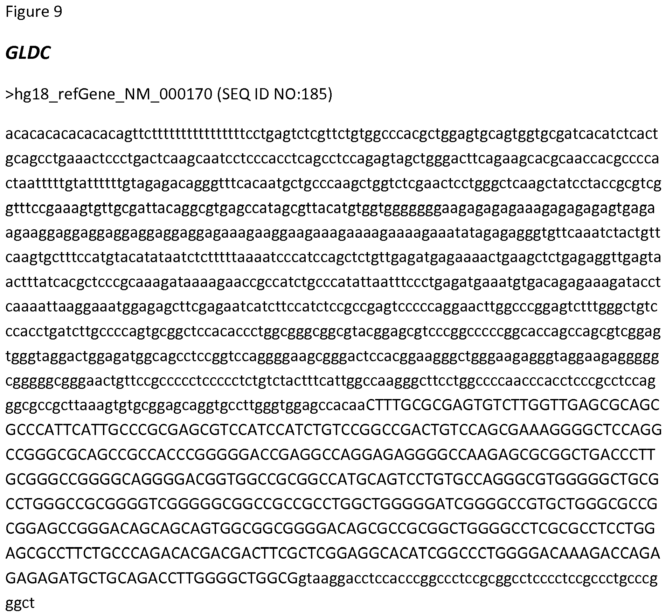

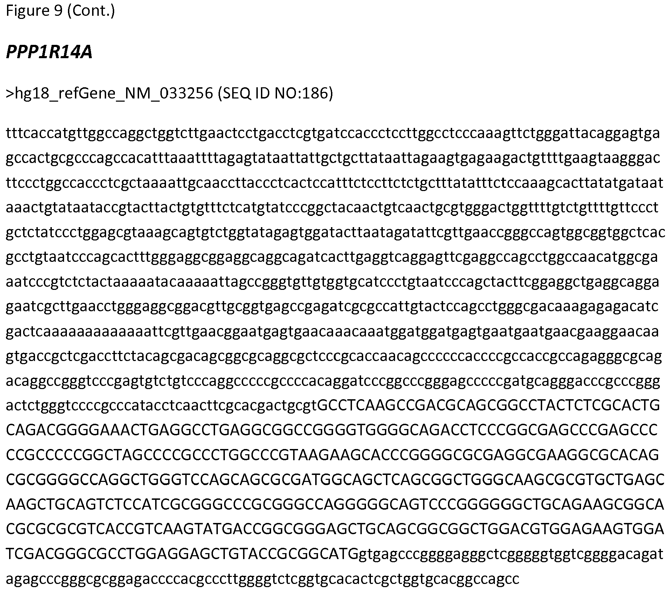

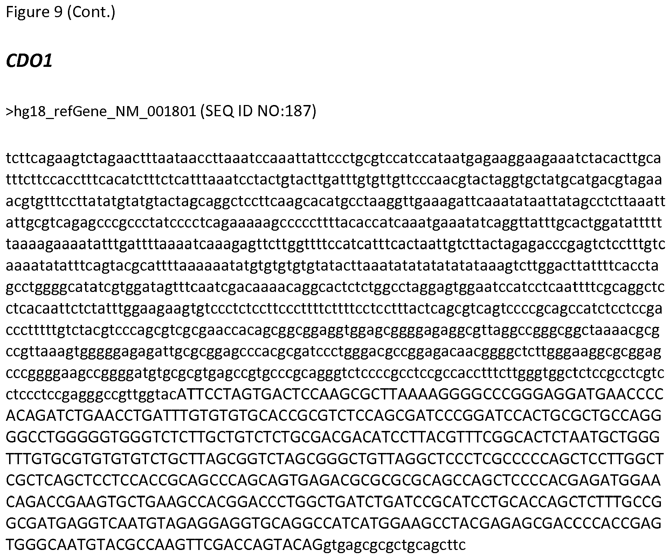

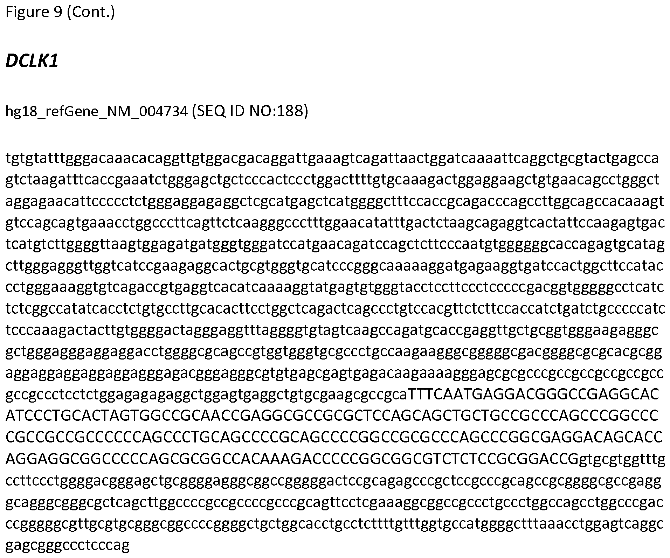

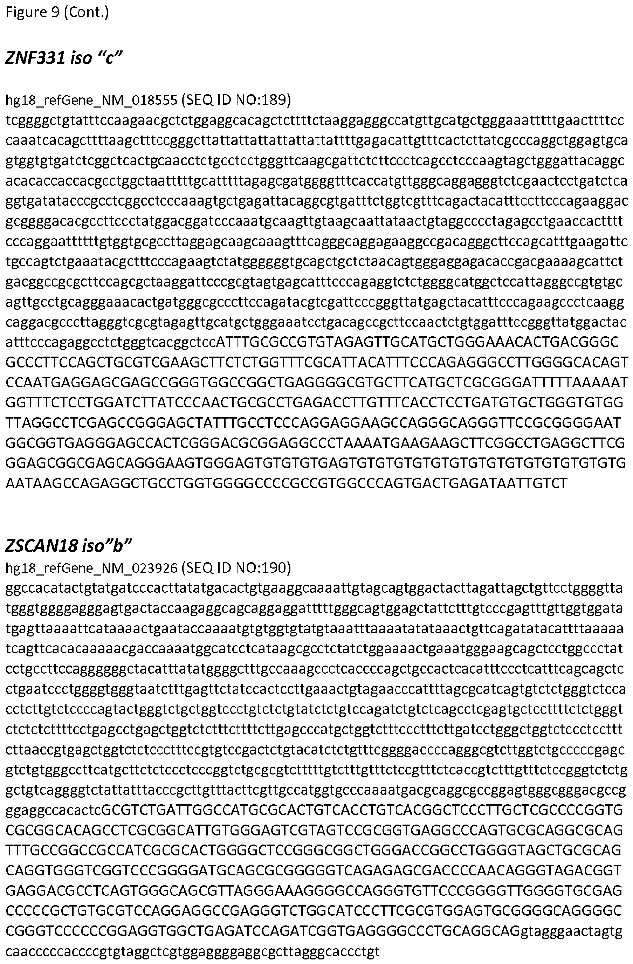

**Figure 9****.** Sequences of exemplary target genes.

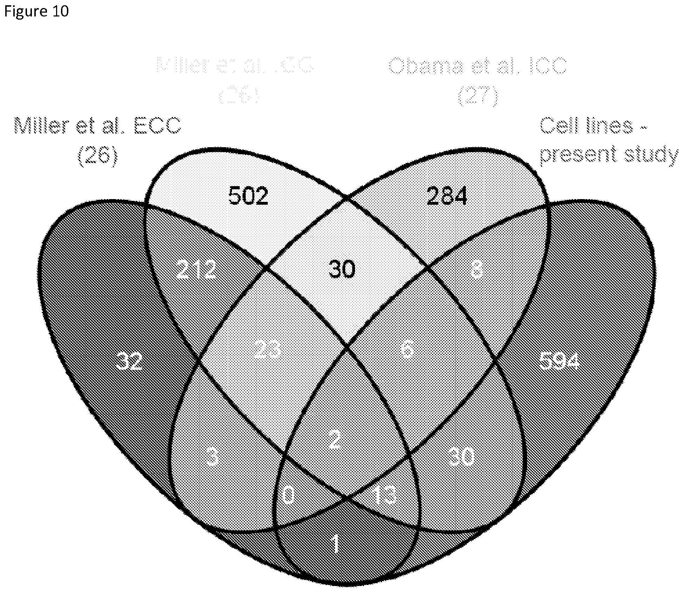

**Figure 10****:** Venn diagram illustrating overlapping de-regulated genes between cancer cell lines and cholangiocarcinomas. Using microarray analyses, sixty genes were identified as up-regulated in CCA cell lines after epigenetic drug treatment, and simultaneously down-regulated in tumor compared to non-malignant controls in previously published datasets. Abbreviations: ICC, intrahepatic cholangiocarcinoma; ECC, extrahepatic cholangiocarcinoma.

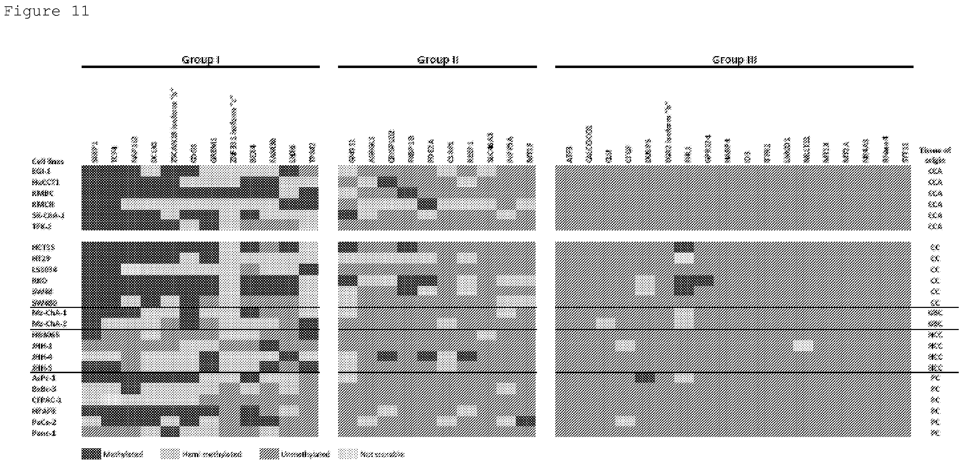

**Figure 11****:** Summary of promoter methylation status in cancer cell lines. Forty loci were analyzed by MSP in cancer cell lines and grouped according to their methylation frequency in CCA cell lines. Group I; frequently methylated (minimum five out of six cell lines), group II; intermediately methylated (from one to four cell lines), group III; unmethylated.

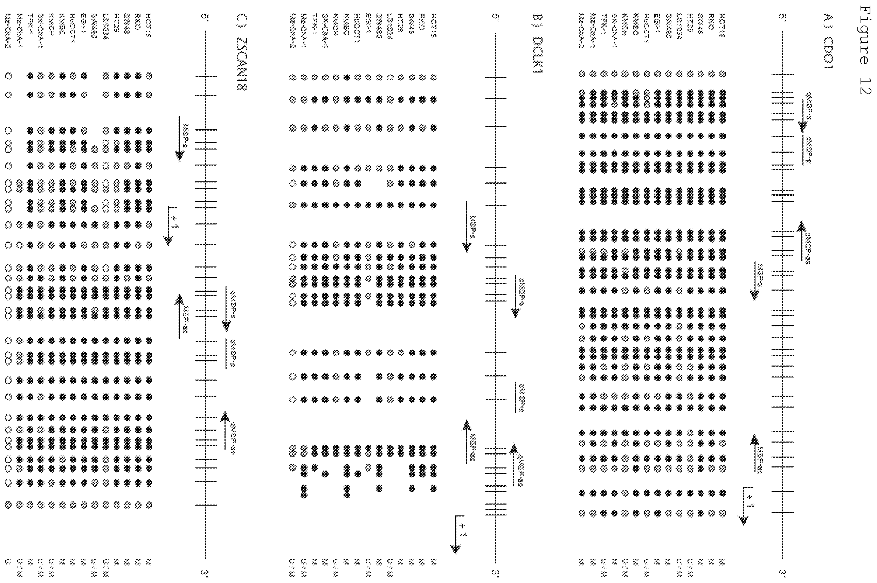

**Figure 12****:** Direct bisulfite sequencing of *CDO1, DCLK1* and *ZSCAN18* verified the methylation status as assessed by MSP. A) *CDO1.* B) *DCLK1.* C) *ZSCAN18.* For all panels, the upper line represents the individual CpG sites (vertical bars) in the fragment amplified by the bisulfite sequencing primers. Transcription start site is denoted by +1 and arrows indicate the location of MSP and the subsequently designed qMSP primers and probe. In the lower part of the panels, dark circles indicate methylated CpGs, grey circles indicate partially methylated CpGs, and white circles indicate unmethylated CpGs. The column on the right side in each panel (M, U/M and U) lists the methylation status as assessed by MSP.

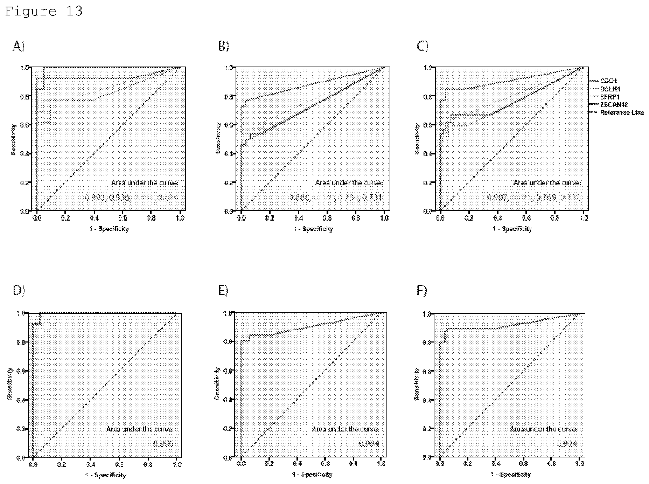

**Figure 13****:** Receiver operating characteristics curves for individual and combined genes in cholangiocarcinomas and non-malignant samples. The panels depict the resulting area under the receiver operating characteristics curve values based on the PMR for the individual biomarkers in the A) fresh frozen sample series, B) archival sample series and C) combined for fresh frozen and archival material. D-F shows the performance of the combined biomarker panel in D) fresh frozen-, E) archival- and F) combined sample sets.

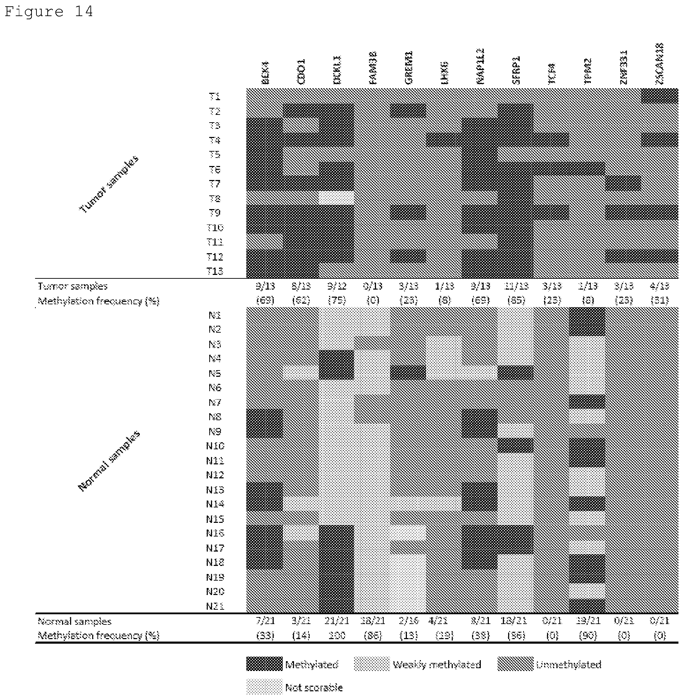

**Figure 14****:** Methylation frequencies in patient material assessed by qualitative methylation-specific polymerase chain reaction (qMSP). The twelve group I genes were investigated in a fresh frozen sample set. Band intensities from the methylated reaction were considerably weaker in non-malignant samples than in carcinomas.

### DEFINITIONS

To facilitate an understanding of the present invention, a number of terms and phrases are defined below:
As used herein, the term "sensitivity" is defined as a statistical measure of performance of an assay (e.g., method, test), calculated by dividing the number of true positives by the sum of the true positives and the false negatives.

As used herein, the term "specificity" is defined as a statistical measure of performance of an assay (e.g., method, test), calculated by dividing the number of true negatives by the sum of true negatives and false positives.

As used herein, the term "informative" or "informativeness" refers to a quality of a marker or panel of markers, and specifically to the likelihood of finding a marker (or panel of markers) in a positive sample.

As used herein, the term "CpG island" refers to a genomic DNA region that contains a high percentage of CpG sites relative to the average genomic CpG incidence (per same species, per same individual, or per subpopulation (e.g., strain, ethnic subpopulation, or the like). Various parameters and definitions for CpG islands exist; for example, in some embodiments, CpG islands are defined as having a GC percentage that is greater than 50% and with an observed/expected CpG ratio that is greater than 60% (Gardiner-Garden et al. (1987) J Mol. Biol. 196:261-282; Baylin et al. (2006) Nat. Rev. Cancer 6:107-116; Irizarry et al. (2009) Nat. Genetics 41:178-186). In some embodiments, CpG islands may have a GC content >55% and observed CpG/expected CpG of 0.65 (Takai et al. (2007) PNAS 99:3740-3745). Various parameters also exist regarding the length of CpG islands. As used herein, CpG islands may be less than 100 bp; 100-200 bp, 200-300 bp, 300-500 bp, 500-750 bp; 750-1000 bp; 100 or more bp in length. In some embodiments, CpG islands show altered methylation patterns relative to controls (e.g., altered methylation in cancer subjects relative to subjects without cancer; tissue-specific altered methylation patterns; altered methylation in biological samples (e.g., tissue, stool, blood, plasma, serum, cells, bile) from subjects with gastrointestinal neoplasia (e.g., colorectal cancer, gastric cancer, pancreatic cancer, liver cancer, cancers of the gall bladder and/ or bile ducts (e.g., cholangiocarcinoma)) relative to subjects without gastrointestinal neoplasia). In some embodiments, altered methylation involves hypermethylation. In some embodiments, altered methylation involves hypomethylation.

As used herein, the term "CpG shore" or "CpG island shore" refers to a genomic region external to a CpG island that is or that has potential to have altered methylation patterns (see, e.g., Irizarry et al. (2009) Nat. Genetics 41:178-186). CpG island shores may show altered methylation patterns relative to controls (e.g., altered methylation in cancer subjects relative to subjects without cancer; tissue-specific altered methylation patterns; altered methylation in biological samples (e.g., stool, tissue, blood, cells, bile) from subjects with gastrointestinal neoplasia (e.g., colorectal cancer, gastric cancer, pancreatic cancer, liver cancer, cancers of the gall bladder and/ or bile ducts (e.g., cholangiocarcinoma)) relative to subjects without gastrointestinal neoplasia). In some embodiments, altered methylation involves hypermethylation. In some embodiments, altered methylation involves hypomethylation. CpG island shores may be located in various regions relative to CpG islands (see, e.g., Irizarry et al. (2009) Nat. Genetics 41;178-186). Accordingly, in some embodiments, CpG island shores are located less than 100 bp; 100-250 bp; 250-500 bp; 500-1000 bp; 1000-1500 bp; 1500-2000 bp; 2000-3000 bp; 3000 bp or more away from a CpG island.

As used herein, the term "epigenetic" refers to a non-sequence-based alteration that is inherited through cell division. For example, in some embodiments, epigenetic changes are altered methylation patters or levels (e.g. hypermethylation).

As used herein the term "methylation state" is a measure of the presence or absence of a methyl modification in one or more CpG sites in at least one nucleic acid sequence. It is to be understood that in some embodiments, the methylation state of one or more CpG sites is determined in multiple copies of a particular gene of interest.

As used herein, the term "methylation level" refers to the amount of methylation in one or more copies of a gene or nucleic acid sequence of interest. The methylation level may be calculated as an absolute measure of methylation within the gene or nucleic acid sequence of interest. Also a "relative methylation level" may be determined as the amount of methylated DNA, relative to the total amount DNA present or as the number of methylated copies of a gene or nucleic acid sequence of interest, relative to the total number of copies of the gene or nucleic acid sequence. Additionally, the "methylation level" can be determined as the percentage of methylated CpG sites within the DNA stretch of interest.

The term methylation level also encompasses the situation wherein one or more CpG site in e.g. the promoter region is methylated but where the amount of methylation is below amplification threshold. Thus methylation level may be an estimated value of the amount of methylation in a gene of interest.

The methylation level of the gene of interest can be 15% to 100%, such as 50% to 100%, more preferably 60%- 100 %, more preferably 70- 100 %, more preferably 80% to 100%, more preferably 90% to 100%. The methylation level of the genes according to the invention can be 80%, 81%, 82%, 83%, 84%, 85%, 86%, 87%, 88%, 89%, 90%, 91%, 92%, 93%, 94%, 95%, 96%, 97%, 98%, 99% or 100%.
As used herein, the term "methylation specific nucleic acid detection sequence" refers to a probe, probes or primers or sets thereof that are used to specifically detect, determine or analyze the methylation status of a target nucleic acid sequence, e.g., the sequences encoding *GLDC, PPP1R14A, CDO1*, *DCLK1, SCAN18* and *ZNF331* (SEQ ID NOs.: 185, 186, 187, 188, 189, and 190, respectively). The detection sequence may be a single probe or comprise multiple probes, such as would be the case for a set of PCR primers specific for the target sequence. Specific examples of "methylation specific nucleic acid detection sequences" include, but are not limited to, MSP and qMSP pimer sets.

As used herein, the term "metastasis" is meant to refer to the process in which cancer cells originating in one organ or part of the body relocate to another part of the body and continue to replicate. Metastasized cells subsequently form tumors which may further metastasize. Metastasis thus refers to the spread of cancer from the part of the body where it originally occurs to other parts of the body. As used herein, the term "metastasized gastrointestinal cancer cells" is meant to refer to gastrointestinal cancer cells that have metastasized; gastrointestinal cancer cells localized in a part of the body other than the gastrointestinal system.

As used herein, "an individual is suspected of being susceptible to metastasized gastrointestinal cancer" is meant to refer to an individual who is at an above-average risk of developing metastasized gastrointestinal cancer. Examples of individuals at a particular risk of developing gastrointestinal cancer are those whose family medical history indicates above average incidence of gastrointestinal cancer among family members and/or those who have already developed gastrointestinal cancer and have been effectively treated who therefore face a risk of relapse and recurrence. Other factors which may contribute to an above-average risk of developing metastasized gastrointestinal cancer which would thereby lead to the classification of an individual as being suspected of being susceptible to metastasized gastrointestinal cancer may be based upon an individual's specific genetic, medical and/or behavioral background and characteristics.

The term "neoplasm" as used herein refers to any new and abnormal growth of tissue. Thus, a neoplasm can be a premalignant neoplasm or a malignant neoplasm. The term "neoplasm-specific marker" refers to any biological material that can be used to indicate the presence of a neoplasm. Examples of biological materials include, without limitation, nucleic acids, polypeptides, carbohydrates, fatty acids, cellular components (e.g., cell membranes and mitochondria), and whole cells. The term "aero-digestive system neoplasm-specific marker" refers to any biological material that can be used to indicate the presence of a gastrointestinal system neoplasm (e.g., a premalignant gastrointestinal system neoplasm; a malignant gastrointestinal system neoplasm). Examples of gastrointestinal system-specific markers include, but are not limited to, *GLDC, PPP1R14A, CDO1*, *DCLK1, SCAN18* and *ZNF331.*

As used herein, the term "amplicon" refers to a nucleic acid generated using primer pairs. The amplicon is typically single-stranded DNA (e.g., the result of asymmetric amplification), however, it may be RNA or dsDNA.

The term "amplifying" or "amplification" in the context of nucleic acids refers to the production of multiple copies of a polynucleotide, or a portion of the polynucleotide, typically starting from a small amount of the polynucleotide (*e.g*., a single polynucleotide molecule), where the amplification products or amplicons are generally detectable. Amplification of polynucleotides encompasses a variety of chemical and enzymatic processes. The generation of multiple DNA copies from one or a few copies of a target or template DNA molecule during a polymerase chain reaction (PCR) or a ligase chain reaction (LCR; see, e.g., U.S. Patent No. 5,494,810) are forms of amplification. Additional types of amplification include, but are not limited to, allele-specific PCR (see, e.g., U.S. Patent No. 5,639,611), assembly PCR (see, e.g., U.S. Patent No. 5,965,408), helicase-dependent amplification (see, e.g., U.S. Patent No. 7,662,594), hot-start PCR (see, e.g., U.S. Patent Nos. 5,773,258 and 5,338,671), intersequence-specfic PCR, inverse PCR (see, e.g., Triglia, et al. (1988) Nucleic Acids Res., 16:8186), ligation-mediated PCR (see, e.g., Guilfoyle, R. et al., Nucleic Acids Research, 25:1854-1858 (1997); U.S. Patent No. 5,508,169), methylation-specific PCR (see, e.g., Herman, et al., (1996) PNAS 93(13) 9821-9826), miniprimer PCR, multiplex ligation-dependent probe amplification (see, e.g., Schouten, et al., (2002) Nucleic Acids Research 30(12): e57), multiplex PCR (see, e.g., Chamberlain, et al., (1988) Nucleic Acids Research 16(23) 11141-11156; Ballabio, et al., (1990) Human Genetics 84(6) 571-573; Hayden, et al., (2008) BMC Genetics 9:80), nested PCR, overlap-extension PCR (see, e.g., Higuchi, et al., (1988) Nucleic Acids Research 16(15) 7351-7367), real time PCR (see, e.g., Higuchi, etl al., (1992) Biotechnology 10:413-417; Higuchi, et al., (1993) Biotechnology 11:1026-1030), reverse transcription PCR (see, e.g., Bustin, S.A. (2000) J. Molecular Endocrinology 25:169-193), solid phase PCR, thermal asymmetric interlaced PCR, and Touchdown PCR (see, e.g., Don, et al., Nucleic Acids Research (1991) 19(14) 4008; Roux, K. (1994) Biotechniques 16(5) 812-814; Hecker, et al., (1996) Biotechniques 20(3) 478-485). Polynucleotide amplification also can be accomplished using digital PCR (see, e.g., Kalinina, et al., Nucleic Acids Research. 25; 1999-2004, (1997); Vogelstein and Kinzler, Proc Natl Acad Sci USA. 96; 9236-41, (1999); International Patent Publication No. WO05023091A2; US Patent Application Publication No. 20070202525).

As used herein, the terms "complementary" or "complementarity" are used in reference to polynucleotides (*i.e.,* a sequence of nucleotides) related by the base-pairing rules. For example, the sequence "5'-A-G-T-3'," is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

As used herein, the term "primer" refers to an oligonucleotide, whether occurring naturally as in a purified restriction digest or produced synthetically, that is capable of acting as a point of initiation of synthesis when placed under conditions in which synthesis of a primer extension product that is complementary to a nucleic acid strand is induced (e.g., in the presence of nucleotides and an inducing agent such as a biocatalyst (e.g., a DNA polymerase or the like) and at a suitable temperature and pH). The primer is typically single stranded for maximum efficiency in amplification, but may alternatively be double stranded. If double stranded, the primer is generally first treated to separate its strands before being used to prepare extension products. In some embodiments, the primer is an oligodeoxyribonucleotide. The primer is sufficiently long to prime the synthesis of extension products in the presence of the inducing agent. The exact lengths of the primers will depend on many factors, including temperature, source of primer and the use of the method. In certain embodiments, the primer is a capture primer.

As used herein, the term "nucleic acid molecule" refers to any nucleic acid containing molecule, including but not limited to, DNA or RNA. The term encompasses sequences that include any of the known base analogs of DNA and RNA including, but not limited to, 4 acetylcytosine, 8-hydroxy-N6-methyladenosine, aziridinylcytosine, pseudoisocytosine, 5-(carboxyhydroxyl-methyl) uracil, 5-fluorouracil, 5-bromouracil, 5-carboxymethylaminomethyl-2-thiouracil, 5-carboxymethyl-aminomethyluracil, dihydrouracil, inosine, N6-isopentenyladenine, 1-methyladenine, 1-methylpseudo-uracil, 1-methylguanine, 1-methylinosine, 2,2-dimethyl-guanine, 2-methyladenine, 2-methylguanine, 3-methyl-cytosine, 5-methylcytosine, N6-methyladenine, 7-methylguanine, 5-methylaminomethyluracil, 5-methoxy-amino-methyl-2-thiouracil, beta-D-mannosylqueosine, 5'-methoxycarbonylmethyluracil, 5-methoxyuracil, 2-methylthio-N- isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

As used herein, the term "nucleobase" is synonymous with other terms in use in the art including "nucleotide," "deoxynucleotide," "nucleotide residue," "deoxynucleotide residue," "nucleotide triphosphate (NTP)," or deoxynucleotide triphosphate (dNTP).

An "oligonucleotide" refers to a nucleic acid that includes at least two nucleic acid monomer units (e.g., nucleotides), typically more than three monomer units, and more typically greater than ten monomer units. The exact size of an oligonucleotide generally depends on various factors, including the ultimate function or use of the oligonucleotide. To further illustrate, oligonucleotides are typically less than 200 residues long (e.g., between 15 and 100), however, as used herein, the term is also intended to encompass longer polynucleotide chains. Oligonucleotides are often referred to by their length. For example a 24 residue oligonucleotide is referred to as a "24-mer". Typically, the nucleoside monomers are linked by phosphodiester bonds or analogs thereof, including phosphorothioate, phosphorodithioate, phosphoroselenoate, phosphorodiselenoate, phosphoroanilothioate, phosphoranilidate, phosphoramidate, and the like, including associated counterions, e.g., H⁺, NH₄⁺, Na⁺, and the like, if such counterions are present. Further, oligonucleotides are typically single-stranded. Oligonucleotides are optionally prepared by any suitable method, including, but not limited to, isolation of an existing or natural sequence, DNA replication or amplification, reverse transcription, cloning and restriction digestion of appropriate sequences, or direct chemical synthesis by a method such as the phosphotriester method of Narang et al. (1979) Meth Enzymol. 68: 90-99; the phosphodiester method of Brown et al. (1979) Meth Enzymol. 68: 109-151; the diethylphosphoramidite method of Beaucage et al. (1981) Tetrahedron Lett. 22: 1859-1862; the triester method of Matteucci et al. (1981) J Am Chem Soc. 103:3185-3191; automated synthesis methods; or the solid support method of U.S. Pat. No. 4,458,066, entitled "PROCESS FOR PREPARING POLYNUCLEOTIDES," issued Jul. 3, 1984 to Caruthers et al., or other methods known to those skilled in the art.

A "sequence" of a biopolymer refers to the order and identity of monomer units (e.g., nucleotides, etc.) in the biopolymer. The sequence (e.g., base sequence) of a nucleic acid is typically read in the 5' to 3' direction.

A "subsequence" is any portion of an entire sequence. Thus, a subsequence refers to a consecutive sequence of amino acids or nucleic acids which is part of a longer sequence of nucleic acids (e.g. polynucleotide).

As used herein, the term "subject" refers to any animal (*e.g*., a mammal), including, but not limited to, humans, non-human primates, rodents, and the like, which is to be the recipient of a particular treatment. Typically, the terms "subject" and "patient" are used interchangeably herein in reference to a human subject.

As used herein, the term "non-human animals" refers to all non-human animals including, but are not limited to, vertebrates such as rodents, non-human primates, ovines, bovines, ruminants, lagomorphs, porcines, caprines, equines, canines, felines, aves, etc.

The term "gene" refers to a nucleic acid (*e.g.,* DNA) sequence that comprises coding sequences necessary for the production of a polypeptide, RNA (*e.g*., including but not limited to, mRNA, tRNA and rRNA) or precursor. The polypeptide, RNA, or precursor can be encoded by a full length coding sequence or by any portion of the coding sequence so long as the desired activity or functional properties (*e.g*., enzymatic activity, ligand binding, signal transduction, etc.) of the full-length or fragment are retained. The term also encompasses the coding region of a structural gene and the including sequences located adjacent to the coding region on both the 5' and 3' ends for a distance of about 1 kb on either end such that the gene corresponds to the length of the full-length mRNA. The sequences that are located 5' of the coding region and which are present on the mRNA are referred to as 5' untranslated sequences. The sequences that are located 3' or downstream of the coding region and that are present on the mRNA are referred to as 3' untranslated sequences. The term "gene" encompasses both cDNA and genomic forms of a gene. A genomic form or clone of a gene contains the coding region interrupted with non-coding sequences termed "introns" or "intervening regions" or "intervening sequences". Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) processed transcript. The mRNA functions during translation to specify the sequence or order of amino acids in a nascent polypeptide.

The term "locus" as used herein refers to a nucleic acid sequence on a chromosome or on a linkage map and includes the coding sequence as well as 5' and 3' sequences involved in regulation of the gene.

### DETAILED DESCRIPTION OF THE INVENTION

The present invention relates to methods and biomarkers (e.g., epigenetic biomarkers) for detection of gastrointestinal cancers (e.g., colorectal cancer, gastric cancer, pancreatic cancer, liver cancer, cancers of the gall bladder and/ or bile ducts (e.g., cholangiocarcinoma)) in biological samples (e.g., tissue samples, stool samples, blood samples, plasma samples, serum samples, cell samples, gall samples, bile samples).

Impaired epigenetic regulation is as common as gene mutations in human cancer. These mechanisms lead to quantitative and qualitative gene expression changes causing a selective growth advantage, which may result in cancerous transformation. Aberrantly hypermethylated CpG islands in the gene promoter associated with transcriptional inactivation are among the most frequent epigenetic changes in cancer. Since early detection of disease can result in improved clinical outcome for most types of cancer, the identification of cancer-associated aberrant gene methylation represents promising novel biomarkers. For cancers in the gastrointestinal system, including, e.g., colorectal cancer, gastric cancer, pancreatic cancer, liver cancer, cancers of the gall bladder and/ or bile ducts (e.g., cholangiocarcinoma), initial studies have identified the presence of aberrantly methylated DNA in patient blood and feces. Genes aberrantly hypermethylated in high frequencies already among benign tumours and only rarely in normal mucosa would be good candidate diagnostic biomarkers due to the potential clinical benefit of early detection of high risk adenomas as well as of low risk stages of carcinomas.

In general, however, the sensitivity and specificity of existing early markers for cancers in the gastrointestinal system remain poor. Consequently, there is a need for individual genes or a panel of genes in which each gene is hypermethylated at a high frequency and specificity in cancers. In particular, there is a need for a gene panel which is useful in non-invasive techniques, such as techniques involving the use of stool samples, or in techniques which may be used on sample material which is easily obtained, such as blood or blood products or mucous. Such a gene panel would greatly improve the possibility for early detection of these cancers.

Accordingly, embodiments of the present invention provide compositions and methods comprising detection of the methylation status of *GLDC* and one or more of, *CDO1, DCLK1, ZSCAN18* and *ZNF331,* and optionally *PPP1R14A.* The compositions and methods find use in research, screening and clinical applications (e.g., related to colorectal cancer, gastric cancer, pancreatic cancer, liver cancer, cancers of the gall bladder and/ or bile ducts (e.g., cholangiocarcinoma)).

Therefore, using a highly sensitive automated and quantitative screening methodology for detecting cancer-related promoter methylation, novel epi-biomarkers and epi-biomarker panels were identified that are frequently and specifically methylated in gastrointestinal cancer.

While the present invention exemplifies several markers specific for detecting gastrointestinal cancer, any marker that is correlated with the presence or absence of gastrointestinal cancer may be used. A marker, as used herein, includes, for example, nucleic acid(s) whose production or mutation or lack of production is characteristic of a gastrointestinal neoplasm. Depending on the particular set of markers employed in a given analysis, the statistical analysis will vary. For example, where a particular combination of markers is highly specific for gastrointestinal cancer, the statistical significance of a positive result will be high. It may be, however, that such specificity is achieved at the cost of sensitivity (e.g., a negative result may occur even in the presence of gastrointestinal cancer). By the same token, a different combination may be very sensitive (e.g., few false negatives, but has a lower specificity).

Particular combinations of markers may be used that show optimal function with different ethnic groups or sex, different geographic distributions, different stages of disease, different degrees of specificity or different degrees of sensitivity. Particular combinations may also be developed which are particularly sensitive to the effect of therapeutic regimens on disease progression. Subjects may be monitored after a therapy and/or course of action to determine the effectiveness of that specific therapy and/or course of action.

The methods of the present invention are not limited to particular indicators of gastrointestinal neoplasm. In some embodiments, indicators of gastrointestinal neoplasm include, for example, epigenic alterations. Epigenetic alterations include but are not limited to DNA methylation (e.g., CpG methylation). In some embodiments, the level (e.g., frequency, score) of methylation (e.g., hypermethylation relative to a control, hypomethylation relative to a control) is determined without limitation to the technique used for such determining. Methods of the present invention are not limited to particular epigenetic alterations (e.g., DNA methylation) (e.g., CpG methylation) (e.g., CpG methylation in coding or regulatory regions *of GLDC,* and of one or more of, *CDO1*, *DCLK1, SCAN18* or *ZNF331,* and optionally *PPP1R14A).* Altered methylation may occur in, for example, CpG islands; CpG island shores; or regions other than CpG islands or CpG island shores.

In certain embodiments, methods, kits, and systems of the present invention involve determination of methylation state of a locus of interest (e.g., in human DNA) (e.g., in human DNA extracted from a stool sample, from a gastrointestinal tissue sample, from a tumor sample, from a blood sample, from a serum sample, from a plasma sample, from a cell sample, from a bile sample, etc). Any appropriate method can be used to determine whether a particular DNA is hypermethylated or hypomethylated. Standard PCR techniques, for example, can be used to determine which residues are methylated, since unmethylated cytosines converted to uracil are replaced by thymidine residues during PCR. PCR reactions can contain, for example, 10 µL of captured DNA that either has or has not been treated with sodium bisulfite, IX PCR buffer, 0.2 mM dNTPs, 0.5 µM sequence specific primers (e.g., primers flanking a CpG island or CpG shore within the captured DNA), and 5 units DNA polymerase (e.g., Amplitaq DNA polymerase from PE Applied Biosystems, Norwalk, CT) in a total volume of 50 µl. A typical PCR protocol can include, for example, an initial denaturation step at 94°C for 5 min, 40 amplification cycles consisting of 1 minute at 94°C, 1 minute at 60°C, and 1 minute at 72°C, and a final extension step at 72°C for 5 minutes.

To analyze which residues within a captured DNA are methylated, the sequences of PCR products corresponding to samples treated with and without sodium bisulfite can be compared. The sequence from the untreated DNA will reveal the positions of all cytosine residues within the PCR product. Cytosines that were unmethylated will be converted to thymidine residues in the sequence of the bisulfite-treated DNA, while residues that were methylated will be unaffected by bisulfite treatment.

Some embodiments of the present invention utilize next generation or high-throughput sequencing. A variety of nucleic acid sequencing methods are contemplated for use in the methods of the present disclosure including, for example, chain terminator (Sanger) sequencing, dye terminator sequencing, and high-throughput sequencing methods. Many of these sequencing methods are well known in the art. See, e.g., Sanger et al., Proc. Natl. Acad. Sci. USA 74:5463-5467 (1997); Maxam et al., Proc. Natl. Acad. Sci. USA 74:560-564 (1977); Drmanac, et al., Nat. Biotechnol. 16:54-58 (1998); Kato, Int. J. Clin. Exp. Med. 2:193-202 (2009); Ronaghi et al., Anal. Biochem. 242:84-89 (1996); Margulies et al., Nature 437:376-380 (2005); Ruparel et al., Proc. Natl. Acad. Sci. USA 102:5932-5937 (2005), and Harris et al., Science 320:106-109 (2008); Levene et al., Science 299:682-686 (2003); Korlach et al., Proc. Natl. Acad. Sci. USA 105:1176-1181 (2008); Branton et al., Nat. Biotechnol. 26(10): 1146-53 (2008); Eid et al., Science 323:133-138 (2009). Similarly, in some embodiments, methods of the present invention involve the determination (e.g., assessment, ascertaining, quantitation) of methylation level of an indicator of gastrointestinal neoplasm (e.g., the methylation level of a CpG island or CpG shore in the coding or regulatory region of a gene locus) in a sample (e.g., a DNA sample extracted from stool, bile or blood). A skilled artisan understands that an increased, decreased, informative, or otherwise distinguishably different methylation level is articulated with respect to a reference (e.g., a reference level, a control level, a threshold level, or the like). For example, the term "elevated methylation" as used herein with respect to the methylation status (e.g., CpG DNA methylation) of a gene locus (e.g., *GLDC, PPP1R14A, CDO1*, *DCLK1, ZSCAN18* or *ZNF331*) is any methylation level that is above a median methylation level in a sample from a random population of mammals (e.g., a random population of 10, 20, 30, 40, 50, 100, or 500 mammals) that do not have a gastrointestinal neoplasm (e.g., gastrointestinal cancer). Elevated levels of methylation can be any level provided that the level is greater than a corresponding reference level. For example, an elevated methylation level of a locus of interest (e.g., *GLDC,* and one or more of, *CDO1, DCLK1, SCAN18* and *ZNF331,* and optionally *PPP1R14A)* methylation can be 0.5, 1, 2, 3, 4, 5, 6, 7, 8, 9, 10, or more fold greater than the reference level methylation observed in a normal sample. It is noted that a reference level can be any amount. The term "elevated methylation score" as used herein with respect to detected methylation events in a matrix panel of particular nucleic acid markers is any methylation score that is above a median methylation score in a sample from a random population of mammals (e.g., a random population of 10, 20, 30, 40, 50, 100, or 500 mammals) that do not have a gastrointestinal neoplasm (e.g., colorectal cancer or cholangiocarcinoma). An elevated methylation score in a matrix panel of particular nucleic acid markers can be any score provided that the score is greater than a corresponding reference score. For example, an elevated score of methylation in a locus of interest (e.g., *GLDC,* and one or more of, *CDO1, DCLK1,* ZSCAN18 and *ZNF331,* and optionally *PPP1R14A)* can be 0.5, 1, 2, 3, 4, 5, 6, 7, 8, 9, 10, or more fold greater than the reference methylation score observed in a normal sample. It is noted that a reference score can be any amount.

The methods are not limited to a particular type of mammal. In some embodiments, the mammal is a human. In some embodiments, the gastrointestinal neoplasm is premalignant. In some embodiments, the gastrointestinal neoplasm is malignant. In some embodiments, the gastrointestinal neoplasm is gastrointestinal (e.g., colorectal cancer, gastric cancer, pancreatic cancer, liver cancer, cancers of the gall bladder and/ or bile ducts (e.g., cholangiocarcinoma)) without regard to stage of the cancer (e.g., stage I, II, III, or IV).

The present invention also provides methods and materials to assist medical or research professionals in determining whether or not a mammal has a gastrointestinal neoplasm (e.g., colorectal cancer, gastric cancer, pancreatic cancer, liver cancer, cancers of the gall bladder and/ or bile ducts (e.g., cholangiocarcinoma)). Medical professionals can be, for example, doctors, nurses, medical laboratory technologists, and pharmacists. Research professionals can be, for example, principle investigators, research technicians, postdoctoral trainees, and graduate students. A professional can be assisted by (1) determining the ratio of particular markers in a sample, and (2) communicating information about the ratio to that professional, for example.

After the level (score, frequency) of particular markers in a stool, blood, serum, bile or plasma sample is reported, a medical professional can take one or more actions that can affect patient care. For example, a medical professional can record the results in a patient's medical record. In some cases, a medical professional can record a diagnosis of a gastrointestinal neoplasia, or otherwise transform the patient's medical record, to reflect the patient's medical condition. In some cases, a medical professional can review and evaluate a patient's entire medical record, and assess multiple treatment strategies, for clinical intervention of a patient's condition. In some cases, a medical professional can record a prediction of tumor occurrence with the reported indicators. In some cases, a medical professional can review and evaluate a patient's entire medical record and assess multiple treatment strategies, for clinical intervention of a patient's condition.

A medical professional can initiate or modify treatment of a gastrointestinal neoplasm after receiving information regarding the level (score, frequency) associated with markers in a patient's stool, blood, serum, bile or plasma sample. In some cases, a medical professional can compare previous reports and the recently communicated level (score, frequency) of markers, and recommend a change in therapy. In some cases, a medical professional can enroll a patient in a clinical trial for novel therapeutic intervention of gastrointestinal neoplasm. In some cases, a medical professional can elect waiting to begin therapy until the patient's symptoms require clinical intervention.

A medical professional can communicate the assay results to a patient or a patient's family. In some cases, a medical professional can provide a patient and/or a patient's family with information regarding gastrointestinal neoplasia, including treatment options, prognosis, and referrals to specialists, e.g., oncologists and/or radiologists. In some cases, a medical professional can provide a copy of a patient's medical records to communicate assay results to a specialist. A research professional can apply information regarding a subject's assay results to advance gastrointestinal neoplasm research. For example, a researcher can compile data on the assay results, with information regarding the efficacy of a drug for treatment of gastrointestinal neoplasia to identify an effective treatment. In some cases, a research professional can obtain assay results to evaluate a subject's enrollment, or continued participation in a research study or clinical trial. In some cases, a research professional can classify the severity of a subject's condition, based on assay results. In some cases, a research professional can communicate a subject's assay results to a medical professional. In some cases, a research professional can refer a subject to a medical professional for clinical assessment of gastrointestinal neoplasia, and treatment thereof. Any appropriate method can be used to communicate information to another person (e.g., a professional). For example, information can be given directly or indirectly to a professional. For example, a laboratory technician can input the assay results into a computer-based record. In some cases, information is communicated by making a physical alteration to medical or research records. For example, a medical professional can make a permanent notation or flag a medical record for communicating a diagnosis to other medical professionals reviewing the record. In addition, any type of communication can be used to communicate the information. For example, mail, e-mail, telephone, and face-to-face interactions can be used. The information also can be communicated to a professional by making that information electronically available to the professional. For example, the information can be communicated to a professional by placing the information on a computer database such that the professional can access the information. In addition, the information can be communicated to a hospital, clinic, or research facility serving as an agent for the professional.

It is noted that a single sample can be analyzed for one gastrointestinal neoplasm-specific marker or for multiple gastrointestinal neoplasm-specific markers. In preferred embodiments, a single sample is analyzed for multiple gastrointestinal neoplasm-specific markers, for example, using multi-marker assays. In addition, multiple samples can be collected for a single mammal and analyzed as described herein. In some embodiments, a sample is split into first and second portions, where the first portion undergoes cytological analysis and the second portion undergoes further purification or processing (e.g., sequence-specific capture step(s) (e.g., for isolation of specific markers for analysis of methylation levels). In some embodiments, the sample undergoes one or more preprocessing steps before being split into portions. In some embodiments, the sample is treated, handled, or preserved in a manner that promotes DNA integrity and/or inhibits DNA degradation (e.g., through use of storage buffers with stabilizing agents (e.g., chelating agents, DNase inhibitors) or handling or processing techniques that promote DNA integrity (e.g., immediate processing or storage at low temperature (e.g., -80 degrees C)).

Some embodiments of the invention provides a diagnostic kit for the diagnosis or screening of cancer comprising one or reagents for detection of methylation status *of GLDC* and one or more genes selected from, *CDO1*, *DCLK1, SCAN18* and *ZNF331,* and optionally *PPP1R14A.* For example, in some embodiments, the reagents comprise nucleic acids (e.g., oligonucleotides, primers, probes, etc.). In some embodiments, kits provide reagents useful, necessary or sufficient for detecting methylation status and/or providing a diagnosis or prognosis.

The diagnostic kits may further comprise any reagent or media necessary, sufficient or useful to perform analyses, such as PCR analyses, such as methylation specific polymerase chain reaction (MSP) sequence analyses, bisulphite treatment, bisulphite sequencing, electrophoresis, pyrosequencing, mass spectrometry and sequence analyses by restriction digestion, next generation sequencing, quantitative and/or qualitative methylation, pyrosequencing, Southern blotting, restriction landmark genome scanning (RLGS), single nucleotide primer extension, CpG island microarray, SNUPE, COBRA, mass spectrometry, by use of methylation specific restriction enzymes or by measuring the expression level of said genes. In particular, the kit may further comprise one or more components selected from the group consisting of: deoxyribonucleoside triphosphates, buffers, stabilizers, thermostable DNA polymerases, restriction endonucleases (including methylation specific endonucleases), and labels (including fluorescent, chemiluminescent and radioactive labels). The diagnostic assay according to the invention may further comprise one or more reagents required for isolation of DNA.

In some embodiments, the kits of the present invention include a means for containing the reagents in close confinement for commercial sale such as, e.g., injection or blow-molded plastic containers into which the desired reagent are retained. Other containers suitable for conducting certain steps of the disclosed methods also may be provided.

In some embodiments, the methods disclosed herein are useful in monitoring the treatment of gastrointestinal neoplasia (e.g., colorectal cancer, gastric cancer, pancreatic cancer, liver cancer, cancers of the gall bladder and/ or bile ducts (e.g., cholangiocarcinoma)). For example, in some embodiments, the methods may be performed immediately before, during and/or after a treatment to monitor treatment success. In some embodiments, the methods are performed at intervals on disease free patients to ensure treatment success.

Also described is a variety of computer-related embodiments. Specifically, described are computer programming for analyzing and comparing a pattern of gastrointestinal neoplasm-specific marker detection results in a sample obtained from a subject to, for example, a library of such marker patterns known to be indicative of the presence or absence of a gastrointestinal neoplasm, or a particular stage or gastrointestinal neoplasm.

Described is computer programming for analyzing and comparing a first and a second pattern of gastrointestinal neoplasm-specific marker detection results from a sample taken at least two different time points. The first pattern may be indicative of a pre-cancerous condition and/or low risk condition for gastrointestinal cancer and/or progression from a pre-cancerous condition to a cancerous condition. In such instances, the comparing provides for monitoring of the progression of the condition from the first time point to the second time point.

Described is further computer programming for analyzing and comparing a pattern of gastrointestinal neoplasm-specific marker detection results from a sample to a library of gastrointestinal neoplasm-specific marker patterns known to be indicative of the presence or absence of a gastrointestinal cancer, wherein the comparing provides, for example, a differential diagnosis between a benign gastrointestinal neoplasm, and an aggressively malignant gastrointestinal neoplasm (e.g., the marker pattern provides for staging and/or grading of the cancerous condition).

The methods and systems described herein can be implemented in numerous ways. The methods may involve use of a communications infrastructure, for example the internet. The methods and systems described herein can be implemented as a combination of hardware and software. The software can be implemented as an application program tangibly embodied on a program storage device, or different portions of the software implemented in the user's computing environment (e.g., as an applet) and on the reviewer's computing environment, where the reviewer may be located at a remote site (e.g., at a service provider's facility).

For example, during or after data input by the user, portions of the data processing can be performed in the user-side computing environment. For example, the user-side computing environment can be programmed to provide for defined test codes to denote platform, carrier/diagnostic test, or both; processing of data using defined flags, and/or generation of flag configurations, where the responses are transmitted as processed or partially processed responses to the reviewer's computing environment in the form of test code and flag configurations for subsequent execution of one or more algorithms to provide a results and/or generate a report in the reviewer's computing environment.

The application program for executing the algorithms described herein may be uploaded to, and executed by, a machine comprising any suitable architecture. In general, the machine involves a computer platform having hardware such as one or more central processing units (CPU), a random access memory (RAM), and input/output (I/O) interface(s). The computer platform also includes an operating system and microinstruction code. The various processes and functions described herein may either be part of the microinstruction code or part of the application program (or a combination thereof) which is executed via the operating system. In addition, various other peripheral devices may be connected to the computer platform such as an additional data storage device and a printing device.

As a computer system, the system generally includes a processor unit. The processor unit operates to receive information, which generally includes test data (e.g., specific gene products assayed), and test result data (e.g., the pattern of gastrointestinal neoplasm-specific marker detection results from a sample). This information received can be stored at least temporarily in a database, and data analyzed in comparison to a library of marker patterns known to be indicative of the presence or absence of a pre-cancerous condition, or known to be indicative of a stage and/or grade of gastrointestinal cancer.

Part or all of the input and output data can also be sent electronically; certain output data (e.g., reports) can be sent electronically or telephonically (e.g., by facsimile, e.g., using devices such as fax back). Exemplary output receiving devices can include a display element, a printer, a facsimile device and the like. Electronic forms of transmission and/or display can include email, interactive television, and the like. In some embodiments, all or a portion of the input data and/or all or a portion of the output data (e.g., usually at least the library of the pattern of gastrointestinal neoplasm-specific marker detection results known to be indicative of the presence or absence of a pre-cancerous condition) are maintained on a server for access, e.g., confidential access. The results may be accessed or sent to professionals as desired.

A system for use in the methods described herein generally includes at least one computer processor (e.g., where the method is carried out in its entirety at a single site) or at least two networked computer processors (e.g., where detected marker data for a sample obtained from a subject is to be input by a user (e.g., a technician or someone performing the assays)) and transmitted to a remote site to a second computer processor for analysis (e.g., where the pattern of gastrointestinal neoplasm-specific marker) detection results is compared to a library of patterns known to be indicative of the presence or absence of a pre-cancerous condition), where the first and second computer processors are connected by a network, e.g., via an intranet or internet). The system can also include a user component(s) for input; and a reviewer component(s) for review of data, and generation of reports, including detection of a pre-cancerous condition, staging and/or grading of a gastrointestinal neoplasm, or monitoring the progression of a pre-cancerous condition or a gastrointestinal neoplasm. Additional components of the system can include a server component(s); and a database(s) for storing data (e.g., as in a database of report elements, e.g., a library of marker patterns known to be indicative of the presence or absence of a pre-cancerous condition and/or known to be indicative of a grade and/or a stage of a gastrointestinal neoplasm, or a relational database (RDB) which can include data input by the user and data output. The computer processors can be processors that are typically found in personal desktop computers (e.g., IBM, Dell, Macintosh), portable computers, mainframes, minicomputers, or other computing devices.

The input components can be complete, stand-alone personal computers offering a full range of power and features to run applications. The user component usually operates under any desired operating system and includes a communication element (e.g., a modem or other hardware for connecting to a network), one or more input devices (e.g., a keyboard, mouse, keypad, or other device used to transfer information or commands), a storage element (e.g., a hard drive or other computer-readable, computer-writable storage medium), and a display element (e.g., a monitor, television, LCD, LED, or other display device that conveys information to the user). The user enters input commands into the computer processor through an input device. Generally, the user interface is a graphical user interface (GUI) written for web browser applications.

The server component(s) can be a personal computer, a minicomputer, or a mainframe, or distributed across multiple servers (e.g., as in cloud computing applications) and offers data management, information sharing between clients, network administration and security. The application and any databases used can be on the same or different servers. Other computing arrangements for the user and server(s), including processing on a single machine such as a mainframe, a collection of machines, or other suitable configuration are contemplated. In general, the user and server machines work together to accomplish the processing of the present invention.

Where used, the database(s) is usually connected to the database server component and can be any device which will hold data. For example, the database can be any magnetic or optical storing device for a computer (e.g., CDROM, internal hard drive, tape drive). The database can be located remote to the server component (with access via a network, modem, etc.) or locally to the server component.

Where used in the system and methods, the database can be a relational database that is organized and accessed according to relationships between data items. The relational database is generally composed of a plurality of tables (entities). The rows of a table represent records (collections of information about separate items) and the columns represent fields (particular attributes of a record). In its simplest conception, the relational database is a collection of data entries that "relate" to each other through at least one common field.

Additional workstations equipped with computers and printers may be used at point of service to enter data and, in some embodiments, generate appropriate reports, if desired. The computer(s) can have a shortcut (e.g., on the desktop) to launch the application to facilitate initiation of data entry, transmission, analysis, report receipt, etc. as desired.

In certain embodiments, the present invention provides methods for obtaining a subject's risk profile for developing gastrointestinal neoplasm (e.g., colorectal cancer, gastric cancer, pancreatic cancer, liver cancer, cancers of the gall bladder and/ or bile ducts (e.g., cholangiocarcinoma)). In some embodiments, such methods involve obtaining a stool, bile or blood sample from a subject (e.g., a human at risk for developing gastrointestinal cancer; a human undergoing a routine physical examination), detecting the presence, absence, or level (e.g., methylation frequency or score) of one or more markers specific for a gastrointestinal neoplasm in or associated with the stool, blood, plasma, bile or serum sample (e.g., specific for a gastrointestinal neoplasm) in the stool, blood, plasma, bile or serum sample, and generating a risk profile for developing gastrointestinal neoplasm (e.g., colorectal cancer, gastric cancer, pancreatic cancer, liver cancer, cancers of the gall bladder and/ or bile ducts (e.g., cholangiocarcinoma)) based upon the detected level (score, frequency) or presence or absence of the indicators of gastrointestinal neoplasia. For example, in some embodiments, a generated risk profile will change depending upon specific markers and detected as present or absent or at defined threshold levels. The present invention is not limited to a particular manner of generating the risk profile. A processor (e.g., computer) can be used to generate such a risk profile. The processor can use an algorithm (e.g., software) specific for interpreting the presence and absence of specific exfoliated epithelial markers as determined with the methods of the present invention. The presence and absence of specific markers as determined with the methods of the present invention can be imputed into such an algorithm, and the risk profile is reported based upon a comparison of such input with established norms (e.g., established norm for pre-cancerous condition, established norm for various risk levels for developing gastrointestinal cancer, established norm for subjects diagnosed with various stages of gastrointestinal cancer).The risk profile may indicate a subject's risk for developing gastrointestinal cancer or a subject's risk for re-developing gastrointestinal cancer. In some embodiments, the risk profile indicates a subject to be, for example, a very low, a low, a moderate, a high, and a very high chance of developing or re-developing gastrointestinal cancer. In some embodiments, a health care provider (e.g., an oncologist) will use such a risk profile in determining a course of treatment or intervention (e.g., biopsy, wait and see, referral to an oncologist, referral to a surgeon, etc.).

The following examples are provided in order to demonstrate and further illustrate certain preferred embodiments and aspects of the present invention and are not to be construed as limiting the scope thereof.

### Example 1

### Identification of novel epigenetic biomarkers in colorectal cancer, GLDC and PPP1R14A

### Materials:

A panel of twenty colon cancer cell lines was analysed in this project. The panel included eleven microsatellite stable (MSS; ALA, Colo320, EB, FRI, HT29, IS1, IS2, IS3, LS1034, SW480, V9P) and nine microsatellite unstable (MSI; Co115 HCT15, HCT116, LoVo, LS174T, RKO, SW48, TC7, TC71) cell lines, thereby representing both of the phenotypical subgroups of colorectal cancer. Forty-seven primary colorectal carcinoma samples, including 27 MSS and 20 MSI tumours, were subjected to DNA promoter methylation analysis in the present study. Twenty-four of the samples derived from a series which was collected at seven hospitals in the South-Eastern part of Norway from 1987-1989. The remaining 23 samples were collected at Aker University Hospital from 2005-2007. Also included in the present project were 49 normal colorectal mucosa samples derived from deceased colorectal cancer-free individuals.

### Methods:

### Genome-wide gene expression analysis

The AB1700 microarray platform was utilised to analyse the gene expression of colon cancer cell lines before and after treatment with AZA and TSA, identifying novel gene targets epigenetically inactivated in colorectal tumorigenesis. Six cell lines were analysed, including three MSI (SW48, RKO, HCT15) and three MSS (SW480, LS1034, HT29) cell lines. Only genes up-regulated four or more times after treatment in at least five of the six cell lines analysed were chosen for further investigation. In order to increase the likelihood of selecting true epigenetic targets, the gene expression of the same genes were analysed in primary colorectal carcinomas and normal tissue samples, using the same microarray platform. Only those genes that responded in cell lines and simultaneously were down-regulated in the carcinomas as compared to normal tissue were chosen for further analysis. The selection process for the discovery of new, hypermethylated genes is summarised in figure 1.

### Methylation-specific experimental analyses:

Because loss of gene expression often is associated with aberrant methylation of promoter CpG islands, suitable target genes for DNA methylation analysis should contain a CpG island in their promoter region. Therefore, CpG Island Searcher was applied to analyse the candidate genes for the presence of one or more islands. Bisulfite treatment, qualitative and quantitative methylation-specific polymerase chain reaction as well as direct bisulfite sequencing was performed on the most promising genes from the genome wide gene expression studies.

### Statistics:

All 2 x 2 contingency tables were analysed using a two-sided Fisher's exact test, whereas a two-sided Pearson Chi-square test was used on 2 x 3 and 2 x 4 contingency tables. P values less than or equal to 0.05 (5%) were considered significant. Binary regression analyses were used to examine possible association between DNA methylation and patient age. Receiver Operating Characteristics (ROC) curves for individual genes were created using PMR values and tissue type (carcinoma and normal) as input. All calculations are derived from two-tailed statistical tests using the SPSS 16.0 software.

### Results:

### Qualitative and quantitative methylation analyses of candidate genes in cell lines and tissue samples.

Among 20 colon cancer cell lines, *BNIP3, CBS, DDX43, GLDC, IQCG, PEG10, PPP1R14A, RASSF4, RBP7* and *WDR21B* were methylated in 70%, 74%, 89%, 75%, 0%, 90%, 95%, 5%, 45% and 100%, respectively. The six most frequently hypermethylated genes were subjected to methylation analysis in a pilot of primary colorectal carcinomas and normal mucosa samples. *BNIP3, GLDC,* and *PPP1R14A* were methylated in 8, 14 and 11 of the carcinomas and in 1, 0 and 0 of the normal samples, respectively.

*GLDC* and *PPP1R14A* were selected for further investigation by real-time quantitative MSP in a larger series of malignant and normal colorectal tissue samples.

### Quantitative methylation profiles of GLDC and PPP1R14A.

In the larger validation study, promoter hypermethylation for *GLDC* and *PPP1R14A* were found in 60% and 57% of the primary colorectal tumours. Samples were scored as positive for methylation if the PMR value was > 3.5 for *PPP1R14A* and 2.5 for *GLDC.* With these cut-off values, none of the normal mucosa samples for either gene were scored as methylated, resulting in 100% specificity for both assays.

ROC curve analysis was applied to provide a statistical method to assess the diagnostic accuracy of the genes as biomarkers. *GLDC* had a sensitivity of 64% and a specificity of 100%, with an area under the curve (AUC) of 0.819 (P = 7 · 10⁻⁸). *PPP1R14A* had a sensitivity of 57.5% and a specificity of 100%, with an AUC of 0.792 (P = 8.59 · 10⁻⁷). The ROC curves are visualised in figure 2 and figure 3.

### Concordance of conventional MSP and quantitative real-time MSP

The results of qMSP analyses were compared with those obtained by conventional MSP in colon cancer cell lines (n = 20), primary tumours (n = 11) and normal tissue samples (n = 8). While conventional MSP scores samples as methylated, partially methylated or unmethylated for cell lines, and methylated or unmethylated for tissue samples, qMSP data gives a quantitative measurement of DNA methylation levels ranging from 0 to 100 (Figure 4). The cut-off values of 2.5 for *GLDC* and 3.5 for *PPP1R14A* resulted in good concordance between data obtained from qMSP and conventional MSP analyses. For *GLDC,* 39/39 (100%) of the samples were concordant (P = 0.000). *PPP1R14A,* however, had one sample which was scored as unmethylated from qualitative, gel-based MSP and as methylated from the quantitative real-time MSP analysis. Consequently, the methylation status was in agreement for 38/39 (97%) of the samples (P = 2 · 10⁻⁹). The results are illustrated in figure 4 and figure 5 and summarised in table 1 and table 2.

**Table 1. Concordance of classification of the GLDC status by the two methods.**

| | Quantitative real-time MSP with cut-off = 2.5 | | |
|---|---|---|---|
| Conventional MSP | Unmethylated | Methylated | Total |
| Unmethylated | 15 | 0 | 15 |
| Methylated | 0 | 24 | 24 |
| Total | 15 | 24 | 39 |

**Table 2. Concordance of classification of the PPP1R14A status by the two methods.**

| | Quantitative real-time MSP with cut-off = 3.5 | | |
|---|---|---|---|
| Conventional MSP | Unmethylated | Methylated | Total |
| Unmethylated | 11 | 1 | 12 |
| Methylated | 0 | 27 | 27 |
| Total | 11 | 28 | 39 |

### Bisulfite sequencing confirms the promoter methylation status of GLDC and PPP1R14A

Bisulfite sequencing of *GLDC* and *PPP1R14A* in colon cancer cell lines showed that all non-CpG cytosines were fully converted to thymine. These results, along with detailed sequencing results and MSP status are shown in figure 6 and figure 7. In general, the majority of the cell lines that were scored as fully methylated by MSP, were also fully methylated from the bisulfite sequencing analyses.

### Association of DNA tumour methylation with genetic and clinico-pathological features

DNA methylation status for *GLDC* and *PPP1R14A* were compared with genetic and clinico-pathological features of the tumours. Promoter methylation was independent of tumour stage, age and gender of the patients. *PPP1R14A* was significantly more methylated in tumours with microsatellite instability and thus in tumours located on the right side of the colon.

### Example 2

### Identification of novel epigenetic biomarkers in gastrointestinal cancer (cholangiocarcinomas), CDO1, DCLK1, ZNF33, and ZSCAN.

Cholangiocarcinoma (CCA) is notoriously difficult to diagnose and displays a high mortality due to late clinical presentation. CpG island promoter hypermethylation is associated with cancer development. We aimed to identify novel epigenetic biomarkers with a potential to improve the diagnostic accuracy of CCA. Microarray analyses performed in CCA cell lines were compared with previously published expression profiles in tumors compared to non-malignant controls. Common candidate genes were interrogated for their promoter methylation status in cancer cell lines from the gastrointestinal tract, using a qualitative methylation specific polymerase chain reaction (MSP). Frequently methylated genes were subjected to quantitative methylation specific polymerase chain reaction (qMSP) in two CCA sample series, including fresh frozen (n = 34) and formalin-fixed paraffin embedded tissues (n = 59). From microarray analyses, 43 genes with a CpG island in their promoter region responded to epigenetic treatment and were simultaneously down-regulated in cholangiocarcinomas compared with non-malignant controls. We identified twelve genes as frequently hypermethylated in CCA cell lines. Of these, *CDO1,* DCLK1, SFRP1, and ZSCAN18 displayed high methylation frequencies also in tumors. Non-malignant samples were unmethylated for the same genes. The combined sensitivity of at least one positive among the four markers was 100% for fresh frozen tumors and 81 % for archival tumors, and the specificity was 100% for both series. The resulting area under the receiver operating characteristics curve was 0.996 and 0.904, respectively. In conclusion, the novel epigenetic biomarker panel showed high sensitivity and specificity for CCA.

This example describes applications of an epigenome-wide approach (25) to identify a list of DNA methylation candidate genes in CCA. Potential target genes have been subjected to qualitative and quantitative promoter methylation analyses in cancer cell lines and patient material and three novel potential biomarkers for CCA have been identified.

### Materials and Methods

### Experimental approach

The step-wise experimental approach used in the present study is illustrated in Figure 8. Briefly, genes responding to epigenetic drug treatment in cancer cell lines were compared with a list of genes down-regulated in CCA samples versus non-malignant tissue. Responding and simultaneously down-regulated genes harboring a CpG island in the promoter region were subjected to qualitative methylation analysis in cancer cell lines. The most frequently methylated genes were further subjected to qualitative and subsequently quantitative methylation analyses in patient material.

### Cancer cell lines

Twenty-four cancer cell lines were analyzed, including bile duct (n=6; EGI-1, HuCCT1, KMBC, KMCH-1, SK-ChA-1, and TFK-1), colon (n=6; HCT15, HT29, LS1034, RKO, SW48, and SW480), pancreas (n=6; AsPc-1, BxBc-3, CFPAC-1, HPAFII, PaCa-2, and Panc-1), liver (n=4; HB8065, JHH-1, JHH-4, and JHH-5), and gallbladder (n=2; Mz-ChA-1 and Mz-ChA-2) cancers. The cell lines were cultured according to the manufacturer's guidelines, and the conditions are summarized in Table 5. All cell lines were harvested before reaching confluence.

Cell line authentication was performed using the AmpFLSTR Identifiler PCR Amplification Kit (Applied Biosystems, CA, USA) according to manufacturer's protocol. Samples were run on an AB Prism 3730 and analyzed in GeneMapper (Applied Biosystems). For commercially available cancer cell lines the genotypes were compared with previously published data. Results for non-commercial cell lines are listed in Table 6.

The six cholangiocarcinoma cell lines were subjected to treatment with a combination of the demethylating drug 5-aza-2'deoxycytidine (1 mM for 72 hours; Sigma-Aldrich Company Ltd., Dorset, UK) and the histone deacetylase trichostatin A (1 mM added the last 12 hours; Sigma-Aldrich, Dorset, UK). Untreated controls were cultured in parallel.

### Patient samples

### Fresh frozen material

Thirteen bile duct carcinomas were derived from patients undergoing surgery at Oslo University Hospital, Rikshospitalet, and Imperial College, London, UK. Samples were snap-frozen immediately after surgery and stored at -80°C. Carcinomas were embedded in Tissue-Tek (Sakura Finetek, CA, USA), according to the manufacturer's protocol, and subsequently subjected to cryo-sectioning and haematoxylin and eosin staining before they were evaluated by an expert pathologist. All carcinoma samples included (n=13) displayed >5% tumor cells. A cohort of 21 samples from non-malignant liver diseases, including autoimmune hepatitis (n=2), alcohol related liver disease (n=5), cryptogenic cirrhosis (n=1), hemochromatosis (n=1), primary biliary cirrhosis (n=3), and primary sclerosing cholangitis (n=9) were used as non-malignant controls. In addition, tissue from dissected bile ducts from six cancer free patients with primary sclerosing cholangitis was included as a separate sample set. The non-malignant biopsies were obtained from the peripheral liver region of patients undergoing surgery at Oslo University Hospital, Rikshospitalet.

### Archival material

The archival sample series comprised 26 CCA- and 33 non-malignant formalin-fixed, paraffin-embedded samples obtained from the Department of pathology, Oslo University Hospital. The tissue specimens were routinely stained with haematoxylin and erythrosine. All carcinoma samples included (n=26) displayed >5% tumor cells. Tissue from non-malignant liver disease patients, including autoimmune hepatitis (n=4), alcohol related liver disease (n=1), primary biliary cirrhosis (n=4), and primary sclerosing cholangitis (n=21), as well as disease free patients (n=3) were included as non-malignant tissue controls. Liver biopsies were obtained from extrahepatic bile duct, hilum, and peripheral liver region.

### Gene expression microarray analyses of cancer cell lines

RNA from six CCA cell lines and their epigenetic drug treated counterparts were subjected to gene expression microarray analyses (Applied Biosystems). Genes up-regulated at least two-fold after 5-aza-2'deoxycytidine and trichostatin A treatment in a minimum of four of the analyzed cancer cell lines, were considered to be potential targets for DNA methylation.

### Microarray gene expression data sets from cholangiocarcinoma patients and healthy controls

In order to increase the likelihood of identifying putative hypermethylated genes in CCA, the gene list generated from the microarray approach in CCA cell lines was compared with gene lists generated from published and available gene expression microarray data comparing CCAs with non-malignant controls (26;27). Only responding genes from the cell line approach that were simultaneously down-regulated in CCA compared to non-malignant controls, were considered methylation candidates and subjected to further analyses.

### DNA promoter methylation analyses

Candidate genes were interrogated for CpG island in the promoter region. Bisulfite treatment was performed prior to DNA methylation analyses. Qualitative methylation-specific polymerase chain reaction (MSP) was performed in cancer cell lines. Genes methylated in a minimum of five CCA cell lines were further subjected to MSP in fresh frozen tissue samples (n=34). The best performing genes (*CDO1*, *DCLK1,* and *ZSCAN18*) were subjected to direct promoter bisulfite sequencing. Primer sequences and -location, amplicon length, MgCl₂ concentration, and annealing temperatures are listed in Table 1. The methylation status of the three abovementioned genes in addition to the previously reported *SFRP1* was finally assessed in both fresh frozen- and archival patient material using quantitative methylation-specific polymerase chain reaction (qMSP). Sequences are listed in Table 2. Table 3 provides detailed information about the genes. Figure 9 shows sequences of the genes in Table 3.

### Nucleic acid isolation

For cancer cell lines, DNA was isolated using a standard phenol/chloroform procedure and total RNA was isolated using Trizol (Invitrogen, Carlsbad, CA) according to manufacturer's instructions. RNA quality was assessed using a 2100 bioanalyzer (Agilent Technologies, Palo Alto, CA). For fresh frozen samples, DNA was extracted from approximately 25 mg of tissue using AllPrep DNA/RNA kit (Qiagen Inc, Valencia, CA). For archival material DNA was isolated from five sections of paraffin embedded tissue á 20 µm, using QIAamp DNA kit (Qiagen). Nucleic acid concentration was determined using the ND-1000 Nanodrop (NanoDrop Technologies, Wilmington, DE).

### Description of the gene expression microarray analysis

Standard one-round amplification was performed using the NanoAmp RT-IVT Labeling Kit (Applied Biosystems) according to the manufacturers' protocol. Briefly, cDNA was synthesized from one µg total RNA in an oligo dT primed reaction. Labeled complementary RNA (cRNA) was obtained from double-stranded cDNA in the presence of digoxigenin (DIG)-UTP in an *in vitro* labeling reaction. Samples were hybridized to gene expression microarrays (Human Genome Survey Microarray V2.0, Applied Biosystems) containing 32,878 oligonucleotide probes representing 29,098 individual genes. Chemoluminescense was measured using the AB1700 Chemoluminescense Analyzer (Applied Biosystems) after incubating the array with alkalic phosphatase-linked digoxygenin antibody. Probe signals were post-processed and quantile normalized using the R-script (R 2.5.0) "ABarray" file 1.2.0 and Bioconductor (www.bioconductor.org) and further analyzed in Excel (Microsoft office, version 2007). Array elements with a signal-to-noise ratio <3, and/or a flag value >8191 were discarded from further analysis.

### CpG island search

The candidate genes for DNA methylation were interrogated for the presence of a CpG island in the promoter region using the CpG island searcher algorithm (48). Default criteria were applied for CpG island detection (49).

### Bisulfite treatment of DNA

Bisulfite treatment of DNA results in the conversion of unmethylated but not methylated cytosines to uracil (50;51). DNA (1.3 µg) was bisulfite treated using the EpiTect bisulfite kit (Qiagen) according to manufacturers' protocol. Desulfonation and washing steps were performed using a QIAcube (Qiagen) and the bisulfite treated DNA was eluted in 40 µl elution buffer.

### Qualitative methylation-specific polymerase chain reaction (MSP)

MSP primers were designed in close proximity of the transcription start site, according to the Human Genome browser (genome.ucsc.edu), using the Methyl Primer Express Software v1.0 (Applied Biosystems). Primers were purchased from MedProbe (MedProbe, Oslo, Norway). Two pairs of primers were used to amplify the loci of interest, one specific for methylated- and one specific for unmethylated template. The MSP mixture contained one unit HotStarTaq DNA polymerase (Qiagen), 1×PCR buffer (Tris-Cl, KCl(NH₄)₂SO₄, 15 mM MgCl₂; pH 8.7; Qiagen), deoxynucleotide triphosphates (each at 2.5 mM), primers (10 µm each) and 24 ng of template DNA in a total volume of 25 µl. All MSP reactions were optimized with respect to MgCl₂ concentration, annealing temperature and elongation time (Table 2). Thermal cycling (Tetrad 2, Bio-Rad, CA, USA) was performed at 95C° for 15 minutes and 35 subsequent cycles (30 seconds at 95°C, 30 seconds at variable annealing temperatures (Table 2), and 30-60 seconds elongation at 72°C) with a final extension of seven minutes at 72°C. Human placental DNA (Sigma Chemical Co, St Louis, MO) treated *in vitro* with *SssI* methyltransferase (New England Biolabs Inc., Beverly, MA) was used as positive control for the methylated reaction and DNA from normal lymphocytes was used as a positive control for the unmethylated reaction. Water, replacing the template was used as a negative control in both reactions. MSP products were mixed with five µl gel loading buffer (1 x TAE buffer, 20% Ficoll; Sigma Aldrich, and 0.1% xylen cyanol; Sigma Aldrich), and loaded onto a 2% agarose gel (BioRad, Hercules, CA, USA) in 1 x TAE and ethidium bromide (Sigma Aldrich). Electrophoresis was performed at 200V for 25 minutes before MSP products were visualized by UV irradiation using a Gel Doc XR+ (BioRad). All results were verified by a second independent round of MSP and scored independently by two authors. In cases of discrepancies, a third round of analysis was performed. Scoring was performed using the methylated band intensities of positive controls as reference. In tissue samples, the methylated band intensities were scored on a scale from 0-5. Tumor samples were only considered hypermethylated when band intensities were equal to or stronger than three. For non-malignant samples, methylated band intensities equal to or stronger than three were scored as hypermethylated, while methylated band intensities equal to or below two were determined weakly methylated. Only non-malignant samples not displaying MSP fragments for the methylated reaction were scored as unmethylated.

### Direct DNA bisulfite sequencing

Fourteen cancer cell lines (colon, n=6; cholangiocarcinoma, n=6 and gallbladder carcinoma, n=2) were subjected to bisulfite sequencing. DNA bisulfite primers were designed using the Methyl Primer Express Software v1.0 (Applied Biosystems) to cover the area amplified by the MSP. The experimental procedure has been published previously (52). Briefly, fragments were amplified for 35 cycles using HotStarTaq (Qiagen) and purified using ExoSAP-IT, according to the protocol of the manufacturer (GE Healthcare, USB Corporation, Ohio, USA). Sequencing was performed using the dGTP BigDye Terminator Cycle Sequencing Ready Reaction kit on the AB Prism 3730 (Applied Biosystems). Bisulfite-treated completely methylated DNA (CpGenome Universal Methylated DNA, Millipore, MA, USA) and DNA from normal lymphocytes served as positive and negative controls, respectively. In accordance with a previous report (53), the amount of methylcytosine of each CpG site was calculated by the peak height ratio of the cytosine signal versus the sum of cytosine and thymine signal. Methylation of individual CpG sites was determined after the following criteria: 0-0.2 was unmethylated, 0.21-0.8 was partially methylated and 0.81-1.0 was scored as hypermethylated.

### Quantitative methylation-specific polymerase chain reaction (qMSP)

Primers and probes were designed using Primer Express v3.0 and purchased from Medprobe and Applied Biosystems, respectively. All qMSP reactions were carried out in triplicates in 384-well plates. The total reaction volume was 20 µl, and included 0.9 µM of each primer, 0.2 µM probes (labelled with 6-FAM and a non-fluorescent quencher), 30 ng bisulfite treated template and 1x TaqMan Universal PCR master mix NoAmpErase UNG (Applied Biosystems). Amplification was performed at 95°C for 15 minutes before 45 cycles of 15 seconds at 95°C and 1 minute elongation at 60 °C, using the TaqMan 7900HF (Applied Biosystems). Bisulfite-converted completely methylated DNA (Millipore) served as a positive control and was also used to generate a standard curve by 1:5 serial dilutions (32.5 - 0.052 ng). The ALU-C4 gene (54) was used for normalization. In addition, bisulfite treated and untreated DNA from normal lymphocytes, and water blanks were used as negative controls.

All samples were censored after cycle 35 (in accordance with Applied Biosystems protocol) and the median quantity value was used for further processing. Briefly, percent methylated reference (PMR) was calculated by dividing the GENE:ALU ratio in the sample by the GENE:ALU ratio of the positive control (completely methylated DNA) and multiplying by 100. To ensure high specificity, individual fixed thresholds were established for each assay (one for fresh frozen material and one for archival material), using the integer above the highest PMR value across the normal samples. Samples with higher PMR values than the thresholds were scored as methylation positive. For *CDO1*, *DCLK1, SFRP1,* and *SCAN18* the thresholds for the fresh frozen sample series were 1, 2, 1, and 1, respectively. For the archival sample series, thresholds were set at 2, 2, 5, and 3.

### Statistical analyses

For statistical analyses, PASW 18.0 (SPSS, Chicago, IL, USA) was used. Pearson's chi-square and Fishers exact tests were used for categorical variables. Student T-test and Mann-Whitney U test were used to investigate potential relationships between tumor DNA methylation and patient age. To evaluate the suitability of the methylated target genes to separate CCA from non-malignant controls, receiver operating characteristics curves were generated using the individual percentage methylated reference (PMR) values. Similarly, the combined performance of the candidate genes as a panel was evaluated using the sum of the PMR values. *P*-values were considered statistically significant at P<0.05.

| | | **Table 1: Primer sequences, fragment size and PCR conditions** | | | | | | | | |
|---|---|---|---|---|---|---|---|---|---|---|
| **Primer set** | **Sense primer** | **SEQ ID NO.** | **Antisense primer** | **SEQ ID NO.** | **Frg. Size, bp** | **Fragment location** | **An. Tem p** | **Mg Cl₂** | **El. Time (sec)** | **Accesion number** |
| ASRGL1_MSP_M | | 1 | | 2 | 91 | +60 to +151 | 57 | 1.5 | 30 | NM_001083926 |
| ASRGL1_MSP_U | | 3 | | 4 | 94 | +57 to +151 | 57 | 1.5 | 30 | |
| ATF3_MSP_M | | 5 | | 6 | 174 | -221 to - 47 | 53 | 1.5 | 30 | NM_001040619 |
| ATF3_MSP_U | | 7 | | 8 | 180 | -224 to - 44 | 53 | 1.5 | 30 | |
| BEX4_MSP_M | | 9 | | 10 | 132 | -90 to +42 | 55 | 1.5 | 30 | NM_001080425 |
| BEX4_MSP_U | | 11 | | 12 | 138 | -93 to +45 | 55 | 1.5 | 30 | |
| CALCOCO1_MSP_M | | 13 | | 14 | 116 | -118 to - 2 | 55 | 1.5 | 30 | NM_020898 |
| CALCOCO1_MSP_U | | 15 | | 16 | 121 | -121 to 0 | 55 | 1.5 | 30 | |
| CDO1_MSP_M | | 17 | | 18 | 145 | -153 to - 8 | 53 | 1.5 | 30 | NM_001801 |
| CDO1_MSP_U | | 19 | | 20 | 148 | -156 to - 8 | 53 | 1.5 | 30 | |
| CDO1_BS | | 21 | | 22 | 350 | -280 to +70 | 49 | 1.7 | 30 | |
| CLU_MSP_M | | 23 | | 24 | 165 | -147 to +18 | 52 | 2.4 | 30 | NM_001831 |
| CLU_MSP_U | | 25 | | 26 | 170 | -150 to +20 | 52 | 2.4 | 30 | |
| CRISPLD2_MSPM | | 27 | | 28 | 172 | -178 to - 6 | 52 | 1.5 | 30 | NM_031476 |
| CRISPLD2_MSP _U | | 29 | | 30 | 178 | -181 to - 3 | 52 | 1.5 | 30 | |
| CSRP1_MSP_M | | 31 | | 32 | 147 | -126 to +21 | 55 | 1.7 | 30 | NM_004078 |
| CSRP1_MSP_U | | 33 | | 34 | 149 | -128 to +21 | 56 | 1.5 | 30 | |
| CTGF_MSP_M | | 35 | | 36 | 122 | -34 to +88 | 56 | 2.5 | 30 | NM_001901 |
| CTGF_M SP_U | | 37 | | 38 | 124 | -36 to +88 | 56 | 2.5 | 30 | |
| DCLK1_MSP_M | | 39 | | 40 | 108 | -127 to - 19 | 53 | 1.5 | 30 | NM_004734 |
| DCLK1_MSP_U | | 41 | | 42 | 108 | -127 to - 19 | 53 | 1.5 | 30 | |
| DCLK1_BS | | 43 | | 44 | 271 | -247 to +24 | 57 | 1.5 | 30 | |
| DUSP5_MSP_M | | 45 | | 46 | 198 | -192 to +6 | 52 | 1.5 | 30 | NM_004419 |
| DUSP5_MSP_U | | 47 | | 48 | 198 | -192 to +6 | 52 | 1.5 | 30 | |
| EGR2_isoform "a"_MSP_M | | 49 | | 50 | 104 | -108 to - 4 | 52 | 2.0 | 30 | NM_001136177 |
| EGR2_isoform "a"_MSP_U | | 51 | | 52 | 110 | -111 to - 1 | 52 | 1.5 | 30 | |
| FAM3B_MSP_M | | 53 | | 54 | 137 | -120 to +17 | 53 | 1.5 | 30 | NM_058186 |
| FAM3B_MSP_U | | 55 | | 56 | 137 | -120 to +17 | 53 | 1.5 | 30 | |
| FHL1_MSP_M | | 57 | | 58 | 165 | -160 to +5 | 57 | 1.5 | 30 | NM_001449 |
| FHL1_MSP_U | | 59 | | 60 | 171 | -163 to +8 | 57 | 1.5 | 30 | |
| FKBP1 _MSP_M | | 61 | | 62 | 126 | -158 to - 32 | 55 | 2.0 | 30 | NM_054033 |
| FKBP1B_MSP_U | | 63 | | 64 | 130 | -161 to - 31 | 52 | 1.5 | 30 | |
| GNG11_MSP_M | | 65 | | 66 | 112 | -48 to +64 | 56 | 1.5 | 30 | NM_004126 |
| GNG11_MSP_U | | 67 | | 68 | 116 | -50 to +66 | 56 | 1.5 | 30 | |
| GPR124_MSP_M | | 69 | | 70 | 119 | -124 to - 5 | 55 | 2.5 | 30 | NM_032777 |
| GPR124_MSP_U | | 71 | | 72 | 125 | -127 to - 2 | 55 | 1.5 | 30 | |
| | | | | | | | | | | |
| GREM1_MSP_M | | 73 | | 74 | 172 | -198 to - 26 | 53 | 1.5 | 30 | NM_013372 |
| GREM1_MSP_U | | 75 | | 76 | 175 | -201 to - 26 | 53 | 1.5 | 30 | |
| HABP4_MSP_M | | 77 | | 78 | 149 | -115 to +34 | 58 | 1.5 | 30 | NM_014282 |
| HABP4_MSP_U | | 79 | | 80 | 151 | -116 to +35 | 59 | 1.5 | 30 | |
| ID3_MSP_M | | 81 | | 82 | 173 | -32 to +141 | 55 | 1.5 | 30 | NM_002167 |
| ID3_MSP_U | | 83 | | 84 | 176 | -35 to +141 | 55 | 1.5 | 30 | |
| INPP5A_MSP_M | | 85 | | 86 | 113 | -20 to +93 | 50 | 1.5 | 30 | NM_005539 |
| INPP5A_MSP_U | | 87 | | 88 | 113 | -20 to +93 | 50 | 1.5 | 30 | |
| ITPR1_MSP_M | | 89 | | 90 | 148 | -146 to +2 | 55 | 2.5 | 30 | NM_002222 |
| ITPR1_MSP_U | | 91 | | 92 | 154 | -149 to +5 | 55 | 2.0 | 30 | |
| LHX6_MSP_M | | 93 | | 94 | 100 | -69 to +31 | 54 | 1.5 | 30 | NM_014368 |
| LHX6_MSP_U | | 95 | | 96 | 106 | -72 to +34 | 54 | 1.5 | 30 | |
| LMCD1_MSP_M | | 97 | | 98 | 165 | -176 to - 11 | 55 | 1.5 | 30 | NM_014583 |
| LMCD1_MSP_U | | 99 | | 100 | 171 | -179 to - 8 | 55 | 1.5 | 30 | |
| MLLT11MSP_M | | 101 | | 102 | 110 | -118 to - 8 | 51 | 1.8 | 30 | NM_006818 |
| MLLT11_MSP_U | | 103 | | 104 | 116 | -121 to - 5 | 52 | 1.5 | 30 | |
| MT1F_MSP_M | | 105 | | 106 | 147 | -110 to +37 | 55 | 1.5 | 30 | NM_005949 |
| MT1F_M SP_U | | 107 | | 108 | 147 | -110 to +37 | 55 | 1.5 | 30 | |
| MT1X_MSP_M | | 109 | | 110 | 129 | -136 to - 7 | 55 | 1.5 | 30 | NM_005952 |
| MT1X_MSP_U | | 111 | | 112 | 131 | -137 to - 6 | 55 | 1.5 | 30 | |
| MT2A_MSP_M | | 113 | | 114 | 132 | -108 to - 240 | 55 | 1.5 | 30 | NM_005953 |
| MT2A_MSP_U | | 115 | | 116 | 132 | -108 to - 240 | 55 | 1.5 | 30 | |
| NAP1L2_MSP_M | | 117 | | 118 | 116 | +8 to +124 | 56 | 1.5 | 30 | NM_021963 |
| NAP1L2_MSP_U | | 119 | | 120 | 122 | +5 to +127 | 56 | 1.5 | 30 | |
| NR4A3_MSP_M | | 121 | | 122 | 142 | -126 to +16 | 52 | 1.5 | 30 | NM_173198 |
| NR4A3_MSP_U | | 123 | | 124 | 148 | -129 to +19 | 52 | 1.5 | 30 | |
| PDE2A_MSP_M | | 125 | | 126 | 161 | +10 to +171 | 53 | 1.8 | 30 | NM_001143839 |
| PDE2A_MSP_U | | 127 | | 128 | 165 | +8 to +173 | 53 | 1.8 | 30 | |
| REEP1_MSP_M | | 129 | | 130 | 149 | -162 to - 13 | 55 | 2.5 | 30 | NM_001164732 |
| REEP1_MSP_U | | 131 | | 132 | 152 | -165 to - 13 | 55 | 2.5 | 30 | |
| RNASE4_MSP_M | | 133 | | 134 | 101 | -143 to - 42 | 53 | 2.5 | 30 | NM_002937 |
| RNASE4_ MSP_U | | 135 | | 136 | 107 | -146 to - 39 | 53 | 1.5 | 30 | |
| SFRP1_MSP_M | | 137 | | 138 | 141 | -138 to +3 | 45 | 1.5 | 30 | NM_003012 |
| SFRP1_MSP_U | | 139 | | 140 | 144 | -141 to +3 | 45 | 1.5 | 30 | |
| SLC46A3_MSP_M | | 141 | | 142 | 151 | -152 to - 1 | 57 | 1.5 | 30 | NM_181785 |
| SLC46A3_MSP_U | | 143 | | 144 | 155 | -154 to +1 | 58 | 1.5 | 30 | |
| SYT11_MSP_M | | 145 | | 146 | 158 | -222 to - 64 | 50 | 1.8 | 30 | NM_152280 |
| SYT11_MSP_U | | 147 | | 148 | 164 | -225 to - 61 | 50 | 1.8 | 30 | |
| TCF4_MSP_M* | | 149 | | 150 | 258 | +322 to +580 | 57 | 1.5 | 60 | NM_00108396 2 |
| TCF4_M | | 151 | | 152 | 259 | +321 to | 57 | 1.5 | 60 | |
| SP_U* | | | | | | +580 | | | | |
| TPM2_MSP_M | | 153 | | 154 | 152 | -156 to - 4 | 61 | 1.5 | 30 | NM_001145822 |
| TPM2_MSP_U | | 155 | | 156 | 155 | -158 to - 3 | 61 | 1.5 | 30 | |
| ZNF331_isoform "c"_MSP_M | | 157 | | 158 | 143 | -120 to +23 | 55 | 1.7 | 30 | NM_018555 |
| ZNF331_isoform "c"_MSP_U | | 159 | | 160 | 143 | -120 to +23 | 55 | 1.5 | 30 | |
| ZSCAN1 8_isoform "b"_MSP_M | | 161 | | 162 | 131 | -52 to +79 | 55 | 1.8 | 30 | NM_023926 |
| ZSCAN1 8_isofor m "b"_MSP_U | | 163 | | 164 | 131 | -55 to +79 | 55 | 1.5 | 30 | |
| ZSCAN18_BS | | 165 | | 166 | 302 | -106 to +196 | 59 | 1.5 | 30 | |
| | | From hg18 to hg19, the transcription start point of *DCLK1* NM_004734 was moved 50 bp upstream. The MSP primers were originally designed to be located -177 to -69 relative to the transcription start site. | | | | | | | | |
| | | * Primer sequences and amplification conditions were obtained from Kim and colleagues (46). | | | | | | | | |

| | | | **Table 2: Assays used for quantitative methylation-specific polymerase chain reaction (qMSP)** | | | | |
|---|---|---|---|---|---|---|---|
| **Assay** | **Sense primer** | **SEQ ID NO.** | **Antisense primer** | **SEQ ID NO.** | **Probe** | **SEQ ID NO.** | **Frg. Size** (**bp**) |
| ALU qMSP | | 167 | | 168 | | 169 | 98 |
| CDO1 qMSP | | 170 | | 171 | | 172 | 101 |
| DCLK1 qMSP | | 173 | | 174 | | 175 | 86 |
| SFRP1 qMSP* | | 176 | | 177 | | 178 | 70 |
| ZSCA N18 qMSP | | 179 | | 180 | | 181 | 84 |
| | | | * qMSP assay was modified from Rawson and colleagues (47). | | | | |

**Table 3: Detailed information about the described genes**

| **HGNC gene symbol** | **Entrez gene ID** | **Ensembl gene ID** | **Aliases** | **Approved gene name** | **SEQ ID NO** |
|---|---|---|---|---|---|
| **GLDC** | 2731 | ENSG00000178445 | GCSP, NKH | glycine dehydrogenase (decarboxylating) | 185 |
| **PPP1R14A** | 94274 | ENSG00000167641 | CPI-17 | protein phosphatase 1, regulatory (inhibitor) subunit 14A | 186 |
| **CDO1** | 1036 | ENSG00000129596 | | cysteine dioxygenase, type I | 187 |
| **DCLK1** | 9201 | ENSG00000133083 | KIAA0369, DCLK, DCDC3A | doublecortin-like kinase 1 | 188 |
| **ZSCAN18** | 65982 | ENSG00000121413 | FLJ12895 | zinc finger and SCAN domain containing 18 | 190 |

**Table 4: Overview of hypermethylated genes previously identified in CCA**

| **Gene symbol** | **Location** | **Function** | **Sensitivity (%)** | **Spesificity(%)** | **Number of CCAs analyzed** | **Refs** |
|---|---|---|---|---|---|---|
| APC | 5q21 | Cell adhesion | 26-46 | 100,90 | 72-111 | (55-57) |
| BCL2 | 18q21 | Apoptosis | 23 | 97 | 111 | (55) |
| CACNA1G | 17q22 | Ion channel | 4 | 94 | 111 | (55) |
| CDH1 | 16q22 | Cell adhesion | 22-43 | 100,90 | 15-111 | (55-59) |
| CDKN2A (p14^{ARF}) | 9p21 | Cell cycle regulation | 9-38 | 100 | 51-111 | (55-57;60) |
| CDKN2A (p16^{INK4a}) | 9p21 | Cell cycle regulation | 14-77 | 100,90 | 7-111 | (55-62) |
| CDKN2B (p15^{INK4b}) | 9p21 | Cell cycle regulation | 51 | 100 | 72 | (57) |
| CHFR | 12q24 | Apoptosis | 5, 17 | 100 | 23, 111 | (55;59) |
| DAPK1 | 9q34 | Apoptosis | 3-40 | 100,93 | 15-79 | (56-59;63) |
| FHIT | 3p14 | Purine metabolism | 42 | 89 | 19 | (64) |
| GSTP1 | 11q13 | Drug metabolism | 6-18 | 100,90 | 72-111 | (55-57) |
| HOXA1 | 7p15.2 | Development | 90 | 95 | 111 | (55) |
| IGF2 | 11p15.5 | Cell growth and differentiation | 23 | 89 | 111 | (55) |
| MGMT | 10q26 | DNA repair | 4-33 | 100 | 15-111 | (55-58) |
| MINTS1 | 22q11 | Unknown | 38,41 | 100 | 79, 111 | (55;56) |
| MINT12 | 22q11 | Unknown | 51 | 100 | 79 | (56) |
| MINT2 | 22q11 | Unknown | 0,7 | 100 | 79, 111 | (55;56) |
| MINT25 | 22q11 | Unknown | 15 | 100 | 79 | (56) |
| MINT31 | 22q11 | Unknown | 1, 15 | 100 | 79, 111 | (55;56) |
| MINT32 | 22q11 | Unknown | 35 | 100 | 79 | (56) |
| MLH1 | 3p22 | DNA repair | 13-47 | 100 | 15-72 | (57-59) |
| NEUROG1 | 5q23 | Cell differentiation | 53 | 89 | 111 | (55) |
| PTGS2 | 1q25 | Biosynthesis in inflammation | 5 | 100 | 79 | (56) |
| PYCARD | 16p11 | Apoptosis | 36 | 92 | 36 | (65) |
| RARB | 3p24 | Cell growth | 14-18 | 100 | 72, 111 | (55;57) |
| | | and differentiation | | | | |
| RARRES1 | 3q25 | Membrane protein | 22 | 97 | 111 | (55) |
| RASSF1 | 3p21 | Cell cycle regulation | 28-73 | 50-100 | 13-111 | (55;57;59;64;66;6 7) |
| RBP1 | 3q21 | Retinol transport | 14 | 100 | 111 | (55) |
| RUNX3 | 1p36 | Apoptosis | 33,78 | 100 | 23,111 | (55;59) |
| SEMA3B | 3p21 | Apoptosis | 100 | 100 | 15 | (66) |
| SFN | 1p36 | Apoptosis | 59 | 100 | 79 | (56) |
| SOCS3 | 17q25 | Cytokine signaling | 88 | 100 | 8 | (68) |
| THBS1 | 15q15 | Cell adhesion | 2-11 | 100 | 79, 111 | (55;56) |
| TIMP3 | 22q12 | Cell adhesion | 1-9 | 100 | 79-111 | (55;56) |
| TMEFF2 | 2q32.3 | Cell growth and differentiation | 73 | 92 | 111 | (55) |
| TP73 | 1p36 | Cell cycle regulation | 36 | 100 | 72 | (57) |
| ZMYND10 | 3p21 | Unknown | 20 | 100 | 15 | (66) |

Genes are listed according to approved gene symbols (HUGO Gene Nomenclature Committee). Genes reported to be unmethylated are not included in the table.

**Table 5: Culturing conditions of cancer cell lines**

| **Cell line** | **Medium** | **Additives** |
|---|---|---|
| TFK-1 | Roswell Park Memorial Institute (RPMI) 1640 medium * | Penicillin-Streptomycin-Glutamine*, Fetal Bovine Serum* |
| EGI-1 | Dulbecco's Modified Eagle Medium (DMEM) * | Penicillin-Streptomycin-Glutamine*, Fetal Bovine Serum* |
| HuCCT1 | Roswell Park Memorial Institute (RPMI) 1640 medium * | Penicillin-Streptomycin-Glutamine*, Fetal Bovine Serum* |
| SK-ChA-1 | Minimum Essential Medium (MEM) * | Penicillin-Streptomycin-Glutamine*, Fetal Bovine Serum* |
| Mz-ChA-1 | Roswell Park Memorial Institute (RPMI) 1640 medium * | Penicillin-Streptomycin-Glutamine*, Fetal Bovine Serum* |
| Mz-ChA-2 | Minimum Essential Medium (MEM) * | Penicillin-Streptomycin-Glutamine*, Fetal Bovine Serum* |
| KMCU | Dulbecco's Modified Eagle Medium (DMEM) * | Penicillin-Streptomycin-Glutamine*, Fetal Bovine Serum* |
| KMBC | Dulbecco's Modified Eagle Medium (DMEM) * | Penicillin-Streptomycin-Glutamine*, Fetal Bovine Serum* and Horse Serum* |
| PaCa-2 | Dulbecco's Modified Eagle Medium (DMEM) * | Penicillin-Streptomycin-Glutamine*, Fetal Bovine Serum* and MEM Non Essential Amino Acids* |
| HPAFII | Minimum Essential Medium (MEM) * | Penicillin-Streptomycin-Glutamine*, Fetal Bovine Serum* |
| BxBc-3 | Roswell Park Memorial Institute (RPMI) 1640 medium * | Penicillin-Streptomycin-Glutamine*, Fetal Bovine Serum* |
| AsPc-1 | Roswell Park Memorial Institute (RPMI) 1640 medium * | Penicillin-Streptomycin-Glutamine*, Fetal Bovine Serum* |
| SW48 | Dulbecco's Modified Eagle Medium: Nutrient Mixture F-12 (D-MEM/F12) * | Penicillin-Streptomycin-Glutamine*, Fetal Bovine Serum* |
| SW480 | Dulbecco's Modified Eagle Medium: Nutrient Mixture F-12 (D-MEM/F12) * | Penicillin-Streptomycin-Glutamine*, Fetal Bovine Serum* |
| RKO | Dulbecco's Modified Eagle Medium: Nutrient Mixture F-12 (D-MEM/F12) * | Penicillin-Streptomycin-Glutamine*, Fetal Bovine Serum* |
| HCT15 | Dulbecco's Modified Eagle Medium: Nutrient Mixture F-12 (D-MEM/F12) * | Penicillin-Streptomycin-Glutamine*, Fetal Bovine Serum* |
| LS1034 | Dulbecco's Modified Eagle Medium: Nutrient Mixture F-12 (D-MEM/F12) * | Penicillin-Streptomycin-Glutamine*, Fetal Bovine Serum* |
| HT29 | Dulbecco's Modified Eagle Medium: Nutrient Mixture F-12 (D-MEM/F12) * | Penicillin-Streptomycin-Glutamine*, Fetal Bovine Serum* |
| HB8065 | Minimum Essential Medium (MEM) * | Penicillin-Streptomycin-Glutamine*, Fetal Bovine Serum* |
| JHH-1 | William's Medium E * | Penicillin-Streptomycin-Glutamine*, Fetal Bovine Serum* |
| JHH-4 | Minimum Essential Medium (MEM) * | Penicillin-Streptomycin-Glutamine*, Fetal Bovine Serum* |
| JHH-5 | William's Medium E | Penicillin-Streptomycin-Glutamine*, Fetal Bovine Serum* |
| Panc-1 | Dulbecco's Modified Eagle Medium (DMEM) * | Penicillin-Streptomycin-Glutamine*, Fetal Bovine Serum* |
| CFPA C-1 | Iscove's Modified Dulbecco's Medium (IMDM) # | Penicillin-Streptomycin-Glutamine*, Fetal Bovine Serum* |

| | | |
|---|---|---|
| Medium and additives were added according to requirements for each cell line. ^{*} Gibco, Invitrogen, Carlsbad, CA, USA ^{#} ATCC, Manassas, VA, USA | | |

**Table 6: Cancer cell line genotypes**

| **Cell line** | **AM EL** | **CSF 1PO** | **D13 S317** | D16 **S539** | **D18 S51** | **D19 S433** | **D21 S11** | **D2S 1338** | **D3S 1358** | **D5S 818** | **D7S 820** | **D8S 1179** | **FG A** | **TH 01** | **TP OX** | v**WA** |
|---|---|---|---|---|---|---|---|---|---|---|---|---|---|---|---|---|
| **KMB C** | X | 12 | 11 | 10 | 13 | 14, 15.2 | 29 | 17 | 15 | 12, 13 | 10, 12 | 10, 14 | 22 | 9.3 | 8, 11 | 16 |
| **KMC H-1** | XY | 11 | 10 | 9 | 14, 17 | 13, 14.2 | 30, 32.2 | 22, 25 | 16 | 10, 12 | 10, 12 | 10, 16 | 22, 23 | 9 | 8, 11 | 14, 18, 19 |
| **SK-ChA-1** | X | 12, 13 | 11, 12 | 9, 13 | 16 | 13.2, 14 | 28 | 25 | 15 | 11, 13 | 10 | 13, 14 | 25 | 6 | 8 | 14, 18 |
| **Mz-ChA-1** | X | 11 | 8, 10 | 11, 12 | 14 | 13, 15 | 28 | 16, 24 | 15 | 11 | 13, 14 | 13 | 20, 23 | 7 | 8 | 15, 19 |
| **Mz-ChA-2** | X | 12 | 12, 13 | 11 | 15 | 13, 16 | 28, 30 | 17, 26 | 18 | 10 | 11, 12 | 13 | 22, 24 | 8 | 8, 11 | 15, 17 |

All genotypes for non-commercial cell lines were obtained using the AmpFLSTR Identifiler PCR Amplification Kit (Applied Biosystems). The size of the analyzed short tandem repeats (STR) as detected by PCR and subsequent fragment analysis are shown for each loci. Amelogenin (Amel) is a gender-determining locus.

### Results

### Identification of candidate genes for promoter DNA hypermethylation in cholangiocarcinomas

From gene expression microarray analysis we observed 672 array-elements that were up-regulated two-fold or more in a minimum of four out of the six CCA cell lines after epigenetic drug treatment (5-aza-2'deoxycytidine and trichostatin A). Sixty of these genes were simultaneously found to be down-regulated in CCA samples compared with non-malignant controls in previously published data sets (26;27) (Figure 10). A CpG island was found in the promoter region of 43 of the candidates (and four isoforms from three of these candidates) and were regarded as potential DNA methylation target genes.

### DNA promoter methylation analyses of candidate genes in cancer cell lines

The promoter methylation status of 40 loci was investigated in 24 cancer cell lines using MSP, and grouped according to their methylation frequency in CCA cell lines (Figure 11). Genes in group I (n=12) were frequently methylated (≥5/6; *BEX4, CDO1*, *DCLK1, FAM3B, GREM1, LHX6, NAP1L2, SFRP1, TCF4, TPM2, ZNF331* "isoform c", and *SCAN18* "isoform b"). Genes in group II (n=10) displayed intermediate methylation frequencies (1/6-4/6; *ASRGL1, CRISPLD2, CSPP1, FKBP1B, GNG11, INPPSA, MTIF, PDE2A, REEP1,* and *SLC46A3*). The remaining 18 genes (group III) were unmethylated in all CCA cell lines *(ATF3, CALCOCO1, CLU, CTGF, DUSP5, EGR2* "isoform b", *FHL1, GPR124, HABP4, ID3, ITPR1, LMCD1, MLLT11, MT1X, MT2A, NR4A3, RNase4,* and *SYT11*)*.* Genes *CXCL14, DPYSL3, EGR2* "isoform a", *STXBP1, ZNF331* "isoform a", *ZNF331* "isoform b", and *ZSCAN18* "isoform a", were excluded from further analysis based on the presence of a weak band in one of the following control reactions; the methylated reaction using normal blood, the unmethylated reaction using completely methylated DNA, or the methylated reaction using non-bisulfite treated DNA.

Interestingly, the methylation frequencies within groups I, II, and III seemed comparable among the gastrointestinal cancer cell lines included in the present study, with the exception of *LHX6* and *NAP1L2* which displayed little or no methylation in cell lines from hepatocellular carcinoma, and *TPM2* and *ZNF331* "isoform c" which showed no to little methylation in cell lines from pancreatic cancer.

### Qualitative DNA promoter methylation analysis of target genes in tissue samples

All group I genes were subjected to MSP analysis in 13 CCA and 21 non-malignant fresh-frozen clinical samples. We observed methylation of *BEX4, CDO1*, *DCLK1, GREM1, NAP1L2, SFRP1, TCF4, ZNF331,* and *SCAN18* in 69%, 62%, 83%, 23%, 69%, 85%, 23%, 23%, and 31% in tumors and 33%, 14%, 100%, 13%, 38%, 86%, 0%, 0%, and 0% in non-malignant controls (Figure 14). The remaining three loci, *FAM3B, LHX6,* and *TPM2,* from group I had low methylation frequencies (<10%) in tumors and varying methylation (19 - 90%) in the non-malignant controls. The methylated band intensities in the non-malignant controls were considerably weaker compared to tumors, indicating that a quantitative methylation analysis might discriminate more accurately between these groups. Subsequently, gene promoters exhibiting more than 30% methylation in tumors (*CDO1, DCLK1, SFRP1,* and *ZSCAN18*) were subjected to quantitative methylation analysis (qMSP). *BEX4* and *NAP1L2* were excluded from further analysis since they displayed methylation in female normal blood controls. Normal blood controls were included in testing of each assay)

### Validation of promoter methylation status by direct bisulfite sequencing

To verify the promoter methylation status as assessed by MSP, the promoter region of *CDO1*, *DCLK1,* and *SCAN18* were subjected to direct bisulfite sequencing in representative cancer cell lines. A good concordance was seen between the MSP and bisulfite sequencing results (Figure 12). The results were used to guide the design of the quantitative DNA methylation assays. *SFRP1* has previously been analyzed by qMSP and was therefore not included in the bisulfite sequencing analysis.

### Quantitative DNA methylation analyses in fresh frozen and archival clinical material

*CDO1, DCLK1, SFRP1,* and *SCAN18* were analyzed with qMSP in two sample series; fresh frozen material comprising 13 CCAs and 21 non-malignant controls; and archival material comprising 26 tumor and 33 non-malignant controls. For the fresh frozen sample series, we detected promoter hypermethylation in 46%, 69%, 77%, and 85%, for *DCLK1, SFRP1, SCAN18,* and *CDO1,* respectively in tumors and no methylation in the non-malignant controls. By combining all four genes and scoring samples with methylation in minimum one out of the four genes as positive, 100% of the tumors and 0% non-malignant samples were methylation positive. The individual and combined performance of these genes was investigated by reciever operating characteristics curves (Figure 13). Combining the four genes (summarizing the PMR values) resulted in an area under the curve of 0.996.

Frequencies of methylation in the archival series were in general lower than the findings in the fresh frozen sample set, although not statistically significant. The four genes *DCLK1, SCAN18, SFRP1,* and *CDO1* displayed promoter methylation frequencies of 42%, 42%, 54%, and 73% in tumors, whereas no methylation was observed in non-malignant controls. The combined panel was methylation positive in 81 % of the tumors. The resulting area under the curve for this sample set was 0.904 (Figure 13).

We further investigated the methylation frequency of the four genes *DCLK1, SCAN18, SFRP1,* and *CDO1* in a non-malignant sample set of six fresh frozen dissected bile duct samples with primary sclerosing cholangitis. Interestingly, two samples (33%) were methylation positive for all four biomarkers, and one sample (17%) was methylation positive for *ZSCAN18.*

### Discussion

In the present study, we have identified *CDO1*, *DCLK1,* and *SCAN18* as novel frequently methylated genes in cholangiocarcinoma, in addition to the previously reported *SFRP1* gene (28;29). Tissue samples from carcinoma-free individuals were unmethylated for the same genes, indicating that the promoter hypermethylation was tumor specific. The high sensitivity and specificity of these genes suggest that *CDO1, DCLK1,* and *ZSCAN18* individually represent novel and promising biomarkers for cholangiocarcinoma. By including *SFRP1,* the combined biomarker panel had a sensitivity and specificity of 100% in fresh frozen material. Although the sensitivity decreased somewhat in archival material, the present biomarker panel has the potential to improve the diagnostic accuracy of CCA compared with existing clinical approaches (4;7;8).

In accordance with a previous study from our group (25) we used a step-wise experimental approach to identify novel methylation biomarkers. The approach included strict selection criteria in order to minimize the probability of selecting false positive methylation candidates. From the microarray analysis of epigenetic drug treated cancer cell lines, only genes up-regulated at least two-fold in a minimum of four of the six cholangiocarcinoma cell lines were selected. The gene expression of these candidates was further examined in available microarray data sets (26;27) and only genes down-regulated in primary CCA tumors compared with cancer-free controls were selected for further studies and subjected to promoter methylation analysis in cancer cell lines. Frequently methylated loci were subsequently analyzed in patient material and three genes (*CDO1, DCLK1,* and *SFRP1*) had methylation frequencies above 50% in tumor samples from qualitative methylation analysis. These genes, including *SCAN18,* were further subjected to a quantitative methylation analysis, resulting in 100% sensitivity and specificity for the combined biomarker panel in fresh frozen tissue.

The combined biomarker panel was methylated in 100% of cholangiocarcinomas and unmethylated across all non-malignant control samples. To increase the number of cancer samples we included a second sample series consisting of archival formalin fixed and paraffin embedded samples. As expected, the biomarker panel performance in the archival material was somewhat poorer than for the fresh frozen samples. However, the combined sensitivity (87%) and specificity (100%) for both sample series display a high performance of this biomarker panel.

Furthermore, the biomarker panel was evaluated in six non-malignant dissected bile duct samples with primary sclerosing cholangitis. Interestingly, two samples displayed positive promoter methylation in all four biomarkers. Due to positive methylation detected by this biomarker panel, we hypothesize that these findings may indicate cancer development at an early stage which may have avoided detection due to the current challenges in CCA diagnosis. Thus, this biomarker panel could add positive value to the detection of CCA in a clinical setting.

In order to detect CCA irrespective of sub-classification and disease background, the tumor material used in this study included both extrahepatic and intrahepatic lesions with and without a primary sclerosing cholangitis. A pool of benign liver diseases with biopsies taken from different locations in the liver served as controls. This was done to avoid a potential skewedness when comparing bile duct epithelial derived tumors with non-malignant samples devoid of biliary epithelium.

Epigenome-wide expression profiling has previously been used to identify potential epigenetic markers in several tumor types, including bladder, pancreas, and prostate (30-32). Using a similar experimental protocol as presented in this study (25), we have previously identified epigenetic markers for early detection of colorectal cancer (33;34). From cell line analyses we observed that these markers were present also in other cancers of the gastrointestinal tract, although at different frequencies (34). Cell lines from several hepatopancreatobiliary tumors as well as colon cancer were therefore included in the present study. We observed similarities in methylation frequencies across the majority of cancer cell lines, indicating that the genes may be aberrantly methylated also in tissue samples from other gastrointestinal tumors.

One of the four biomarkers presented here, *SFRP1,* has previously been investigated as a potential epigenetic biomarker in several cancers, including CCA (28;29;35;36). Secreted frizzled-related protein (SFRP) family members act as modulators of the Wnt-pathway, and hypermethylation of these promoter regions can lead to deregulation of this pathway and subsequent cancer development (37). The methylation frequencies published for *SFRP1* by Uhm et al. (29), and Sriraksa et al. (28) (64%) are in the same range as presented here.

Cysteine dioxygenase, type 1 (*CDO1*) is reported to be highly expressed in the liver (38). It is involved in initiation of metabolic pathways related to pyruvate and sulfurate compounds, including taurine which is a major constituent of bile. *CDO1* promoter hypermethylation was recently shown to be a strong marker for distant metastasis in lymph node positive, estrogen receptor positive breast cancer patients (39). In addition, *CDO1* has been indicated to be epigenetically deregulated in colorectal cancer, lung cancer, and Wilms tumor (40-42). Our results support these findings and show for the first time that the promoter hypermethylation of *CDO1* also may play a role in CCA.

Doublecortin like kinase 1 (*DCLK1*) is a microtubule-associated kinase that can undergo autophosphorylation. To our knowledge, this is the first study reporting promoter hypermethylation of this gene in cancer. However, previous studies have proposed the *DCLK1* protein expression as a marker for intestinal stem cells with a role in the epithelial-mesenchymal transition (43;44). Promoter hypermethylation suggests a silencing of this gene, and further studies should be performed to clarify the role of aberrantly regulated *DCLK1* in

### CCA.

Morris and colleagues recently reported on a putative tumor suppressor function of zinc finger and SCAN domain-containing protein 18 (*SCAN18*) in renal cell carcinoma (45). In the present study we show that *ZSCAN18* is slightly more frequently methylated in cholangiocarcinomas compared with renal cancer (32%).

The number of samples analyzed represents a limitation to the present study. An increase in the number of fresh frozen and/or archival tumor- and normal samples would increase the statistical power in evaluating the presented biomarker panel. Validation in a larger sample set is therefore warranted.

Summarized, we have identified four hypermethylated genes (*CDO1*, *DCLK1, SFRP1,* and *ZSCAN18*) of which three (*CDO1*, *DCLK1,* and *ZSCAN18*) have not previously been described in CCA. The combined performance of this biomarker panel reached an 87% sensitivity and 100% specificity across fresh frozen and archival material. Further studies should be performed in minimal invasive samples, e.g. bile, biliary brush cytology specimens and/or blood in order to evaluate if the presented panel can contribute in non- to minimally-invasive CCA diagnostics.

### References:

(1) Khan SA, Thomas HC, Davidson BR, Taylor-Robinson SD. Cholangiocarcinoma. Lancet 2005;366:1303-14.
(2) Shaib YH, Davila JA, McGlynn K, El-Serag HB. Rising incidence of intrahepatic cholangiocarcinoma in the United States: a true increase? J Hepatol 2004;40:472-7.
(3) Burak K, Angulo P, Pasha TM, Egan K, Petz J, Lindor KD. Incidence and risk factors for cholangiocarcinoma in primary sclerosing cholangitis. Am J Gastroenterol 2004;99:523-6.
(4) Boberg KM, Bergquist A, Mitchell S, Pares A, Rosina F, Broome U, Chapman R et al. Cholangiocarcinoma in primary sclerosing cholangitis: risk factors and clinical presentation. Scand J Gastroenterol 2002;37:1205-11.
(5) Sripa B, Pairojkul C. Cholangiocarcinoma: lessons from Thailand. Curr Opin Gastroenterol 2008;24:349-56.
(6) Cai WK, Sima H, Chen BD, Yang GS. Risk factors for hilar cholangiocarcinoma: a case-control study in China. World J Gastroenterol 2011;17:249-53.
(7) Campbell WL, Ferris JV, Holbert BL, Thaete FL, Baron RL. Biliary tract carcinoma complicating primary sclerosing cholangitis: evaluation with CT, cholangiography, US, and MR imaging. Radiology 1998;207:41-50.
(8) Bonney GK, Craven RA, Prasad R, Melcher AF, Selby PJ, Banks RE. Circulating markers of biliary malignancy: opportunities in proteomics? Lancet Oncol 2008;9:149-58.
(9) Nehls O, Gregor M, Klump B. Serum and bile markers for cholangiocarcinoma. Semin Liver Dis 2004;24:139-54.
(10) Akdogan M, Sasmaz N, Kayhan B, Biyikoglu I, Disibeyaz S, Sahin B. Extraordinarily elevated CA19-9 in benign conditions: a case report and review of the literature. Tumori 2001;87:337-9.
(11) Steinberg W. The clinical utility of the CA 19-9 tumor-associated antigen. Am J Gastroenterol 1990;85:350-5.
(12) Vestergaard EM, Hein HO, Meyer H, Grunnet N, Jorgensen J, Wolf H, Orntoft TF. Reference values and biological variation for tumor marker CA 19-9 in serum for different Lewis and secretor genotypes and evaluation of secretor and Lewis genotyping in a Caucasian population. Clin Chem 1999;45:54-61.
(13) Jones PA, Baylin SB. The fundamental role of epigenetic events in cancer. Nat Rev Genet 2002;3:415-28.
(14) Markowitz SD, Bertagnolli MM. Molecular origins of cancer: Molecular basis of colorectal cancer. N Engl J Med 2009;361:2449-60.
(15) Hanahan D, Weinberg RA. Hallmarks of cancer: the next generation. Cell 2011;144:646-74.
(16) Hatziapostolou M, Iliopoulos D. Epigenetic aberrations during oncogenesis. Cell Mol Life Sci 2011;68:1681-702.
(17) Brooks J, Cairns P, Zeleniuch-Jacquotte A. Promoter methylation and the detection of breast cancer. Cancer Causes Control 2009;20:1539-50.
(18) Suzuki H, Tokino T, Shinomura Y, Imai K, Toyota M. DNA methylation and cancer pathways in gastrointestinal tumors. Pharmacogenomics 2008;9:1917-28.
(19) Bird A. DNA methylation patterns and epigenetic memory. Genes Dev 2002;16:6-21.
(20) Chan AO, Broaddus RR, Houlihan PS, Issa JP, Hamilton SR, Rashid A. CpG island methylation in aberrant crypt foci of the colorectum. Am J Pathol 2002;160:1823-30.
(21) Baylin SB, Herman JG, Graff JR, Vertino PM, Issa JP. Alterations in DNA methylation: a fundamental aspect of neoplasia. Adv Cancer Res 1998;72:141-96.
(22) Belinsky SA. Gene-promoter hypermethylation as a biomarker in lung cancer. Nat Rev Cancer 2004;4:707-17.
(23) Fujiwara K, Fujimoto N, Tabata M, Nishii K, Matsuo K, Hotta K, Kozuki T et al. Identification of epigenetic aberrant promoter methylation in serum DNA is useful for early detection of lung cancer. Clin Cancer Res 2005;11:1219-25.
(24) Park SY, Kwon HJ, Lee HE, Ryu HS, Kim SW, Kim JH, Kim IA et al. Promoter CpG island hypermethylation during breast cancer progression. Virchows Arch 2011;458:73-84.
(25) Lind GE, Kleivi K, Meling GI, Teixeira MR, Thiis-Evensen E, Rognum TO, Lothe RA. ADAMTS1, CRABP1, and NR3C1 identified as epigenetically deregulated genes in colorectal tumorigenesis. Cell Oncol 2006;28:259-72.
(26) Miller G, Socci ND, Dhall D, D'Angelica M, DeMatteo RP, Allen PJ, Singh B et al. Genome wide analysis and clinical correlation of chromosomal and transcriptional mutations in cancers of the biliary tract. J Exp Clin Cancer Res 2009;28:62.
(27) Obama K, Ura K, Li M, Katagiri T, Tsunoda T, Nomura A, Satoh S et al. Genome-wide analysis of gene expression in human intrahepatic cholangiocarcinoma. Hepatology 2005;41:1339-48.
(28) Sriraksa R, Zeller C, El-Bahrawy MA, Dai W, Daduang J, Jearanaikoon P, Chau-In S et al. CpG-island methylation study of liver fluke-related cholangiocarcinoma. Br J Cancer 2011;104:1313-8.
(29) Uhm KO, Lee ES, Lee YM, Kim HS, Park YN, Park SH. Aberrant promoter CpG islands methylation of tumor suppressor genes in cholangiocarcinoma. Oncol Res 2008;17:151-7.
(30) Costa VL, Henrique R, Danielsen SA, Duarte-Pereira S, Eknaes M, Skotheim RI, Rodrigues A et al. Three epigenetic biomarkers, GDF15, TMEFF2, and VIM, accurately predict bladder cancer from DNA-based analyses of urine samples. Clin Cancer Res 2010;16:5842-51.
(31) Lodygin D, Epanchintsev A, Menssen A, Diebold J, Hermeking H. Functional epigenomics identifies genes frequently silenced in prostate cancer. Cancer Res 2005;65:4218-27.
(32) Sato N, Matsubayashi H, Abe T, Fukushima N, Goggins M. Epigenetic downregulation of CDKN1C/p57KIP2 in pancreatic ductal neoplasms identified by gene expression profiling. Clin Cancer Res 2005;11:4681-8.
(33) Lind GE, Ahlquist T, Lothe RA. DNA hypermethylation of MAL: a promising diagnostic biomarker for colorectal tumors. Gastroenterology 2007;132:1631-2.
(34) Lind GE, Danielsen SA, Ahlquist T, Merok MA, Andresen K, Skotheim RI, Hektoen M et al. Identification of an epigenetic biomarker panel with high sensitivity and specificity for colorectal cancer and adenomas. Mol Cancer 2011;10:85.
(35) Gonzalo V, Lozano JJ, Munoz J, Balaguer F, Pellise M, Rodriguez de MC, Andreu M et al. Aberrant gene promoter methylation associated with sporadic multiple colorectal cancer. PLoS One 2010;5:e8777.
(36) Huang ZH, Hu Y, Hua D, Wu YY, Song MX, Cheng ZH. Quantitative analysis of multiple methylated genes in plasma for the diagnosis and prognosis of hepatocellular carcinoma. Exp Mol Pathol 2011;91:702-7.
(37) Kawano Y, Kypta R. Secreted antagonists of the Wnt signalling pathway. J Cell Sci 2003;116:2627-34.
(38) Tsuboyama-Kasaoka N, Hosokawa Y, Kodama H, Matsumoto A, Oka J, Totani M. Human cysteine dioxygenase gene: structural organization, tissue-specific expression and downregulation by phorbol 12-myristate 13-acetate. Biosci Biotechnol Biochem 1999;63:1017-24.
(39) Dietrich D, Krispin M, Dietrich J, Fassbender A, Lewin J, Harbeck N, Schmitt M et al. CDO1 promoter methylation is a biomarker for outcome prediction of anthracycline treated, estrogen receptor-positive, lymph node-positive breast cancer patients. BMC Cancer 2010;10:247.
(40) Kwon YJ, Lee SJ, Koh JS, Kim SH, Lee HW, Kang MC, Bae JB et al. Genome-Wide Analysis of DNA Methylation and the Gene Expression Change in Lung Cancer. J Thorac Oncol 2011.
(41) Mossman D, Scott RJ. Long term transcriptional reactivation of epigenetically silenced genes in colorectal cancer cells requires DNA hypomethylation and histone acetylation. PLoS One 2011;6:e23127.
(42) Maschietto M, Piccoli FS, Costa CM, Camargo LP, Neves JI, Grundy PE, Brentani H et al. Gene expression analysis of blastemal component reveals genes associated with relapse mechanism in Wilms tumour. Eur J Cancer 2011.
(43) Sureban SM, May R, Lightfoot SA, Hoskins AB, Lerner M, Brackett DJ, Postier RG et al. DCAMKL-1 regulates epithelial-mesenchymal transition in human pancreatic cells through a miR-200a-dependent mechanism. Cancer Res 2011;71:2328-38.
(44) Souza RF, Krishnan K, Spechler SJ. Acid, bile, and CDX: the ABCs of making Barrett's metaplasia. Am J Physiol Gastrointest Liver Physiol 2008;295:G211-G218.
(45) Morris MR, Ricketts CJ, Gentle D, McRonald F, Carli N, Khalili H, Brown M et al. Genome-wide methylation analysis identifies epigenetically inactivated candidate tumour suppressor genes in renal cell carcinoma. Oncogene 2011;30:1390-401.
(46) Kim SK, Jang HR, Kim JH, Kim M, Noh SM, Song KS, Kang GH et al. CpG methylation in exon 1 of transcription factor 4 increases with age in normal gastric mucosa and is associated with gene silencing in intestinal-type gastric cancers. Carcinogenesis 2008;29:1623-31.
(47) Rawson JB, Manno M, Mrkonjic M, Daftary D, Dicks E, Buchanan DD, Younghusband HB et al. Promoter methylation of Wnt antagonists DKK1 and SFRP1 is associated with opposing tumor subtypes in two large populations of colorectal cancer patients. Carcinogenesis 2011;32:741-7.
(48) Takai D, Jones PA. The CpG island searcher: a new WWW resource. In Silico Biol 2003;3:235-40.
(49) Takai D, Jones PA. Comprehensive analysis of CpG islands in human chromosomes 21 and 22. Proc Natl Acad Sci U S A 2002;99:3740-5.
(50) Clark SJ, Harrison J, Paul CL, Frommer M. High sensitivity mapping of methylated cytosines. Nucleic Acids Res 1994;22:2990-7.
(51) Herman JG, Graff JR, Myohanen S, Nelkin BD, Baylin SB. Methylation-specific PCR: a novel PCR assay for methylation status of CpG islands. Proc Natl Acad Sci U S A 1996;93:9821-6.
(52) Lind GE, Ahlquist T, Kolberg M, Berg M, Eknaes M, Alonso MA, Kallioniemi A et al. Hypermethylated MAL gene - a silent marker of early colon tumorigenesis. J Transl Med 2008;6:13.
(53) Melki JR, Vincent PC, Clark SJ. Concurrent DNA hypermethylation of multiple genes in acute myeloid leukemia. Cancer Res 1999;59:3730-40.
(54) Weisenberger DJ, Campan M, Long TI, Kim M, Woods C, Fiala E, Ehrlich M et al. Analysis of repetitive element DNA methylation by MethyLight. Nucleic Acids Res 2005;33:6823-36.
(55) Kim BH, Cho NY, Choi M, Lee S, Jang JJ, Kang GH. Methylation profiles of multiple CpG island loci in extrahepatic cholangiocarcinoma versus those of intrahepatic cholangiocarcinomas. Arch Pathol Lab Med 2007;131:923-30.
(56) Lee S, Kim WH, Jung HY, Yang MH, Kang GH. Aberrant CpG island methylation of multiple genes in intrahepatic cholangiocarcinoma. Am J Pathol 2002;161:1015-22.
(57) Yang B, House MG, Guo M, Herman JG, Clark DP. Promoter methylation profiles of tumor suppressor genes in intrahepatic and extrahepatic cholangiocarcinoma. Mod Pathol 2005;18:412-20.
(58) Koga Y, Kitajima Y, Miyoshi A, Sato K, Kitahara K, Soejima H, Miyazaki K. Tumor progression through epigenetic gene silencing of O(6)-methylguanine-DNA methyltransferase in human biliary tract cancers. Ann Surg Oncol 2005;12:354-63.
(59) Tozawa T, Tamura G, Honda T, Nawata S, Kimura W, Makino N, Kawata S et al. Promoter hypermethylation of DAP-kinase is associated with poor survival in primary biliary tract carcinoma patients. Cancer Sci 2004;95:736-40.
(60) Tannapfel A, Sommerer F, Benicke M, Weinans L, Katalinic A, Geissler F, Uhlmann D et al. Genetic and epigenetic alterations of the INK4a-ARF pathway in cholangiocarcinoma. J Pathol 2002;197:624-31.
(61) Ahrendt SA, Eisenberger CF, Yip L, Rashid A, Chow JT, Pitt HA, Sidransky D. Chromosome 9p21 loss and p16 inactivation in primary sclerosing cholangitis-associated cholangiocarcinoma. J Surg Res 1999;84:88-93.
(62) Hong SM, Choi J, Ryu K, Ro JY, Yu E. Promoter hypermethylation of the p16 gene and loss of its protein expression is correlated with tumor progression in extrahepatic bile duct carcinomas. Arch Pathol Lab Med 2006;130:33-8.
(63) Liu XF, Kong FM, Xu Z, Yu SP, Sun FB, Zhang CS, Huang QX et al. Promoter hypermethylation of death-associated protein kinase gene in cholangiocarcinoma. Hepatobiliary Pancreat Dis Int 2007;6:407-11.
(64) Foja S, Goldberg M, Schagdarsurengin U, Dammann R, Tannapfel A, Ballhausen WG. Promoter methylation and loss of coding exons of the fragile histidine triad (FHIT) gene in intrahepatic cholangiocarcinomas. Liver Int 2005;25:1202-8.
(65) Liu XF, Zhu SG, Zhang H, Xu Z, Su HL, Li SJ, Zhou XT. The methylation status of the TMS1/ASC gene in cholangiocarcinoma and its clinical significance. Hepatobiliary Pancreat Dis Int 2006;5:449-53.
(66) Tischoff I, Markwarth A, Witzigmann H, Uhlmann D, Hauss J, Mirmohammadsadegh A, Wittekind C et al. Allele loss and epigenetic inactivation of 3p21.3 in malignant liver tumors. Int J Cancer 2005;115:684-9.
(67) Wong N, Li L, Tsang K, Lai PB, To KF, Johnson PJ. Frequent loss of chromosome 3p and hypermethylation of RASSF1A in cholangiocarcinoma. J Hepatol 2002;37:633-9.
(68) Isomoto H, Mott JL, Kobayashi S, Werneburg NW, Bronk SF, Haan S, Gores GJ. Sustained IL-6/STAT-3 signaling in cholangiocarcinoma cells due to SOCS-3 epigenetic silencing. Gastroenterology 2007;132:384-96.

## Claims

1. Method for detecting a gastrointestinal neoplasm in a subject comprising:
a) obtaining DNA from a biological sample of said subject; and
b) determining the level, presence, or frequency of methylation of nucleic acid polymers corresponding to *GLDC* and one or more genes selected from the group consisting of *CDO1, DCLK1, ZSCAN18* and *ZNF331.*

2. Method of claim 1, wherein the level, presence, or frequency of methylation of a nucleic acid polymer corresponding to at least one additional gene is determined, said at least one additional gene being selected from the group consisting of *SFRP1* and *PPP1R14A*.

3. Method of claim 1 or 2, wherein the level, presence, or frequency of methylation of said nucleic acid polymer is compared to a reference level, presence, or frequency of methylation, wherein an altered level, presence, or frequency of methylation for said subject DNA relative to said reference provides an indication that the subject has gastrointestinal cancer.

4. Method of any of the preceding claims wherein said nucleic acid comprises a region selected from the group consisting of a CpG island and a CpG island shore, said CpG island or shore preferably being present in a coding region or a regulatory region.

5. Method of any of the preceding claims, wherein said determining of the level of altered methylation of a nucleic acid polymer comprises determining the methylation frequency of said CpG island or island shore, preferably by a technique selected from the group consisting of methylation-specific PCR, quantitative methylation-specific PCR, methylation-sensitive DNA restriction enzyme analysis, methylation - insensitive DNA restriction enzyme analysis, quantitative bisulfite pyrosequencing, and bisulfite genomic sequencing PCR.

6. Method of any of the preceding claims, further comprising: c) generating a risk profile using the results of steps a) and b).

7. Method of any of the preceding claims, wherein said gastrointestinal neoplasm is colorectal cancer, gastric cancer, pancreatic cancer, liver cancer, cancers of the gall bladder and/ or bile ducts, or cholangiocarcinoma.

8. Method of any of the preceding claims, wherein said method permits detection of gastrointestinal cancer in said subject with a sensitivity of at least 85% at a specificity of at least 85%, or with a sensitivity of at least 80% at a specificity of at least 90%.

9. Method of any of the preceding claims, wherein said biological sample is selected from the group consisting of a tissue sample, a stool sample, a cell sample, a bile sample and a blood sample.

10. A set of methylation specific nucleic acid detection sequences, said detection sequences being specific for *GLDC* and one or more genes selected from the group consisting of *CDO1, DCLK1, ZSCAN18* and *ZNF331.*

11. Use of the set of nucleic acid sequences of claim 10 for detecting a cancerous condition in a subject, said cancerous condition being a gastrointestinal neoplasm, said gastrointestinal neoplasm preferably being colorectal cancer, gastric cancer, pancreatic cancer, liver cancer, cancers of the gall bladder and/or bile ducts, or cholangiocarcinoma, more preferably being colorectal cancer, gastric cancer or cholangiocarcinoma.

12. Use of claim 11, wherein an additional methylation specific nucleic acid detection sequence is utilized in addition to detection sequences for *GLDC* and one or more of GLDC and *CDO1, DCLK1, ZSCAN18* and *ZNF331,* preferably one or more of *PPP1R14* and *SFRP1.*

13. Use of Claim 11 or 12, wherein an altered level, presence, or frequency of methylation for a subject relative to a reference provides an indication that the subject has gastrointestinal cancer.

14. Use of a kit in a method according to any of claims 1 to 9, said kit comprising reagents useful, sufficient, or necessary for detecting and/or characterizing level, presence, or frequency of methylation of *GLDC* and one or more genes selected from the group consisting of *CDO1, DCLK1, ZSCAN18* and *ZNF331,* wherein said reagents comprise DNA probes that are specific for *GLDC* and DNA probes that are specific for one or more of said genes *CDO1, DCLK1, ZSCAN18* and *ZNF331.*

15. Use of a kit of claim 14, said kit further comprising reagents useful, sufficient, or necessary for detecting and/or characterizing level, presence, or frequency of methylation of at least one of *PPP1R14A* and *SFRP1,* wherein said reagents comprise DNA probes that are specific to at least one of *PPP1R14A* and *SFRP1.*

## Patentansprüche

1. Verfahren zum Nachweisen eines gastrointestinalen Tumors in einem Subjekt, das Folgendes beinhaltet:
a) Gewinnen von DNA von einer biologischen Probe des Subjekts; und
b) Bestimmen von Spiegel, Anwesenheit oder Häufigkeit der Methylierung von Nukleinsäurepolymeren entsprechend *GLDC* und einem oder mehreren Genen, ausgewählt aus der Gruppe, bestehend aus *CD01, DCLK1, ZSCAN18* und *ZNF331.*

2. Verfahren nach Anspruch 1, wobei Spiegel, Anwesenheit oder Häufigkeit der Methylierung eines Nukleinsäurepolymers entsprechend wenigstens einem zusätzlichen Gen bestimmt wird, wobei das wenigstens eine zusätzliche Gen ausgewählt ist aus der Gruppe, bestehend aus *SFRP1* und *PPP1R14A.*

3. Verfahren nach Anspruch 1 oder 2, wobei Spiegel, Anwesenheit oder Häufigkeit der Methylierung des Nukleinsäurepolymers mit einem Referenzspiegel, einer Referenzanwesenheit oder -häufigkeit der Methylierung verglichen wird, wobei ein veränderter Spiegel, eine veränderte Anwesenheit oder Häufigkeit der Methylierung für die Subjekt-DNA relativ zu der Referenz ein Anzeichen dafür ist, dass das Subjekt Magen-Darm-Krebs hat.

4. Verfahren nach einem der vorhergehenden Ansprüche, wobei die Nukleinsäure eine Region umfasst, ausgewählt aus der Gruppe bestehend aus einer CpG-Insel und einem CpG-Inselufer, wobei die CpG-Insel oder das CpG-Ufer vorzugsweise in einer Codierungsregion oder einer regulatorischen Region vorhanden ist.

5. Verfahren nach einem der vorhergehenden Ansprüche, wobei das Bestimmen des Spiegels von veränderter Methylierung eines Nukleinsäurepolymers das Bestimmen der Methylierungshäufigkeit der CpG-Insel oder des CpG-Ufers beinhaltet, vorzugsweise mit einer Technik, ausgewählt aus der Gruppe, bestehend aus methylierungsspezifischer PCR, quantitativer methylierungsspezifischer PCR, methylierungsempfindlicher DNA-Restriktionsenzymanalyse, methylierungsunempfindlicher DNA-Restriktionsenzymanalyse, quantitativer Bisulfitpyrosequenzierung und genomischer Bisulfit-Sequenzierungs-PCR.

6. Verfahren nach einem der vorhergehenden Ansprüche, das ferner Folgendes beinhaltet: c) Erzeugen eines Risikoprofils anhand der Ergebnisse der Schritte a) und b).

7. Verfahren nach einem der vorhergehenden Ansprüche, wobei der gastrointestinale Tumor Kolorektalkrebs, Magenkrebs, Bauchspeicheldrüsenkrebs, Leberkrebs, Krebs von Gallenblase und/oder Gallenwegen oder Cholangiokarzinom ist.

8. Verfahren nach einem der vorhergehenden Ansprüche, wobei das Verfahren das Nachweisen von Magen-Darm-Krebs in dem Subjekt mit einer Empfindlichkeit von wenigstens 85 % bei einer Spezifizität von wenigstens 85 % oder mit einer Empfindlichkeit von wenigstens 80 % bei einer Spezifizität von wenigstens 90 % ermöglicht.

9. Verfahren nach einem der vorhergehenden Ansprüche, wobei die biologische Probe ausgewählt ist aus der Gruppe bestehend aus Gewebeprobe, Stuhlprobe, Zellprobe, Gallenprobe und Blutprobe.

10. Satz von methylierungsspezifischen Nukleinsäurenachweissequenzen, wobei die Nachweissequenzen für *GLDC* und ein oder mehrere Gene, ausgewählt aus der Gruppe, bestehend aus *CD01, DCLK1, ZSCAN18* und *ZNF331,* spezifisch sind.

11. Verwendung des Satzes von Nukleinsäuresequenzen nach Anspruch 10 zum Nachweisen eines krebsartigen Zustands in einem Subjekt, wobei der krebsartige Zustand ein gastrointestinaler Tumor ist, wobei der gastrointestinale Tumor bevorzugt Kolorektalkrebs, Magenkrebs, Bauchspeicheldrüsenkrebs, Leberkrebs, Krebs von Gallenblase und/oder Gallenwegen oder Cholangiokarzinom, besonders bevorzugt Kolorektalkrebs, Magenkrebs oder Cholangiokarzinom ist.

12. Verwendung nach Anspruch 11, wobei eine zusätzliche methylierungsspezifische Nukleinsäurenachweissequenz zusätzlich zu Nachweissequenzen für *GLDC* und ein oder mehreren aus *GLDC* und *CD01, DCLK1, ZSCAN18* und *ZNF331,* bevorzugt ein oder mehreren aus *PPP1R14* und *SFRP1,* benutzt wird.

13. Verwendung nach Anspruch 11 oder 12, wobei ein veränderter Spiegel, eine veränderte Anwesenheit oder Häufigkeit der Methylierung für ein Subjekt relativ zu einer Referenz ein Anzeichen dafür ist, dass das Subjekt Magen-Darm-Krebs hat.

14. Verwendung eines Kit in einem Verfahren nach einem der Ansprüche 1 bis 9, wobei das Kit Reagenzien umfasst, die zum Nachweisen und/oder Charakterisieren von Spiegel, Anwesenheit oder Häufigkeit der Methylierung von *GLDC* und einem oder mehrerer Gene, ausgewählt aus der Gruppe, bestehend aus *CD01, DCLK1, ZSCAN18* und *ZNF331,* nützlich, ausreichend oder notwendig sind, wobei die Reagenzien DNA-Sonden umfassen, die für *GLDC* spezifisch sind, und DNA-Sonden, die für ein oder mehrere der Gene *CD01, DCLK1, ZSCAN18* und *ZNF331* spezifisch sind.

15. Verwendung eines Kit nach Anspruch 14, wobei das Kit ferner Reagenzien umfasst, die zum Nachweisen und/oder Charakterisieren von Spiegel, Anwesenheit oder Häufigkeit der Methylierung von *PPP1R14A* und/oder *SFRP1* nützlich, ausreichend oder notwendig sind, wobei die Reagenzien DNA-Sonden umfassen, die für *PPP1R14A* und/oder *SFRP1* spezifisch sind.

## Revendications

1. Procédé de détection d'un néoplasme gastro-intestinal chez un sujet comprenant :
a) L'obtention d'un ADN à partir d'un échantillon biologique dudit sujet ; et
b) la détermination du niveau, de la présence ou de la fréquence de méthylation de polymères d'acides nucléiques correspondant à *GLDC* et d'un ou plusieurs gène(s) choisi(s) dans le groupe constitué de *CDO1,* de *DCLK1,* de *ZSCAN18* et de *ZNF331.*

2. Procédé selon la revendication 1, dans lequel le niveau, la présence ou la fréquence de méthylation d'un polymère d'acide nucléique correspondant à au moins un gène supplémentaire est déterminé(e), ledit au moins un gène supplémentaire étant choisi dans le groupe constitué de *SFRP1* et de *PPP1R14A.*

3. Procédé selon la revendication 1 ou 2, dans lequel le niveau, la présence ou la fréquence de méthylation dudit polymère d'acide nucléique est comparé(e) à un niveau, à une présence ou à une fréquence de référence de méthylation, où un niveau, une présence ou une fréquence modifié(e) de méthylation pour ledit ADN de sujet par rapport à ladite référence fournit une indication selon laquelle le sujet a un cancer gastro-intestinal.

4. Procédé selon l'une des revendications précédentes, dans lequel ledit acide nucléique comprend une région choisie dans le groupe constitué d'un îlot CpG et d'un rivage d'îlot CpG, ledit rivage ou îlot CpG étant de préférence présent dans une région codante ou une région régulatrice.

5. Procédé selon l'une des revendications précédentes, dans lequel ladite détermination du niveau de méthylation modifiée d'un polymère d'acide nucléique comprend la détermination de la fréquence de méthylation dudit îlot CpG ou dudit rivage d'îlot, de préférence par une technique choisie dans le groupe constitué de la PCR spécifique de la méthylation, de la PCR spécifique de la méthylation quantitative, de l'analyse par enzyme de restriction d'ADN sensible à la méthylation, de l'analyse par enzyme de restriction d'ADN insensible à la méthylation, du pyroséquençage au bisulfite quantitatif et de la PCR de séquençage génomique au bisulfite.

6. Procédé selon l'une des revendications précédentes, comprenant en outre : c) la génération d'un profil de risque en utilisant les résultats des étapes a) et b).

7. Procédé selon l'une des revendications précédentes, dans lequel ledit néoplasme gastro-intestinal est le cancer colorectal, le cancer gastrique, le cancer du pancréas, le cancer du foie, le cancer de la vésicule biliaire et/ou des voies biliaires ou le cholangiocarcinome.

8. Procédé selon l'une des revendications précédentes, dans lequel ledit procédé permet de détecter un cancer gastro-intestinal chez ledit sujet avec une sensibilité d'au moins 85% à une spécificité d'au moins 85%, ou avec une sensibilité d'au moins 80% à une spécificité d'au moins 90%.

9. Procédé selon l'une des revendications précédentes, dans lequel ledit échantillon biologique est choisi dans le groupe constitué d'un échantillon de tissu, de selles, de cellules, de bile et d'un échantillon de sang.

10. Ensemble de séquences spécifiques de détection d'acide nucléique de la méthylation, lesdites séquences de détection étant spécifiques de *GLDC* et d'un ou de plusieurs gène(s) choisi(s) dans le groupe constitué de *CDO1,* de *DCLK1,* de *ZSCAN18* et de *ZNF331.*

11. Utilisation de l'ensemble de séquences d'acide nucléique de la revendication 10 pour détecter un état cancéreux chez un sujet, ledit état cancéreux étant un néoplasme gastro-intestinal, ledit néoplasme gastro-intestinal étant de préférence le cancer colorectal, le cancer gastrique, le cancer du pancréas, le cancer du foie, le cancer de la vésicule biliaire et/ou des voies biliaires ou le cholangiocarcinome, étant plus préférablement le cancer colorectal, le cancer gastrique ou le cholangiocarcinome.

12. Utilisation selon la revendication 11, dans laquelle une séquence de détection d'acide nucléique spécifique de la méthylation supplémentaire est utilisée en plus des séquences de détection pour *GLDC* et un ou plusieurs parmi *GLDC* et *CDO1, DCLK1, ZSCAN18* et *ZNF331,* de préférence un ou plusieurs parmi *PPP1R14* et *SFRP1.*

13. Utilisation selon la revendication 11 ou 12, dans laquelle un niveau, une présence ou une fréquence modifié(e) de méthylation pour un sujet par rapport à une référence fournit une indication selon laquelle le sujet a un cancer gastro-intestinal.

14. Utilisation d'un kit dans un procédé selon l'une des revendications 1 à 9, ledit kit comprenant des réactifs utiles, suffisants ou nécessaires pour détecter et/ou caractériser le niveau, la présence ou la fréquence de méthylation de *GLDC* et d'un ou de plusieurs gène(s) choisi(s) dans le groupe constitué de *CDO1,* de *DCLK1,* de *ZSCAN18* et de *ZNF331,* où lesdits réactifs comprennent des sondes d'ADN qui sont spécifiques de *GLDC* et des sondes d'ADN qui sont spécifiques d'un ou de plusieurs desdits gènes *CDO1, DCLK1, ZSCAN18* et *ZNF331.*

15. Utilisation d'un kit de la revendication 14, ledit kit comprenant en outre des réactifs utiles, suffisants ou nécessaires pour détecter et/ou caractériser le niveau, la présence ou la fréquence de méthylation d'au moins l'un de *PPP1R14A* et de *SFRP1,* où lesdits réactifs comprennent des sondes d'ADN qui sont spécifiques à au moins l'un de *PPP1R14A* et de *SFRP1.*


### SEQUENCE LISTING

<110> Oslo universitetssykehus HF
   Lothe, Ragnhild A.
   Lind, Guro E.
   Ahmed, Deeqa
   Andresen, Kim
   Skotheim, Rolf I.
<120> Methods and Biomarkers for Detection of Gastrointestinal Cancers
<130> INVEN-31899/WO-1/ORD
<150> US 61/451,198
   <151> 2011-03-10
<160> 190
<170> PatentIn version 3.5
<210> 1
   <211> 20
   <212> DNA
   <213> Artificial Sequence
<220>
   <223> Synthetic
<400> 1
   gagataggtg cgcgttagtc 20
<210> 2
   <211> 20
   <212> DNA
   <213> Artificial Sequence
<220>
   <223> Synthetic
<400> 2
   acaacgattc tacgcctacg 20
<210> 3
   <211> 23
   <212> DNA
   <213> Artificial Sequence
<220>
   <223> Synthetic
<400> 3
   agtgagatag gtgtgtgtta gtt 23
<210> 4
   <211> 23
   <212> DNA
   <213> Artificial Sequence
<220>
   <223> Synthetic
<400> 4
   acaacaattc tacacctaca cac 23
<210> 5
   <211> 20
   <212> DNA
   <213> Artificial Sequence
<220>
   <223> Synthetic
<400> 5
   agcgagtacg tatatttggc 20
<210> 6
   <211> 18
   <212> DNA
   <213> Artificial Sequence
<220>
   <223> Synthetic
<400> 6
   aaaacgaaac cgaaaacg 18
<210> 7
   <211> 23
   <212> DNA
   <213> Artificial Sequence
<220>
   <223> Synthetic
<400> 7
   agtagtgagt atgtatattt ggt 23
<210> 8
   <211> 21
   <212> DNA
   <213> Artificial Sequence
<220>
   <223> Synthetic
<400> 8
   accaaaacaa aaccaaaaac a 21
<210> 9
   <211> 21
   <212> DNA
   <213> Artificial Sequence
<220>
   <223> Synthetic
<400> 9
   aggggttgat tcgaaagttt c 21
<210> 10
   <211> 20
   <212> DNA
   <213> Artificial Sequence
<220>
   <223> Synthetic
<400> 10
   tctaacgcca aaacgaaaca 20
<210> 11
   <211> 24
   <212> DNA
   <213> Artificial Sequence
<220>
   <223> Synthetic
<400> 11
   gataggggtt gatttgaaag tttt 24
<210> 12
   <211> 23
   <212> DNA
   <213> Artificial Sequence
<220>
   <223> Synthetic
<400> 12
   aactctaaca ccaaaacaaa aca 23
<210> 13
   <211> 20
   <212> DNA
   <213> Artificial Sequence
<220>
   <223> Synthetic
<400> 13
   tacgtttttt aggatgtcgc 20
<210> 14
   <211> 20
   <212> DNA
   <213> Artificial Sequence
<220>
   <223> Synthetic
<400> 14
   cttttaccgc tacgtactcg 20
<210> 15
   <211> 23
   <212> DNA
   <213> Artificial Sequence
<220>
   <223> Synthetic
<400> 15
   aattatgttt tttaggatgt tgt 23
<210> 16
   <211> 23
   <212> DNA
   <213> Artificial Sequence
<220>
   <223> Synthetic
<400> 16
   cccttttacc actacatact caa 23
<210> 17
   <211> 19
   <212> DNA
   <213> Artificial Sequence
<220>
   <223> Synthetic
<400> 17
   ttgggacgtc ggagataac 19
<210> 18
   <211> 20
   <212> DNA
   <213> Artificial Sequence
<220>
   <223> Synthetic
<400> 18
   gaccctcgaa aaaaaaacga 20
<210> 19
   <211> 22
   <212> DNA
   <213> Artificial Sequence
<220>
   <223> Synthetic
<400> 19
   tttttgggat gttggagata at 22
<210> 20
   <211> 23
   <212> DNA
   <213> Artificial Sequence
<220>
   <223> Synthetic
<400> 20
   aaccctcaaa aaaaaaacaa aac 23
<210> 21
   <211> 20
   <212> DNA
   <213> Artificial Sequence
<220>
   <223> Synthetic
<400> 21
   ttttttttgt ttaygtttta 20
<210> 22
   <211> 19
   <212> DNA
   <213> Artificial Sequence
<220>
   <223> Synthetic
<400> 22
   acaaatcaaa ttcaaatct 19
<210> 23
   <211> 20
   <212> DNA
   <213> Artificial Sequence
<220>
   <223> Synthetic
<400> 23
   ttttttttat tggaagcgtc 20
<210> 24
   <211> 19
   <212> DNA
   <213> Artificial Sequence
<220>
   <223> Synthetic
<400> 24
   aaaaaatacc gcgaaaaac 19
<210> 25
   <211> 23
   <212> DNA
   <213> Artificial Sequence
<220>
   <223> Synthetic
<400> 25
   ggtttttttt tattggaagt gtt 23
<210> 26
   <211> 22
   <212> DNA
   <213> Artificial Sequence
<220>
   <223> Synthetic
<400> 26
   ccaaaaaata ccacaaaaaa ca 22
<210> 27
   <211> 18
   <212> DNA
   <213> Artificial Sequence
<220>
   <223> Synthetic
<400> 27
   ttcgtttatt cggcgttc 18
<210> 28
   <211> 19
   <212> DNA
   <213> Artificial Sequence
<220>
   <223> Synthetic
<400> 28
   actcaacgta ccgcctctt 19
<210> 29
   <211> 21
   <212> DNA
   <213> Artificial Sequence
<220>
   <223> Synthetic
<400> 29
   ttttttgttt atttggtgtt t 21
<210> 30
   <211> 22
   <212> DNA
   <213> Artificial Sequence
<220>
   <223> Synthetic
<400> 30
   aaaactcaac ataccacctc tt 22
<210> 31
   <211> 20
   <212> DNA
   <213> Artificial Sequence
<220>
   <223> Synthetic
<400> 31
   acgtgtaaga cgtttttcgc 20
<210> 32
   <211> 20
   <212> DNA
   <213> Artificial Sequence
<220>
   <223> Synthetic
<400> 32
   aacccgacga tactaccctc 20
<210> 33
   <211> 22
   <212> DNA
   <213> Artificial Sequence
<220>
   <223> Synthetic
<400> 33
   gtatgtgtaa gatgtttttt gt 22
<210> 34
   <211> 22
   <212> DNA
   <213> Artificial Sequence
<220>
   <223> Synthetic
<400> 34
   aacccaacaa tactaccctc ct 22
<210> 35
   <211> 20
   <212> DNA
   <213> Artificial Sequence
<220>
   <223> Synthetic
<400> 35
   tcggagcgta taaaagtttc 20
<210> 36
   <211> 20
   <212> DNA
   <213> Artificial Sequence
<220>
   <223> Synthetic
<400> 36
   ctatcgaccg aaacgactac 20
<210> 37
   <211> 22
   <212> DNA
   <213> Artificial Sequence
<220>
   <223> Synthetic
<400> 37
   gtttggagtg tataaaagtt tt 22
<210> 38
   <211> 22
   <212> DNA
   <213> Artificial Sequence
<220>
   <223> Synthetic
<400> 38
   ctatcaacca aaacaactac ca 22
<210> 39
   <211> 19
   <212> DNA
   <213> Artificial Sequence
<220>
   <223> Synthetic
<400> 39
   gcgttttgtt aagaagggc 19
<210> 40
   <211> 19
   <212> DNA
   <213> Artificial Sequence
<220>
   <223> Synthetic
<400> 40
   acgcgctccc ttttcttat 19
<210> 41
   <211> 19
   <212> DNA
   <213> Artificial Sequence
<220>
   <223> Synthetic
<400> 41
   gtgttttgtt aagaagggt 19
<210> 42
   <211> 19
   <212> DNA
   <213> Artificial Sequence
<220>
   <223> Synthetic
<400> 42
   acacactccc ttttcttat 19
<210> 43
   <211> 22
   <212> DNA
   <213> Artificial Sequence
<220>
   <223> Synthetic
<400> 43
   aagattattt gtggggatta gg 22
<210> 44
   <211> 22
   <212> DNA
   <213> Artificial Sequence
<220>
   <223> Synthetic
<400> 44
   aacctctctc tccaaaaaaa aa 22
<210> 45
   <211> 21
   <212> DNA
   <213> Artificial Sequence
<220>
   <223> Synthetic
<400> 45
   gagtgagttt tttagcgaag c 21
<210> 46
   <211> 20
   <212> DNA
   <213> Artificial Sequence
<220>
   <223> Synthetic
<400> 46
   ataaataccg tccgtaacgc 20
<210> 47
   <211> 21
   <212> DNA
   <213> Artificial Sequence
<220>
   <223> Synthetic
<400> 47
   gagtgagttt tttagtgaag t 21
<210> 48
   <211> 20
   <212> DNA
   <213> Artificial Sequence
<220>
   <223> Synthetic
<400> 48
   ataaatacca tccataacac 20
<210> 49
   <211> 20
   <212> DNA
   <213> Artificial Sequence
<220>
   <223> Synthetic
<400> 49
   tatatgggta gcgacgttac 20
<210> 50
   <211> 22
   <212> DNA
   <213> Artificial Sequence
<220>
   <223> Synthetic
<400> 50
   tcgccgaact attaatcaat ta 22
<210> 51
   <211> 23
   <212> DNA
   <213> Artificial Sequence
<220>
   <223> Synthetic
<400> 51
   ttatatatgg gtagtgatgt tat 23
<210> 52
   <211> 25
   <212> DNA
   <213> Artificial Sequence
<220>
   <223> Synthetic
<400> 52
   ccctcaccaa actattaatc aatta 25
<210> 53
   <211> 20
   <212> DNA
   <213> Artificial Sequence
<220>
   <223> Synthetic
<400> 53
   ggggaacggg tttatttttc 20
<210> 54
   <211> 18
   <212> DNA
   <213> Artificial Sequence
<220>
   <223> Synthetic
<400> 54
   gcgaccaatc gaacaaat 18
<210> 55
   <211> 20
   <212> DNA
   <213> Artificial Sequence
<220>
   <223> Synthetic
<400> 55
   ggggaatggg tttatttttt 20
<210> 56
   <211> 18
   <212> DNA
   <213> Artificial Sequence
<220>
   <223> Synthetic
<400> 56
   acaaccaatc aaacaaat 18
<210> 57
   <211> 20
   <212> DNA
   <213> Artificial Sequence
<220>
   <223> Synthetic
<400> 57
   tcgtgtagtg ggtagagttc 20
<210> 58
   <211> 18
   <212> DNA
   <213> Artificial Sequence
<220>
   <223> Synthetic
<400> 58
   ctccgccgaa cgataaat 18
<210> 59
   <211> 23
   <212> DNA
   <213> Artificial Sequence
<220>
   <223> Synthetic
<400> 59
   tttttgtgta gtgggtagag ttt 23
<210> 60
   <211> 21
   <212> DNA
   <213> Artificial Sequence
<220>
   <223> Synthetic
<400> 60
   cccctccacc aaacaataaa t 21
<210> 61
   <211> 19
   <212> DNA
   <213> Artificial Sequence
<220>
   <223> Synthetic
<400> 61
   ggttcgttaa tagtcgggc 19
<210> 62
   <211> 20
   <212> DNA
   <213> Artificial Sequence
<220>
   <223> Synthetic
<400> 62
   ctaaaatcga aacctacgcg 20
<210> 63
   <211> 22
   <212> DNA
   <213> Artificial Sequence
<220>
   <223> Synthetic
<400> 63
   ttaggtttgt taatagttgg gt 22
<210> 64
   <211> 23
   <212> DNA
   <213> Artificial Sequence
<220>
   <223> Synthetic
<400> 64
   actaaaatca aaacctacac aaa 23
<210> 65
   <211> 19
   <212> DNA
   <213> Artificial Sequence
<220>
   <223> Synthetic
<400> 65
   tcggatgtga tttggaaac 19
<210> 66
   <211> 19
   <212> DNA
   <213> Artificial Sequence
<220>
   <223> Synthetic
<400> 66
   cgcgaaaaac gactaaact 19
<210> 67
   <211> 21
   <212> DNA
   <213> Artificial Sequence
<220>
   <223> Synthetic
<400> 67
   atttggatgt gatttggaaa t 21
<210> 68
   <211> 21
   <212> DNA
   <213> Artificial Sequence
<220>
   <223> Synthetic
<400> 68
   cccacaaaaa acaactaaac t 21
<210> 69
   <211> 18
   <212> DNA
   <213> Artificial Sequence
<220>
   <223> Synthetic
<400> 69
   gggtttaggt ttggtcgc 18
<210> 70
   <211> 20
   <212> DNA
   <213> Artificial Sequence
<220>
   <223> Synthetic
<400> 70
   ccgctccgta ccataaataa 20
<210> 71
   <211> 21
   <212> DNA
   <213> Artificial Sequence
<220>
   <223> Synthetic
<400> 71
   agagggttta ggtttggttg t 21
<210> 72
   <211> 23
   <212> DNA
   <213> Artificial Sequence
<220>
   <223> Synthetic
<400> 72
   ccaccactcc ataccataaa taa 23
<210> 73
   <211> 20
   <212> DNA
   <213> Artificial Sequence
<220>
   <223> Synthetic
<400> 73
   agtagataaa gaggcgaggc 20
<210> 74
   <211> 19
   <212> DNA
   <213> Artificial Sequence
<220>
   <223> Synthetic
<400> 74
   aaataccgac gacaaaacg 19
<210> 75
   <211> 23
   <212> DNA
   <213> Artificial Sequence
<220>
   <223> Synthetic
<400> 75
   gggagtagat aaagaggtga ggt 23
<210> 76
   <211> 22
   <212> DNA
   <213> Artificial Sequence
<220>
   <223> Synthetic
<400> 76
   aaataccaac aacaaaacac aa 22
<210> 77
   <211> 22
   <212> DNA
   <213> Artificial Sequence
<220>
   <223> Synthetic
<400> 77
   cgtgacgtga tagtagtcgg tc 22
<210> 78
   <211> 19
   <212> DNA
   <213> Artificial Sequence
<220>
   <223> Synthetic
<400> 78
   ctatccgacc cctaccgac 19
<210> 79
   <211> 23
   <212> DNA
   <213> Artificial Sequence
<220>
   <223> Synthetic
<400> 79
   gtgtgatgtg atagtagttg gtt 23
<210> 80
   <211> 20
   <212> DNA
   <213> Artificial Sequence
<220>
   <223> Synthetic
<400> 80
   cctatccaac ccctaccaac 20
<210> 81
   <211> 19
   <212> DNA
   <213> Artificial Sequence
<220>
   <223> Synthetic
<400> 81
   ttcggaggag ttgtggttc 19
<210> 82
   <211> 21
   <212> DNA
   <213> Artificial Sequence
<220>
   <223> Synthetic
<400> 82
   cgctaatacc gaaaaaaaac g 21
<210> 83
   <211> 22
   <212> DNA
   <213> Artificial Sequence
<220>
   <223> Synthetic
<400> 83
   gattttggag gagttgtggt tt 22
<210> 84
   <211> 24
   <212> DNA
   <213> Artificial Sequence
<220>
   <223> Synthetic
<400> 84
   cactaatacc aaaaaaaaac aaac 24
<210> 85
   <211> 20
   <212> DNA
   <213> Artificial Sequence
<220>
   <223> Synthetic
<400> 85
   ttagcggatt taatggttgc 20
<210> 86
   <211> 19
   <212> DNA
   <213> Artificial Sequence
<220>
   <223> Synthetic
<400> 86
   taaccgaaac tccgacctc 19
<210> 87
   <211> 20
   <212> DNA
   <213> Artificial Sequence
<220>
   <223> Synthetic
<400> 87
   ttagtggatt taatggttgt 20
<210> 88
   <211> 19
   <212> DNA
   <213> Artificial Sequence
<220>
   <223> Synthetic
<400> 88
   taaccaaaac tccaacctc 19
<210> 89
   <211> 20
   <212> DNA
   <213> Artificial Sequence
<220>
   <223> Synthetic
<400> 89
   atttagggtt tagttcgggc 20
<210> 90
   <211> 21
   <212> DNA
   <213> Artificial Sequence
<220>
   <223> Synthetic
<400> 90
   acactttaaa acgactccga a 21
<210> 91
   <211> 23
   <212> DNA
   <213> Artificial Sequence
<220>
   <223> Synthetic
<400> 91
   tttatttagg gtttagtttg ggt 23
<210> 92
   <211> 24
   <212> DNA
   <213> Artificial Sequence
<220>
   <223> Synthetic
<400> 92
   actacacttt aaaacaactc caaa 24
<210> 93
   <211> 19
   <212> DNA
   <213> Artificial Sequence
<220>
   <223> Synthetic
<400> 93
   tgcggttgtg gtttttttc 19
<210> 94
   <211> 18
   <212> DNA
   <213> Artificial Sequence
<220>
   <223> Synthetic
<400> 94
   ccgaaacgac gttctcat 18
<210> 95
   <211> 22
   <212> DNA
   <213> Artificial Sequence
<220>
   <223> Synthetic
<400> 95
   tattgtggtt gtggtttttt tt 22
<210> 96
   <211> 21
   <212> DNA
   <213> Artificial Sequence
<220>
   <223> Synthetic
<400> 96
   acaccaaaac aacattctca t 21
<210> 97
   <211> 20
   <212> DNA
   <213> Artificial Sequence
<220>
   <223> Synthetic
<400> 97
   ggtagtcggc gtttagtttc 20
<210> 98
   <211> 20
   <212> DNA
   <213> Artificial Sequence
<220>
   <223> Synthetic
<400> 98
   cgcaactaaa ccgctttaat 20
<210> 99
   <211> 23
   <212> DNA
   <213> Artificial Sequence
<220>
   <223> Synthetic
<400> 99
   tagggtagtt ggtgtttagt ttt 23
<210> 100
   <211> 23
   <212> DNA
   <213> Artificial Sequence
<220>
   <223> Synthetic
<400> 100
   aaacacaact aaaccacttt aat 23
<210> 101
   <211> 19
   <212> DNA
   <213> Artificial Sequence
<220>
   <223> Synthetic
<400> 101
   tttttcgggt tagttttgc 19
<210> 102
   <211> 20
   <212> DNA
   <213> Artificial Sequence
<220>
   <223> Synthetic
<400> 102
   aaccgaacga atttcgtaat 20
<210> 103
   <211> 22
   <212> DNA
   <213> Artificial Sequence
<220>
   <223> Synthetic
<400> 103
   gggttttttg ggttagtttt gt 22
<210> 104
   <211> 23
   <212> DNA
   <213> Artificial Sequence
<220>
   <223> Synthetic
<400> 104
   ccaaaccaaa caaatttcat aat 23
<210> 105
   <211> 20
   <212> DNA
   <213> Artificial Sequence
<220>
   <223> Synthetic
<400> 105
   gtttagggga ttttgcgttc 20
<210> 106
   <211> 20
   <212> DNA
   <213> Artificial Sequence
<220>
   <223> Synthetic
<400> 106
   acaaccgacc gctactttaa 20
<210> 107
   <211> 20
   <212> DNA
   <213> Artificial Sequence
<220>
   <223> Synthetic
<400> 107
   gtttagggga ttttgtgttt 20
<210> 108
   <211> 20
   <212> DNA
   <213> Artificial Sequence
<220>
   <223> Synthetic
<400> 108
   acaaccaacc actactttaa 20
<210> 109
   <211> 21
   <212> DNA
   <213> Artificial Sequence
<220>
   <223> Synthetic
<400> 109
   ggtttacggg ttgttgtatt c 21
<210> 110
   <211> 20
   <212> DNA
   <213> Artificial Sequence
<220>
   <223> Synthetic
<400> 110
   aaaaaccgac gactctcttt 20
<210> 111
   <211> 22
   <212> DNA
   <213> Artificial Sequence
<220>
   <223> Synthetic
<400> 111
   gggtttatgg gttgttgtat tt 22
<210> 112
   <211> 21
   <212> DNA
   <213> Artificial Sequence
<220>
   <223> Synthetic
<400> 112
   caaaaaccaa caactctctt t 21
<210> 113
   <211> 18
   <212> DNA
   <213> Artificial Sequence
<220>
   <223> Synthetic
<400> 113
   gtgtgtagag tcgggtgc 18
<210> 114
   <211> 21
   <212> DNA
   <213> Artificial Sequence
<220>
   <223> Synthetic
<400> 114
   aaaaccgaaa cgaatacaaa a 21
<210> 115
   <211> 18
   <212> DNA
   <213> Artificial Sequence
<220>
   <223> Synthetic
<400> 115
   gtgtgtagag ttgggtgt 18
<210> 116
   <211> 21
   <212> DNA
   <213> Artificial Sequence
<220>
   <223> Synthetic
<400> 116
   aaaaccaaaa caaatacaaa a 21
<210> 117
   <211> 21
   <212> DNA
   <213> Artificial Sequence
<220>
   <223> Synthetic
<400> 117
   gcgtaattat attgcggtat c 21
<210> 118
   <211> 20
   <212> DNA
   <213> Artificial Sequence
<220>
   <223> Synthetic
<400> 118
   tacgttaacc gatcctacaa 20
<210> 119
   <211> 24
   <212> DNA
   <213> Artificial Sequence
<220>
   <223> Synthetic
<400> 119
   gttgtgtaat tatattgtgg tatt 24
<210> 120
   <211> 23
   <212> DNA
   <213> Artificial Sequence
<220>
   <223> Synthetic
<400> 120
   aactacatta accaatccta caa 23
<210> 121
   <211> 19
   <212> DNA
   <213> Artificial Sequence
<220>
   <223> Synthetic
<400> 121
   ttttcgtata cgcggaatc 19
<210> 122
   <211> 21
   <212> DNA
   <213> Artificial Sequence
<220>
   <223> Synthetic
<400> 122
   tcgacacgtc atttatacca c 21
<210> 123
   <211> 22
   <212> DNA
   <213> Artificial Sequence
<220>
   <223> Synthetic
<400> 123
   ttttttttgt atatgtggaa tt 22
<210> 124
   <211> 24
   <212> DNA
   <213> Artificial Sequence
<220>
   <223> Synthetic
<400> 124
   ctctcaacac atcatttata ccac 24
<210> 125
   <211> 18
   <212> DNA
   <213> Artificial Sequence
<220>
   <223> Synthetic
<400> 125
   attaggcgaa gttgtcgc 18
<210> 126
   <211> 19
   <212> DNA
   <213> Artificial Sequence
<220>
   <223> Synthetic
<400> 126
   cgactcgtcc gacttaaaa 19
<210> 127
   <211> 20
   <212> DNA
   <213> Artificial Sequence
<220>
   <223> Synthetic
<400> 127
   ggattaggtg aagttgttgt 20
<210> 128
   <211> 21
   <212> DNA
   <213> Artificial Sequence
<220>
   <223> Synthetic
<400> 128
   aacaactcat ccaacttaaa a 21
<210> 129
   <211> 20
   <212> DNA
   <213> Artificial Sequence
<220>
   <223> Synthetic
<400> 129
   ggacgcgttc gtttttagtc 20
<210> 130
   <211> 19
   <212> DNA
   <213> Artificial Sequence
<220>
   <223> Synthetic
<400> 130
   aaccgcgaca cgttctaac 19
<210> 131
   <211> 23
   <212> DNA
   <213> Artificial Sequence
<220>
   <223> Synthetic
<400> 131
   gtaggatgtg tttgttttta gtt 23
<210> 132
   <211> 22
   <212> DNA
   <213> Artificial Sequence
<220>
   <223> Synthetic
<400> 132
   aaccacaaca cattctaaca ac 22
<210> 133
   <211> 20
   <212> DNA
   <213> Artificial Sequence
<220>
   <223> Synthetic
<400> 133
   taaatttcgg acgagttttc 20
<210> 134
   <211> 21
   <212> DNA
   <213> Artificial Sequence
<220>
   <223> Synthetic
<400> 134
   tcgcgaaaca atttatattt c 21
<210> 135
   <211> 23
   <212> DNA
   <213> Artificial Sequence
<220>
   <223> Synthetic
<400> 135
   gtttaaattt tggatgagtt ttt 23
<210> 136
   <211> 24
   <212> DNA
   <213> Artificial Sequence
<220>
   <223> Synthetic
<400> 136
   ccatcacaaa acaatttata tttc 24
<210> 137
   <211> 21
   <212> DNA
   <213> Artificial Sequence
<220>
   <223> Synthetic
<400> 137
   tagtaaatcg aattcgttcg c 21
<210> 138
   <211> 19
   <212> DNA
   <213> Artificial Sequence
<220>
   <223> Synthetic
<400> 138
   tacgcgaaac tcctacgac 19
<210> 139
   <211> 24
   <212> DNA
   <213> Artificial Sequence
<220>
   <223> Synthetic
<400> 139
   ttttagtaaa ttgaatttgt ttgt 24
<210> 140
   <211> 22
   <212> DNA
   <213> Artificial Sequence
<220>
   <223> Synthetic
<400> 140
   tacacaaaac tcctacaacc aa 22
<210> 141
   <211> 19
   <212> DNA
   <213> Artificial Sequence
<220>
   <223> Synthetic
<400> 141
   gttgagtggt tgttcggtc 19
<210> 142
   <211> 20
   <212> DNA
   <213> Artificial Sequence
<220>
   <223> Synthetic
<400> 142
   cccgactctc ctacgattaa 20
<210> 143
   <211> 21
   <212> DNA
   <213> Artificial Sequence
<220>
   <223> Synthetic
<400> 143
   gtgttgagtg gttgtttggt t 21
<210> 144
   <211> 22
   <212> DNA
   <213> Artificial Sequence
<220>
   <223> Synthetic
<400> 144
   tacccaactc tcctacaatt aa 22
<210> 145
   <211> 19
   <212> DNA
   <213> Artificial Sequence
<220>
   <223> Synthetic
<400> 145
   cgttttggaa ttatagcgc 19
<210> 146
   <211> 19
   <212> DNA
   <213> Artificial Sequence
<220>
   <223> Synthetic
<400> 146
   ttccgaataa tcctcgaaa 19
<210> 147
   <211> 22
   <212> DNA
   <213> Artificial Sequence
<220>
   <223> Synthetic
<400> 147
   ttttgttttg gaattatagt gt 22
<210> 148
   <211> 22
   <212> DNA
   <213> Artificial Sequence
<220>
   <223> Synthetic
<400> 148
   ctcttccaaa taatcctcaa aa 22
<210> 149
   <211> 23
   <212> DNA
   <213> Artificial Sequence
<220>
   <223> Synthetic
<400> 149
   gaatttgtaa tttcgtgcgt ttc 23
<210> 150
   <211> 22
   <212> DNA
   <213> Artificial Sequence
<220>
   <223> Synthetic
<400> 150
   aaaaaaaact ctccgtacac cg 22
<210> 151
   <211> 25
   <212> DNA
   <213> Artificial Sequence
<220>
   <223> Synthetic
<400> 151
   tgaatttgta attttgtgtg ttttg 25
<210> 152
   <211> 24
   <212> DNA
   <213> Artificial Sequence
<220>
   <223> Synthetic
<400> 152
   aaaaaaaact ctccatacac cacc 24
<210> 153
   <211> 21
   <212> DNA
   <213> Artificial Sequence
<220>
   <223> Synthetic
<400> 153
   atcgtcgggg tttttttagt c 21
<210> 154
   <211> 19
   <212> DNA
   <213> Artificial Sequence
<220>
   <223> Synthetic
<400> 154
   aacaaaaaca cgacccgac 19
<210> 155
   <211> 23
   <212> DNA
   <213> Artificial Sequence
<220>
   <223> Synthetic
<400> 155
   gtattgttgg ggttttttta gtt 23
<210> 156
   <211> 21
   <212> DNA
   <213> Artificial Sequence
<220>
   <223> Synthetic
<400> 156
   aaacaaaaac acaacccaac c 21
<210> 157
   <211> 20
   <212> DNA
   <213> Artificial Sequence
<220>
   <223> Synthetic
<400> 157
   ggtaggacgt ttttagggtc 20
<210> 158
   <211> 20
   <212> DNA
   <213> Artificial Sequence
<220>
   <223> Synthetic
<400> 158
   atacaactct acacgacgca 20
<210> 159
   <211> 23
   <212> DNA
   <213> Artificial Sequence
<220>
   <223> Synthetic
<400> 159
   taaggtagga tgtttttagg gtt 23
<210> 160
   <211> 23
   <212> DNA
   <213> Artificial Sequence
<220>
   <223> Synthetic
<400> 160
   aacatacaac tctacacaac aca 23
<210> 161
   <211> 21
   <212> DNA
   <213> Artificial Sequence
<220>
   <223> Synthetic
<400> 161
   gtttaaaatg acgtaggcgt c 21
<210> 162
   <211> 20
   <212> DNA
   <213> Artificial Sequence
<220>
   <223> Synthetic
<400> 162
   aataccgcga aactataccg 20
<210> 163
   <211> 24
   <212> DNA
   <213> Artificial Sequence
<220>
   <223> Synthetic
<400> 163
   ggtgtttaaa atgatgtagg tgtt 24
<210> 164
   <211> 23
   <212> DNA
   <213> Artificial Sequence
<220>
   <223> Synthetic
<400> 164
   acaataccac aaaactatac cac 23
<210> 165
   <211> 22
   <212> DNA
   <213> Artificial Sequence
<220>
   <223> Synthetic
<400> 165
   ttttggttgt taggggttta tt 22
<210> 166
   <211> 21
   <212> DNA
   <213> Artificial Sequence
<220>
   <223> Synthetic
<400> 166
   acccacctac tacrcaacta c 21
<210> 167
   <211> 36
   <212> DNA
   <213> Artificial Sequence
<220>
   <223> Synthetic
<400> 167
   ggttaggtat agtggtttat atttgtaatt ttagta 36
<210> 168
   <211> 33
   <212> DNA
   <213> Artificial Sequence
<220>
   <223> Synthetic
<400> 168
   attaactaaa ctaatcttaa actcctaacc tca 33
<210> 169
   <211> 16
   <212> DNA
   <213> Artificial Sequence
<220>
   <223> Synthetic
<400> 169
   cctaccttaa cctccc 16
<210> 170
   <211> 20
   <212> DNA
   <213> Artificial Sequence
<220>
   <223> Synthetic
<400> 170
   cgaattatag cggcggaggt 20
<210> 171
   <211> 19
   <212> DNA
   <213> Artificial Sequence
<220>
   <223> Synthetic
<400> 171
   aaatcgcgta aactccgcg 19
<210> 172
   <211> 16
   <212> DNA
   <213> Artificial Sequence
<220>
   <223> Synthetic
<400> 172
   cgttaggtcg ggcggt 16
<210> 173
   <211> 15
   <212> DNA
   <213> Artificial Sequence
<220>
   <223> Synthetic
<400> 173
   gcgcgtacgc ggagg 15
<210> 174
   <211> 16
   <212> DNA
   <213> Artificial Sequence
<220>
   <223> Synthetic
<400> 174
   cgacgacgaa cgcgct 16
<210> 175
   <211> 15
   <212> DNA
   <213> Artificial Sequence
<220>
   <223> Synthetic
<400> 175
   cgggagggcg tgtga 15
<210> 176
   <211> 18
   <212> DNA
   <213> Artificial Sequence
<220>
   <223> Synthetic
<400> 176
   gaattcgttc gcgaggga 18
<210> 177
   <211> 21
   <212> DNA
   <213> Artificial Sequence
<220>
   <223> Synthetic
<400> 177
   aaacgaaccg cactcgttac c 21
<210> 178
   <211> 15
   <212> DNA
   <213> Artificial Sequence
<220>
   <223> Synthetic
<400> 178
   cgtcaccgac gcgaa 15
<210> 179
   <211> 21
   <212> DNA
   <213> Artificial Sequence
<220>
   <223> Synthetic
<400> 179
   cgcggtatag tttcgcggta t 21
<210> 180
   <211> 19
   <212> DNA
   <213> Artificial Sequence
<220>
   <223> Synthetic
<400> 180
   cgcgataacg accgacaaa 19
<210> 181
   <211> 17
   <212> DNA
   <213> Artificial Sequence
<220>
   <223> Synthetic
<400> 181
   cgtagttcgc ggtgagg 17
<210> 182
   <211> 67
   <212> DNA
   <213> Artificial Sequence
<220>
   <223> Synthetic
<400> 182
   tttatttttt gtttttaggg tgttgtttaa agtgtgtgga gtaggtgttt tgggtggagt 60
   tataatt 67
<210> 183
   <211> 66
   <212> DNA
   <213> Artificial Sequence
<220>
   <223> Synthetic
<400> 183
   gttttaattt atttttcgtt tttagggcgt cgtttaaagt gtgcggagta ggtgttttgg 60
   gtggag 66
<210> 184
   <211> 67
   <212> DNA
   <213> Artificial Sequence
<220>
   <223> Synthetic
<400> 184
   gtgatcgttc gattttttat agcgatagcg gcgtaggcgt tttcgtatta atagtttttt 60
   atttcgt 67
<210> 185
   <211> 1500
   <212> DNA
   <213> Homo sapiens
<400> 185
   acacacacac acacagttct tttttttttt tttttcctga gtctcgttct gtggcccacg      60
   ctggagtgca gtggtgcgat cacatctcac tgcagcctga aactccctga ctcaagcaat     120
   cctcccacct cagcctccag agtagctggg acttcagaag cacgcaacca cgccccacta     180
   atttttgtat tttttgtaga gacagggttt cacaatgctg cccaagctgg tctcgaactc     240
   ctgggctcaa gctatcctac cgcgtcggtt tccgaaagtg ttgcgattac aggcgtgagc     300
   catagcgtta catgtggtgg gggggaagag agagaaagag agagagtgag aagaaggagg     360
   aggaggagga ggaggagaaa gaaggaagaa agaaaagaaa agaaatatag agagggtgtt     420
   caaatctact gttcaagtgc tttccatgta catataatct ctttttaaaa tcccatccag     480
   ctctgttgag atgagaaaac tgaagctctg agaggttgag taactttatc acgctcccgc     540
   aaagataaaa gaaccgccat ctgcccatat taatttccct gagatgaaat gtgacagaga     600
   aagatacctc aaaattaagg aaatggagag cttcgagaat catcttccat ctccgccgag     660
   tcccccagga acttggcccg gagtctttgg gctgtcccac ctgatcttgc cccagtgcgg     720
   ctccacaccc tggcgggcgg cgtacggagc gtcccggccc ccggcaccag ccagcgtcgg     780
   agtgggtagg actggagatg gcagcctccg gtccagggga agcgggactc cacggaaggg     840
   ctgggaagag ggtaggaaga gggggcgggg gcgggaactg ttccgccccc tccccctctg     900
   tctactttca ttggccaagg gcttcctggc cccaacccac ctcccgcctc cagggcgccg     960
   cttaaagtgt gcggagcagg tgccttgggt ggagccacaa ctttgcgcga gtgtcttggt    1020
   tgagcgcagc gcccattcat tgcccgcgag cgtccatcca tctgtccggc cgactgtcca    1080
   gcgaaagggg ctccaggccg ggcgcagccg ccacccgggg gaccgaggcc aggagagggg    1140
   ccaagagcgc ggctgaccct tgcgggccgg ggcaggggac ggtggccgcg gccatgcagt    1200
   cctgtgccag ggcgtggggg ctgcgcctgg gccgcggggt cgggggcggc cgccgcctgg    1260
   ctgggggatc ggggccgtgc tgggcgccgc ggagccggga cagcagcagt ggcggcgggg    1320
   acagcgccgc ggctggggcc tcgcgcctcc tggagcgcct tctgcccaga cacgacgact    1380
   tcgctcggag gcacatcggc cctggggaca aagaccagag agagatgctg cagaccttgg    1440
   ggctggcggt aaggacctcc acccggccct ccgcggcctc cctccgcccc tgcccgggct    1500
<210> 186
   <211> 1500
   <212> DNA
   <213> Homo sapiens
<400> 186
   tttcaccatg ttggccaggc tggtcttgaa ctcctgacct cgtgatccac cctccttggc 60
   ctcccaaagt tctgggatta caggagtgag ccactgcgcc cagccacatt taaattttag 120
   agtataatta ttgctgctta taattagaag tgagaagact gttttgaagt aagggacttc 180
   cctggccacc ctcgctaaaa ttgcaacctt accctcactc catttctcct tctctgcttt 240
   atatttctcc aaagcactta tatgataata aactgtataa taccgtactt actgtgtttc 300
   tcatgtatcc cggctacaac tgtcaactgc gtgggactgg ttttgtctgt tttgttccct 360
   gctctatccc tggagcgtaa agcagtgtct ggtatagagt ggatacttaa tagatattcg 420
   ttgaaccggg ccagtggcgg tggctcacgc ctgtaatccc agcactttgg gaggcggagg 480
   caggcagatc acttgaggtc aggagttcga ggccagcctg gccaacatgg cgaaatcccg 540
   tctctactaa aaatacaaaa attagccggg tgttgtggtg catccctgta atcccagcta 600
   cttcggaggc tgaggcagga gaatcgcttg aacctgggag gcggacgttg cggtgagccg 660
   agatcgcgcc attgtactcc agcctgggcg acaaagagag acatcgactc aaaaaaaaaa 720
   aaattcgttg aacggaatga gtgaacaaac aaatggatgg atgagtgaat gaatgaacga 780
   aggaacaagt gaccgctcga ccttctacag cgacagcggc gcaggcgctc ccgcaccaac 840
   agccccccac cccgccaccg ccagagggcg cagacaggcc gggtcccgag tgtctgtccc 900
   aggcccccgc cccacaggat cccggcccgg gagcccccga tgcagggacc cgcccgggac 960
   tctgggtccc cgcccatacc tcaacttcgc acgactgcgt gcctcaagcc gacgcagcgg 1020
   cctactctcg cactgcagac ggggaaactg aggcctgagg cggccggggt ggggcagacc 1080
   tcccggcgag cccgagcccc cgcccccggc tagccccgcc ctggcccgta agaagcaccc 1140
   ggggcgcgag gcgaaggcgc acagcgcggg gccaggctgg gtccagcagc gcgatggcag 1200
   ctcagcggct gggcaagcgc gtgctgagca agctgcagtc tccatcgcgg gcccgcgggc 1260
   cagggggcag tcccgggggg ctgcagaagc ggcacgcgcg cgtcaccgtc aagtatgacc 1320
   ggcgggagct gcagcggcgg ctggacgtgg agaagtggat cgacgggcgc ctggaggagc 1380
   tgtaccgcgg catggtgagc ccggggaggg ctcgggggtg gtcggggaca gatagagccc 1440
   gggcgcggag accccacgcc cttggggtct cggtgcacac tcgctggtgc acggccagcc 1500
<210> 187
   <211> 1500
   <212> DNA
   <213> Homo sapiens
<400> 187
   tcttcagaag tctagaactt taataacctt aaatccaaat tattccctgc gtccatccat      60
   aatgagaagg aagaaatcta cacttgcatt tcttccacct ttcacatctt tctcatttaa     120
   atcctactgt acttgatttg tgttgttccc aacgtactag gtgctatgca tgacgtagaa     180
   acgtgtttcc ttatatgtat gtactagcag gctccttcaa gcacatgcct aaggttgaaa     240
   gattcaaata taattatagc ctcttaaatt attgcgtcag agcccgccct atcccctcag     300
   aaaaagcccc cttttacacc atcaaatgaa atatcaggtt atttgcactg gatatttttt     360
   aaaagaaaat atttgatttt aaaatcaaag agttcttggt tttccatcat ttcactaatt     420
   gtcttactag agacccgagt ctcctttgtc aaaatatatt tcagtacgca ttttaaaaaa     480
   tatgtgtgtg tgtatactta aatatatata tatataaagt cttggactta ttttcaccta     540
   gcctggggca tatcgtggat agtttcaatc gacaaaacag gcactctctg gcctaggagt     600
   ggaatccatc ctcaattttc gcaggctcct cacaattctc tatttggaag aagtgtccct     660
   ctccttccct tttcttttcc tcctttactc agcgtcagtc cccgcagcca tctcctccga     720
   ccctttttgt ctacgtccca gcgtcgcgaa ccacagcggc ggaggtggag cggggagagg     780
   cgttaggccg ggcggctaaa acgcgccgtt aaagtggggg agagattgcg cggagcccac     840
   gcgatccctg ggacgccgga gacaacgggg ctcttgggaa ggcgcggagc ccggggaagc     900
   cggggatgtg cgcgtgagcc gtgcccgcag ggtctccccg cctccgccac ctttcttggg     960
   tggctctccg cctcgtcctc cctccgaggg ccgttggtac attcctagtg actccaagcg    1020
   cttaaaaggg gcccgggagg atgaacccca cagatctgaa cctgatttgt gtgtgcaccg    1080
   cgtctccagc gatcccggat ccactgcgct gccaggggcc tgggggtggg tctcttgctg    1140
   tctctgcgac gacatcctta cgtttcggca ctctaatgct gggtttgtgc gtgtgtgtct    1200
   gcttagcggt ctagcgggct gttaggctcc ctcgccccca gctccttggc tcgctcagct    1260
   cctccaccgc agcccagcag tgagacgcgc gcgcagccag ctccccacga gatggaacag    1320
   accgaagtgc tgaagccacg gaccctggct gatctgatcc gcatcctgca ccagctcttt    1380
   gccggcgatg aggtcaatgt agaggaggtg caggccatca tggaagccta cgagagcgac    1440
   cccaccgagt gggcaatgta cgccaagttc gaccagtaca ggtgagcgcg ctgcagcttc    1500
<210> 188
   <211> 1500
   <212> DNA
   <213> Homo sapiens
<400> 188
   tgtgtatttg ggacaaacac aggttgtgga cgacaggatt gaaagtcaga ttaactggat      60
   caaaattcag gctgcgtact gagccagtct aagatttcac cgaaatctgg gagctgctcc     120
   cactccctgg acttttgtgc aaagactgga ggaagctgtg aacagcctgg gctaggagaa     180
   cattccccct ctgggaggag aggctcgcat gagctcatgg ggctttccac cgcagaccca    240
   gccttggcag ccacaaagtg tccagcagtg aaacctggcc cttcagttct caagggccct     300
   ttggaacata tttgactcta agcagaggtc actattccaa gagtgactca tgtcttgggg     360
   ttaagtggag atgatgggtg ggatccatga acagatccag ctcttcccaa tgtggggggc      420
   accagagtgc atagcttggg agggttggtc atccgaagag gcactgcgtg ggtgcatccc     480
   gggcaaaaag gatgagaagg tgatccactg gcttccatac cctgggaaag gtgtcagacc     540
   gtgaggtcac atcaaaaggt atgagtgtgg gtacctcctt ccctcccccg acggtggggg     600
   cctcatctct cggccatatc acctctgtgc cttgcacact tcctggctca gactcagccc     660
   tgtccacgtt ctcttccacc atctgatctg cccccatctc ccaaagacta cttgtgggga     720
   ctagggaggt ttaggggtgt agtcaagcca gatgcaccga ggttgctgcg gtgggaagag     780
   ggcgctggga gggaggagga cctggggcgc agccgtggtg ggtgcgccct gccaagaagg     840
   gcgggggcga cggggcgcgc acgcggagga ggaggaggag gagggagacg ggagggcgtg     900
   tgagcgagtg agacaagaaa agggagcgcg cccgccgccg ccgccgccgc cgccctcctc     960
   tggagagaga ggctggagtg aggctgtgcg aagcgccgca tttcaatgag gacgggccga    1020
   ggcacatccc tgcactagtg gccgcaaccg aggcgccgcg ctccagcagc tgctgccgcc    1080
   cagcccggcc ccgccgccgc cccccagccc tgcagccccg cagccccggc cgcgcccagc    1140
   ccggcgagga cagcaccagg aggcggcccc cagcgcggcc acaaagaccc ccggcggcgt    1200
   ctctccgcgg accggtgcgt ggtttgcctt ccctggggac gggagctgcg gggagggcgg    1260
   ccgggggact ccgcagagcc cgctccgccc gcagccgcgg ggcgccgagg gcagggcggg    1320
   cgctcagctt ggccccgccg ccccgcccgc agttcctcga aaggcggccg ccctgccctg    1380
   gccagcctgg cccgacccgg gggcgttgcg tgcgggcggc cccggggctg ctggcacctg    1440
   cctcttttgt ttggtgccat ggggctttaa acctggagtc aggcgagcgg gccctcccag    1500
<210> 189
   <211> 1500
   <212> DNA
   <213> Homo sapiens
<400> 189
   tcggggctgt atttccaaga acgctctgga ggcacagctc ttttctaagg agggccatgt 60
   tgcatgctgg gaaatttttg aacttttccc aaatcacagc ttttaagctt tccgggctta 120
   ttattattat tattattatt ttgagacatt gtttcactct tatcgcccag gctggagtgc 180
   agtggtgtga tctcggctca ctgcaacctc tgcctcctgg gttcaagcga ttctcttccc 240
   tcagcctccc aagtagctgg gattacaggc acacaccacc acgcctggct aatttttgca 300
   tttttagagc gatggggttt caccatgttg ggcaggaggg tctcgaactc ctgatctcag 360
   gtgatatacc cgcctcggcc tcccaaagtg ctgagattac aggcgtgatt tctggtcgtt 420
   tcagactaca tttccttccc agaaggacgc ggggacacgc cttccctatg gacggatccc 480
   aaatgcaagt tgtaagcaat tataactgta ggcccctaga gcctgaacca cttttcccag 540
   gaattttttg tggtgcgcct taggagcaag caaagtttca gggcaggaga aggccgacag 600
   ggcttccagc atttgaagat tctgccagtc tgaaatacgc tttcccagaa gtctatgggg 660
   ggtgcagctg ctctaacagt gggaggagac accgacgaaa agcattctga cggccgcgct 720
   tccagcgcta aggattcccg cgtagtgagc atttcccaga ggtctctggg gcatggctcc 780
   attagggccg tgtgcagttg cctgcaggga aacactgatg ggcgcccttc cagatacgtc 840
   gattcccggg ttatgagcta catttcccag aagccctcaa ggcaggacgc ccttagggtc 900
   gcgtagagtt gcatgctggg aaatcctgac agccgcttcc aactctgtgg atttccgggt 960
   tatggactac atttcccaga ggcctctggg tcacggctcc atttgcgccg tgtagagttg 1020
   catgctggga aacactgacg ggcgcccttc cagctgcgtc gaagcttctc tggtttcgca 1080
   ttacatttcc cagagggcct tggggcacag tccaatgagg agcgagccgg gtggccggct 1140
   gaggggcgtg cttcatgctc gcgggatttt taaaaatggt ttctcctgga tcttatccca 1200
   actgcgcctg agaccttgtt tcacctcctg atgtgctggg tgtggttagg cctcgagccg 1260
   ggagctattt gcctcccagg aggaagccag ggcagggttc cgcggggaat ggcggtgagg 1320
   gagccactcg ggacgcggag gccctaaaat gaagaagctt cggcctgagg cttcgggagc 1380
   ggcgagcagg gaagtgggag tgtgtgtgag tgtgtgtgtg tgtgtgtgtg tgtgtgtgtg 1440
   aataagccag aggctgcctg gtggggcccc gccgtggccc agtgactgag ataattgtct 1500
<210> 190
   <211> 1500
   <212> DNA
   <213> Homo sapiens
<400> 190
   ggccacatac tgtatgatcc cacttatatg acactgtgaa ggcaaaattg tagcagtgga 60
   ctacttagat tagctgttcc tggggttatg ggtggggagg gagtgactac caagaggcag 120
   caggaggatt tttgggcagt ggagctattc tttgtcccga gtttgttggt ggatatgagt 180
   taaaattcat aaaactgaat accaaaatgt gtggtgtatg taaatttaaa atatataaac 240
   tgttcagata tacattttaa aaatcagttc acacaaaaac gaccaaaatg gcatcctcat 300
   aagcgcctct atctggaaaa ctgaaatggg aagcagctcc tggccctatc ctgccttcca 360
   ggggggctac atttatatgg ggctttgcca aagccctcac cccagctgcc actcacattt 420
   ccctcatttc agcagctcct gaatccctgg ggtgggtaat ctttgagttc tatccactcc 480
   ttgaaactgt agaacccatt ttagcgcatc agtgtctctg ggtctccacc tcttgtctcc 540
   ccagtactgg gtctgctggt ccctgtctct gtatctctgt ccagatctgt ctcagcctcg 600
   agtgctcctt ttctctgggt ctctctcttt tcctgagcct gagctggtct ctttcttttc 660
   ttgagcccat gctggtcttt ccctttcttg atcctgggct ggtctccctc ctttcttaac 720
   cgtgagctgg tctctccctt tccgtgtccg actctgtaca tctctgtttc ggggacccca 780
   gggcgtcttg gtctgccccc gagcgtctgt gggccttcat gcttctctcc ctcccggtct 840
   gcgcgtcttt ttgtctttgt ttctccgttt ctcaccgtct ttgtttctcc gggtctctgg 900
   ctgtcagggg tctattattt acccgcttgt ttacttcgtt gccatggtgc ccaaaatgac 960
   gcaggcgccg gagtgggcgg gacgccggga ggccacactc gcgtctgatt ggccatgcgc 1020
   actgtcacct gtcacggctc ccttgctcgc cccggtgcgc ggcacagcct cgcggcattg 1080
   tgggagtcgt agtccgcggt gaggcccagt gcgcaggcgc agtttgccgg ccgccatcgc 1140
   gcactggggc tccgggcggc tgggaccggc ctggggtagc tgcgcagcag gtgggtcggt 1200
   cccggggatg cagcgcgggg gtcagagagc gaccccaaca gggtagacgg tgaggacgcc 1260
   tcagtgggca gcgttaggga aaggggccag ggtgttcccg gggttggggt gcgagccccc 1320
   gctgtgcgtc caggaggccg agggtctggc atcccttcgc gtggagtgcg gggcaggggc 1380
   cgggtccccc cggaggtggc tgagatccag atcggtgagg ggccctgcag gcaggtaggg 1440
   aactagtgca acccccaccc cgtgtaggct cgtggagggg aggcgcttag ggcaccctgt 1500